(19) Europäisches Patentamt
European Patent Office
Office européen des brevets

(11) Veröffentlichungsnummer: **0 271 824 B1**

(12) **EUROPÄISCHE PATENTSCHRIFT**

(45) Veröffentlichungstag der Patentschrift: **21.07.93**

(21) Anmeldenummer: **87118264.8**

(22) Anmeldetag: **09.12.87**

(51) Int. Cl.⁵: **C12P 21/02**, C12P 21/00, C12N 15/23, C12N 1/20, A61K 37/66, G01N 33/577, G01N 33/68, //(C12P21/00, C12R1:91)

# (54) Pferde-Gamma-Interferon.

(30) Priorität: **10.12.86 DE 3642096**

(43) Veröffentlichungstag der Anmeldung:
**22.06.88 Patentblatt 88/25**

(45) Bekanntmachung des Hinweises auf die Patenterteilung:
**21.07.93 Patentblatt 93/29**

(84) Benannte Vertragsstaaten:
**AT BE CH DE ES FR GB GR IT LI LU NL SE**

(56) Entgegenhaltungen:
**EP-A- 0 088 540**
**EP-A- 0 088 622**
**EP-A- 0 115 613**
**EP-A- 0 161 504**
**EP-A- 0 186 098**
**EP-A- 0 254 593**

**JOURNAL OF INTERFERON RES., Band 2, Nr. 3, 1982, Seiten 363-370; T. YILMA et al.: "Preliminary characterization of equine interferons and their antiviral activities on bovine, ovine, and human cells"**

**Ann. Rev. Biochem. 56, p. 738, 1987**

**Ann. Rev. Biochem., vol. 59, p. 802, 1990**

**Nucleic Acid Hybridization, Oxford, 1985, S. 119**

**J. Mol. Biol. 185, pp. 227-260 (1985)**

(73) Patentinhaber: **BOEHRINGER INGELHEIM INTERNATIONAL GmbH**
**Postfach 20**
**W-6507 Ingelheim am Rhein(DE)**

(72) Erfinder: **Hauptmann, Rudolf, Dr.**
**Döllachstrasse 22**
**A-2483 Ebreichsdorf(AT)**
Erfinder: **Himmler, Adolf, Dr. Dipl.-Ing.**
**800 Memorial Drive 25**
**South San Francisco, CA 94080(US)**
Erfinder: **Swetly, Peter, Prof. Dr.**
**Hietzinger Hauptstrasse 40b**
**A-1130 Wien(AT)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services
(3.10/3.6/3.3.1)

## Beschreibung

Gegenstand der vorliegenden Erfindung ist ein Verfahren zur Herstellung von Pferde-Interferon-Gamma (equines Interferon-Gamma, EqIFN-γ), DNA-Sequenzen, die dieses Polypeptid codieren, geeignete Vektoren und Wirtsorganismen, die diese DNA-Sequenzen enthalten sowie das EqIFN-γ selbst. Gegenstand der Erfindung sind weiterhin DNA-Teilsequenzen, die Polypeptide codieren, die sich strukturell von dem natürlichen EqIFN-γ Polypeptid unterscheiden. Beschrieben wird außerdem die Verwendung der Proteine.

Interferone sind Proteine, die von eukaryotischen Zellen nach Virus Infektionen oder anderen Stimulationen sekretiert werden und ihrerseits die Zellen vor Virusinfektionen schützen können. Drei Klassen von Interferonen sind mittlerweile bekannt: bezeichnet werden sie mit Interferon-α- Interferon-β und Interferon-γ (abgekürzt IFN-α, IFN-β und IFN-γ). Sie unterscheiden sich in ihrem Aufbau und in ihren Wirkungen. So können Interferone regulierend die Zellen des Immunsystems, oder auch die Differenzierung von Zellen und das Wachstum von Tumoren beeinflussen.

F. Wheelock entdeckte 1965 ein Polypeptid, das bestimmte Zellen vor Virusinfektionen schützte (Science 149, 310 (1965). Polypeptide mit diesen Eigenschaften werden Immun-Interferon, TypII-Interferon, Interferon-Gamma oder IFN-γ bezeichnet, obwohl es sich hierbei um ein zur Klasse der Lymphokine zählendes Polypeptid handelt. Neben dem human-Interferon-γ sind bisher bekannt geworden, bovines-, murines- und Ratten-Interferon-γ. Alle bisher bekannt gewordenen γ-Interferone liegen in glykosilierter Form vor, wobei die Glykosilierung jedoch keinen Einfluß auf die biologische Aktivität ausübt (Keller et al., J. Biol. Chem. 258, 8010 (1983)).

Lange Zeit war angenommen worden, daß die Interferone speziespezifisch wirken. In vitro- Versuche zeigten jedoch, daß IFN-Präparate von Rindern eine antivirale Wirkung beim Affen und beim Menschen auslösen können (Tovey, M.G. et al. J. Gen. Virol 36, 341-344 (1977). Diese Speziesinteraktivität hängt möglicherweise mit der mehr oder weniger großen Homologie der Gene bzw. der Proteine zusammen: aufgrund der geringen Mengen an tierischen Interferonen konnte diese Annahme nicht überprüft werden.

Trotz der festgestellten Speziesinteraktivitäten sind Nebenwirkungen wie Antigenitäten bei der Anwendung speziesfremder Interferone zu beobachten, die für eine Therapie nicht zu tolerieren sind.

Da andererseits jedoch die Nutz- und Haustierhaltung einen bedeutenden wirtschaftlichen Wert darstellt, besteht ein Bedarf an Interferonen für die verschiedenen Spezies, die der Veterinärmediziner anwenden kann.

Hochgereinigtes Tierinterferon der verschiedenen Spezies würde darüberhinaus die willkommene Gelegenheit bieten, die Wirkmechanismen für Interferone zu untersuchen, um zu Modellvorstellungen zu kommen, die auf den Menschen zu übertragen sind.

Die ersten Untersuchungen mit tierischen Interferonen wurden mit Präparationen aus natürlichem Zellmaterial durchgeführt; Ausbeute und Reinheit der nach diesem Verfahren hergestellten Interferone lassen sie als ungeeignet für die Herstellung von Arzneimitteln erscheinen.

Durch die Entwicklung der rekombinanten DNA-Technik ist es möglich, von Mikroorganismen heterologe Proteine produzieren zu lassen. So hergestellt wurden beispielsweise auch Human-Interferone (Hu-IFN); seit neuestem auch verschiedene nicht-humane Interferone. EP-A 88 622 offenbart α-, β- und γ-Interferone des Rindes sowie DNA-Moleküle, die für diese Interferone kodieren. EP-A 186 098 beschreibt α-, β- und ω-Interferone des Pferdes sowie sie kodierende DNA-Moleküle. Diese Interferone faßt man gelegentlich zu Typ I-Interferonen zusammen, weil sie zueinander eine hohe Homologie aufweisen und -zumindest IFNα und -β- an den gleichen Rezeptor binden. Sie haben jedoch nur eine sehr geringe Homologie zu IFNγ, das an einen anderen Rezeptor bindet und als Typ II-Interferon bezeichnet wird (Gray et al.; Nature 295, 503-508 (1982)). Yilma et al. wiesen säurelabile antivirale Aktivität in Überständen von mononuklären Blutzellen des Pferdes nach Stimulation mit Phytohämagglutinin nach, ohne diese Aktivität näher zu charakterisieren oder das Protein zu reinigen (Yilma et al., J. Interferon Res. 2, 363-370 (1982)).

Eine Aufgabe der vorliegenden Erfindung war es, das Pferde-Interferon-γ auf gentechnologischem Weg herzustellen und die dafür erforderlichen DNA-Sequenzen bereitzustellen.

Gelöst wurde diese Aufgabe erfindungsgemäß durch die Anwendung der sogenannten Sondentechnik. Als Sonde wurde eine von dem humanen γ-Interferon abgeleitete, literaturbekannte DNA-Sequenz verwendet (Gray und Goeddel; Nature 298, 859-863 (1982)).

Ebensogut sind jedoch auch Teil- oder vollständige Sequenzen der anderen γ-Interferone als Sonden geeignet. Als Ausgangsmaterial bei der Suche diente eine aus normalem Lebergewebe des Pferdes stammende DNA-Bibliothek. Erstmals konnte hierdurch das für EqIFN-γ codierende Gen mit den flankierenden Bereichen der nachfolgenden Formel I isoliert werden:

GGATC 5

CCACAAGAATGGCACGGGTGGGCATAATGGGTCTGTCTCATCGTCAAAAGACCCAAGGAG 65

TTGAAAGGAAACTCTAACTACAACACCAAAATGCCACAAAACCATAGTTATTAATACAAA 125

CTAACTAGCATCTGTGCCTATCTGTCACCATCTCATCTGAAAAAACTTGTGAAAATACGT 185

AATCCTGATGAGACTTCAATTAGGTATAAAAACCAGCCCCAGAAGGCGAGGCAGTACACT 245

CTTCTGATCGCCGGTAGGGCAGCTATTAGAAAAGAAAGATCAGCTGAGGCCTTGGGACCT 305

GATCAGCTTAGTACAGAAGTGACTGCTTTCAACTACTTAGGCCTAACTCTCTCCGAAACA 365

-20 -15 -10

Met Asn Tyr Thr Ser Phe Ile Leu Ala Phe Gln Leu Cys Ala Ile

ATG AAT TAT ACA AGT TTT ATC TTG GCT TTT CAG CTG TGT GCG ATT 410

-5 -1 1 5 10

Leu Gly Ser Ser Thr Tyr Tyr Cys Gln Ala Ala Phe Phe Lys Glu

TTG GGT TCT TCT ACC TAT TAC TGC CAG GCC GCG TTT TTT AAA GAA 455

15

Ile Glu Asn Leu Lys Glu Tyr Phe

ATA GAA AAC CTA AAG GAA TAT TTT GTAAGTATGACCTTTTAATAATACTTAC 507

I N T R O N 1

TTGTGGTTGAAATGACTGAACGTTGTCTTGGAGTTGGATCTCTGATAGGCTGTCCTCTCT 567

ACTCCACAGTCATCTTGAGAAGACTGGGTGTTATTTTCTCTGTTTGTTGACTGGATGAGT 627

TTTTCTTTTTTTTACTAAATGATCTAGATATTGCTTTAACCCTCTGCTCAATTTGCTATA 687

GAGACTTAGAGAGGGTTCATGAATCTTCCAAAAGATGGGCTTAACAGGTTTATAAAGCAT 747

AGTGAAGTTGACAATTTTGTGGTGAGAAGCCACTGAATTGTGATAAGTCAAGTAGTGTGG 807

ACATTGAAAAAATGACTAGCTATTAGTTTCTAACTTCTCAGGTTACTATGATGGTGACAA 867

TAAAAGGTCAAGATTAGCATTAAAATGGTAATCTGAAATAATTGATCAGTTAAAGAAGGC 927

GCTGTCCTGAAAGGTTTGGCTGAAAAAAAATCACTTTCAGGTGTTTTCCTCCAAAAAATG 987

ATTTTAAAATCTTACTGCCCCGTTTGTGTTAGCTGTGAAGTACTCTGGAACTCAGTCAAT 1047

TGCTGAGATTTTGTACGAGTTATAAGCTGGCTTATATTTAAAAAATTTTTTTGTTTTTGT 1107

TTTATGAGTTTCTTTTAAAATGTTATTTATGGTTAATTAAAATAGTTTTTGCATTTTAAA 1167

TATTTTATTATTTGTCCAAAATTTAGCTATTTTAATTATAGTTGGAGCTCTCTTTTAGAG 1227

CTGACATAAGACCATAGGGGAGGCACAGATAGATGTGATGGAGCCCTGTACCAGACGGGG 1287

GCAGTATCTTATAGTGGGTTGCCTTTGCTGATCTTTTTACTAGACTTGAAATTATTTGCT 1347

TTTCCTTCCTATGGTTATTTGGGACTATTGAAGTATCACCAGCCCTGTTGAGTTCATCTG 1407

TAATATTGTAATTCAAGGGTTACACTAGAAAATAAGAAAGCTAAAACAGCACGATAATCT 1467

TTGGCTACATCCAACACAATAGCTTTTGGGAATACTTATTGTTAGAACTAAACAGAGGGT 1527

TGAAAAGAAAATCAGTGAATACTGTCAGCATCTGAGTTCAATAAAACGTGAAGTACATTT 1587

20

Asn Ala

TTAGGGCAATTCATGGACTAATTGTAAACCAAGTTTTCCTTCCTTTTTCAG AAC GCA 1644

25 30 35

Arg Asn Pro Asp Val Gly Asp Gly Gly Pro Leu Phe Leu Asp Ile

AGA AAC CCA GAT GTA GGG GAT GGT GGG CCT CTT TTC CTG GAT ATC 1689

40 I N T R O N 2

Leu Lys Asn Trp Lys Glu

TTG AAG AAC TGG AAA GAG GTAAGCTAAGTATTTCCATTTGGTTGATTTTCCTGT 1743

TGCTTATTTTCTGGTGGATGAATTCACACCAACCTCTCTTTGTGCTCTTTTCTCCCTAG 1802

45 50 55

Asp Ser Asp Lys Lys Ile Ile Gln Ser Gln Ile Val Ser Phe Tyr

GAT AGT GAC AAA AAA ATA ATT CAG AGC CAA ATC GTC TCC TTC TAC 1847

60 65 70

Phe Lys Leu Phe Glu Asn Leu Lys Asp Asn Gln Val Ile Gln Lys

TTC AAA CTC TTT GAA AAC TTG AAA GAT AAC CAG GTC ATT CAA AAG 1892

```
              75                        80                        85
Ser Met Asp Thr Ile Lys Glu Asp Leu Phe Val Lys Phe Phe Asn
AGC ATG GAC ACC ATC AAG GAG GAC CTG TTC GTT AAG TTC TTT AAC     1937

              90                        95                        100
Ser Ser Thr Ser Lys Leu Glu Asp Phe Gln Lys Leu Ile Gln Ile
AGC AGC ACC AGC AAG CTG GAA GAC TTC CAA AAG CTG ATT CAG ATT     1982

               I N T R O N
Pro
CCG GTGAGGAGATCTTAATTCTTTCTTTGGTTTCATTACAGAGGTTCTTGCAAAGTGCT   2041
TACGTCCCAGAAAGTAGAAATGAACTATGAAATGAACCCGTGGCCAAAACTCCTCCTTCC   2101
TAATTCCATTTGTGCTTTGAGAGACTTTGCTAAGTCAGTATGGGAATCATTTAAATTTGT   2161
GATTTGGGGAAATGCTGGCACTATGACTACTGCACAAAGGCAGGTGAAGGGACAAATCCA   2221
GTGAGGAGGGGGCAGTGAAGAAGTGGAGGGGAGTCTGGAGAAGCAGGTCTCTCCTTGCCC   2281
CTTGTTCGTGAGATGAAATCCTCCTGCTTTGGATGGGAGGCTGCGTGTCTTGGTGGAAAG   2341
AGCAGTGGGAGGAGGGAGAAGATTTGTGCTCCTCCCAGCTCAGCCACCAAGAAACTGTGA   2401
CCTCAGATGAATCACAGGCCTGGCTGGGGCTCAGTTTCCTCATCTTAAAAGAGGCCTATT   2461
GGGTTCACTAAAATTTCTATGATCTTCTTTGCTCTATAATCCTACAATTCTGTGGACAGA   2521
AAATGAAATGAGGTAGGAGAAAGAAATAGCCTTTGAAGAGGTTCTTGGGCATTCCACTGC   2581
CAGGCTCTGGTCAACCTTCATACTCTGCAGCCCAAGAAGAGGCAAGACCATTTGTCTGTT   2641
TTTGGAAATGCAAATAGGCGGCATTTATACCTCACGAAAGAACTGTTCTGTCAACTTTTG   2701
GATACTGGGCTATCTTGGCTGGAGAAATCCTTAGGCTCCCAAACTTTCTCTCATGAAATT   2761
GTCTTGAGTCTTTAAATTTATGGCTTCTCGAAGCTGAGAGATAACTTTAAGCATAAAGAC   2821
AAATTACATTTTCCAACATTTTGTCTAAGAGACAAAGACCTCCACATGCCTTTGGGTTTG   2881
GCCTGGATCTAAATGGGCTTGAATGAGAAGGGGAGGGTGTTGTTATGACTATGTTTAGAA   2941
GAGAAAACAGAGGTTTGGAGAGGTTAAGTGGCTGGTTCAAAGTCAGAGTTATTGCACACA   3001
CAGGATTCGAACCCATATGTTTTGTCCCTCCACTTTAGGGTTCTTTTCGCTACATAATTT   3061
TGAGAATTCTGTACCAGTCAATTTAAGGATGTGTGATGTTCCCCATCCTATTACAGCACA   3121
ACCAGCAATTTAATTATAATTTTAGTCTTAACTGCTGAAGAAAGCAGCATTACATATTAA   3181
GCTAACATATTCCTGGTGAAAGCAACTTTTTCAAAGGAATATTTCTATTTTCATGGACCA   3241
TGACAGTAGCACAGCCTGATGGCTTGTATGCCTGAAACTAATTTTGCTGTTTTCTTTCCC   3301

                  105                       110                       115
      Val Asn Asp Leu Lys Val Gln Arg Lys Ala Ile Ser Glu Leu
AATAG GTA AAT GAT CTG AAG GTC CAG CGC AAA GCA ATA AGT GAA CTC  3348

             120                       125                       130
Ile Lys Val Met Asn Asp Leu Ser Pro Lys Ala Asn Leu Arg Lys
ATC AAA GTG ATG AAT GAT CTG TCG CCC AAA GCT AAC CTG AGG AAG     3393

             135                       140                       145
Arg Lys Arg Ser Gln Asn Pro Phe Arg Gly Arg Arg Ala Leu Gln
CGG AAG AGG AGT CAG AAT CCA TTT CGA GGC CGG AGA GCG TTG CAA     3438

***
TAG TGGTCATCCTGCCTGCAATATTTGAATTTTTAAATCTAAATCTATTTATTAATATT   3497
TAATATTTTACATTATTTATATGGGGAATATATTTTTAAACTCATCAAAGTATTTATAAT   3557
AGCAACTTTTATGTAATGAAAATGGGTATCTATTAATATATATATTATTTATAATTCCTG   3617
TATGGTGTGACTATTTCACTTGACCCTTTTTTTTCTGACGAACTAGGCAAGATTATGTGA   3677
TTACAAGGCTTTAACTCAGGGGCCAACTAGGGAGTGGGTAGCCGACCTACCAAGACCCTG   3737
TGAGCTGTGTGTTTATTTCCCTCAATGATACAATGAACACTTATAAAGGAAAGGAGGGCC   3797
TCCAGTCACTGCCTGTTGGAGAACATGTCTGCATTGTGAGCCACTGCTTAATGGCATGTC   3857
```

```
AAACCACGCTTGAATGTGTCAGATGATAGGGCTTGTCCCCTGATAAAGCTTAGTATCTCC  3917
TCTCATGCCTAGTGCTTCAGAATATTGTTGACAACTGTGACTGCACCCAAATGGAAAGTA  3977
ATTTATTTGTTTAGTTTACCAATATTTAATAAATATGAATAAAGTATAATTTCATAACTA  4037
TTTATGCTGCGTCCGGCTTTTTCTAAGTGAGGACTGGGGTAAATGAACTACAAACTAATG  4097
AATCAGTAAGAGGGAACTCGTTTTTAGCGGTGGAAATCTTAGCTGGATTAAGCCCCATGA  4157
AACGTGGTATTTCTCTCCACTGGAGATTTGTTGGCTACTACTCCTCCATGTAGCAGCTCT  4217
TTATCTTTCCAAAATATAAATTTAATTATGTCACCATTTACTTCAGAGCTTCTGCGATGG  4277
AAAGTAGTTCAAATAGTTTAGCTTAGCACACAAAGCTTTGTTTCTCCCTCCTCCCTCAAC  4337
TCTGCACTGTGCTCTTCATCTTGGTGTCCCCACGTCCTCTGTCCACTTCGGGCAAACCAC  4397
CGGGAATGTCATGGTGAGGGTGAGCTCTAGGGAGAGAGGGCTGGATTAGAATTTCGGCCC  4457
CACCATTACCAGTAGTATGACCTTTAATGAATTACTTGTATTCTCTAAGCTCCAGTTTCC  4517
TCATCTGACACAAGAGAATAATTGTGCCTAAAATTGTGGTGAGAGTTTGTTCTTTCACTC  4577
AAGAAGTGTTTACTGGAGCATCCACTAGTTGCCTAGTGCTGTTCTAGGCACTTGAGATAC  4637
ATTTGTGAACAAAATAGTCAAGGATCC                                   4664
```

Bemerkenswert ist, daß EqIFN-γ ebenso wie alle bisher bekannt gewordenen γ-Interferone im jeweiligen Organimus von einem Gen codiert wird, das große Sequenzen aufweist, die das Strukturgen unterbrechen: die Gene bestehen aus Exons und Introns, wobei nur die Exons für das Protein codieren. Die Introns können nur von bestimmten Systemen, beispielsweise von Systemen in Säugetierzellen, verstanden werden. Für andere Systeme beispielsweise in E.coli, sind DNA-Sequenzen, die Introns enthalten, nicht zu verwenden.

Eine weitere Aufgabe der vorliegenden Erfindung war es daher, eine für EqIFN-γ codierende intron-freie DNA-Sequenz bereitzustellen.

Die Lösung dieser Aufgabe ist prinzipiell auf zwei Wegen möglich.

1. Auf dem Weg vom Zellkern, in dem die Transkription stattfindet, in das Cytoplasma werden die Introns ausgeschnitten und durch Spleißen der Exon-RNA-Fragmente entsteht die mRNA des eukaryotischen Proteins. Diese mRNA läßt sich mit einem Enzym, der Reversen Transcriptase in eine DNA umkopieren, die als "copy-DNA" (cDNA) bezeichnet wird.

```
  1               5                  10                  15
TAT TAC TGC CAG GCC GCG TTT TTT AAA GAA ATA GAA AAC CTA AAG
                 20                  25                  30
GAA TAT TTT AAC GCA AGA AAC CCA GAT GTA GGG GAT GGT GGG CCT
                 35                  40                  45
CTT TTC CTG GAT ATC TTG AAG AAC TGG AAA GAG GAT AGT GAC AAA
                 50                  55                  60
AAA ATA ATT CAG AGC CAA ATC GTC TCC TTC TAC TTC AAA CTC TTT
                 65                  70                  75
GAA AAC TTG AAA GAT AAC CAG GTC ATT CAA AAG AGC ATG GAC ACC
                 80                  85                  90
ATC AAG GAG GAC CTG TTC GTT AAG TTC TTT AAC AGC AGC ACC AGC
                 95                 100                 105
AAG CTG GAA GAC TTC CAA AAG CTG ATT CAG ATT CCG GTA AAT GAT
                110                 115                 120
CTG AAG GTC CAG CGC AAA GCA ATA AGT GAA CTC ATC AAA GTG ATG
                125                 130                 135
AAT GAT CTG TCG CCC AAA GCT AAC CTG AGG AAG CGG AAG AGG AGT
                140                 145
CAG AAT CCA TTT CGA GGC CGG AGA GCG TTG CAA TAG
                                        Formel II
```

Diese intron-freie DNA kann dann in geeignete Plasmide inseriert werden, die dann zusammen mit geeigneten Wirtsorganismen, beispielsweise E.coli, zur Produktion von eukaryotischen Proteinen im vorliegeneden Fall von EqIFN-γ verwendet werden können. Nachteilig kann sich bei dieser Vorgehensweise die Degeneration des genetischen Codes auswirken. Diese Degeneration führt nämlich dazu, daß verschiedene Organismen für dieselben Aminosäuren unterschiedliche Codons verwenden. Bei nicht optimaler Anpassung der verwendeten DNA kann es daher zu einer verschlechterten Expression eines eukaryotischen Proteins in einem prokaryotischen System kommen.

2. Die andere Möglichkeit, eine intron-freie DNA Sequenz für ein eukaryotisches Gen zu erhalten, besteht darin, bei Kenntnis der chromosomalen DNA-Sequenz eine intron-freie DNA-Sequenz chemisch zu synthetisieren. Dabei kann man die DNA-Sequenz für die bevorzugte Codon-Benutzung eines bestimmten Wirtsorganismus, z.B. E. coli, optimieren. Eine solche Strategie wurde für humanes IFN-γ in der EP-A 161 504 beschrieben.

Als besonders geeignet zur Lösung der erfindungsgemäßen Aufgabe erwies sich die DNA-Sequenz gemäß Formel III

```
 -1   1               5                  10                  15
ATG TAC TAC TGC CAG GCT GCT TTC TTT AAA GAA ATC GAA AAC CTG AAA
                 20                  25                  30
GAA TAC TTC AAC GCT CGT AAC CCA GAC GTT GGT GAC GGT GGT CCG
                 35                  40                  45
CTG TTC CTG GAC ATC CTG AAA AAC TGG AAA GAA GAC TCT GAC AAA
                 50                  55                  60
AAG ATC ATC CAG TCT CAG ATC GTT TCT TTC TAC TTC AAA CTG TTC
                 65                  70                  75
GAA AAC CTG AAA GAC AAC CAG GTT ATC CAG AAA TCG ATG GAC ACT
                 80                  85                  90
ATC AAA GAA GAT CTG TTC GTT AAA TTC TTC AAC TCG TCG ACT TCT
                 95                 100                 105
AAA CTG GAA GAC TTC CAG AAA CTG ATC CAG ATC CCA GTT AAC GAC
                110                 115                 120
CTG AAA GTT CAG CGT AAG GCT ATC TCT GAA CTG ATC AAA GTT ATG
                125                 130                 135
AAC GAC CTG TCT CCA AAA GCT AAC CTG CGT AAA CGT AAA CGT TCT
                140                 145
CAG AAC CCA TTC CGT GGT CGT CGT GCT CTT CAG TAA
```

Formel III

die nach an sich bekannten Verfahren hergestellt werden kann.

16 verschiedene Oligonukleotide wurden in zwei Varianten synthetisiert. Die erste vollständige Variante codiert für reifes Eq-IFN-γ mit 146 Aminosäuren plus Startmethionin (Formel III), die zweite für ein am Amino-Terminus um 3 Aminosäuren verkürztes Polypeptid plus Startmethionin.

```
-1  1               5                   10                  15
ATG CAG GCT GCT TTC TTT AAA GAA ATC GAA AAC CTG AAA GAA TAC TTC
                20                  25                  30
AAC GCT CGT AAC CCA GAC GTT GGT GAC GGT GGT CCG CTG TTC CTG
                35                  40                  45
GAC ATC CTG AAA AAC TGG AAA GAA GAC TCT GAC AAA AAG ATC ATC
                50                  55                  60
CAG TCT CAG ATC GTT TCT TTC TAC TTC AAA CTG TTC GAA AAC CTG
                65                  70                  75
AAA GAC AAC CAG GTT ATC CAG AAA TCG ATG GAC ACT ATC AAA GAA
                80                  85                  90
GAT CTG TTC GTT AAA TTC TTC AAC TCG TCG ACT TCT AAA CTG GAA
                95                  100                 105
GAC TTC CAG AAA CTG ATC CAG ATC CCA GTT AAC GAC CTG AAA GTT
                110                 115                 120
CAG CGT AAG GCT ATC TCT GAA CTG ATC AAA GTT ATG AAC GAC CTG
                125                 130                 135
TCT CCA AAA GCT AAC CTG CGT AAA CGT AAA CGT TCT CAG AAC CCA
                140             143
TTC CGT GGT CGT CGT GCT CTT CAG TAA
```

**Formel IIIa**

Beide Varianten lassen sich leicht dadurch modifizieren, daß man anstelle des für Methionin codierenden ATG eine für ein hydrophobes Signalpeptid codierende Sequenz, beispielsweise der Formel IV anknüpft.

```
ATG AAT TAT ACA AGT TTT ATC TTG GCT TTT CAG CTG TGT GCG ATT

TTG GGT TCT TCT ACC
```

**Formel IV**

Durch eine solche Signalsequenz wird in bestimmten Wirtsorganismen die Sekretion des gewünschten Polypeptide aus dem Cytoplasma bewirkt. Hierbei wird das Protein prozessiert und das Signalpeptid abgespalten; man erhält das reife Protein. Zellen von Wirtsorganismen, z.B. E.coli, die nicht in der Lage sind, Polypeptide, die die Signalpeptidsequenz enthalten, zu prozessieren, müssen aufgeschlossen werden um das "unreife" Polypeptid zu isolieren. Auch diese "unreifen" EqIFN-γ's mit vollständigen oder unvollständigen Signalpeptidsequenzen sind Gegenstand der vorliegenden Erfindung.

Das Startmethionin läßt sich durch an sich bekannte Verfahren beispielsweise mit CNBr oder CNCl abtrennen um reifes EqIFN-γ zu erhalten.

Diese DNA-Sequenz codiert für EqIFN-γ, enthält jedoch ausschließlich solche Codons, die in von E.coli hoch exprimierten, zelleigenen Genen verwendet werden (Gouy und Gautier, Nucl. Acids Res. 10, 7055 (1982)).

Eine Aufgabe der vorliegenden Erfindung bestand weiterhin darin, erstmals das EqIFN-γ in homogener, reiner Form bereitzustellen.

Wie bereits erwähnt, ist die Isolierung und Reinigung des EqIFN-γ aus natürlichem Zellmaterial nicht geeignet, diese Aufgabe zufriedenstellend zu lösen. Erfindungsgemäß werden daher die DNA-Sequenzen gemäß Formel II, III und IIIa zur Lösung dieser Aufgabe verwendet. Diese Sequenzen werden, mit entsprechenden Kontrollsequenzen versehen, in geeignete Vektoren eingebaut und damit transformierte geeignete Wirtsorganismen oder Wirtszellkulturen kultiviert. Die gebildeten Polypeptide werden nach an sich bekannten Verfahren isoliert und gereinigt.

Die erhaltenen Polypeptide entsprechen der nachfolgenden Formel:

| | | | | | | | | | | | | | | |
|---|---|---|---|---|---|---|---|---|---|---|---|---|---|---|
| 1 | | | | 5 | | | | | 10 | | | | | 15 |
| Tyr | Tyr | Cys | Gln | Ala | Ala | Phe | Phe | Lys | Glu | Ile | Glu | Asn | Leu | Lys |
| | | | | 20 | | | | | 25 | | | | | 30 |
| Glu | Tyr | Phe | Asn | Ala | Arg | Asn | Pro | Asp | Val | Gly | Asp | Gly | Gly | Pro |
| | | | | 35 | | | | | 40 | | | | | 45 |
| Leu | Phe | Leu | Asp | Ile | Leu | Lys | Asn | Trp | Lys | Glu | Asp | Ser | Asp | Lys |
| | | | | 50 | | | | | 55 | | | | | 60 |
| Lys | Ile | Ile | Gln | Ser | Gln | Ile | Val | Ser | Phe | Tyr | Phe | Lys | Leu | Phe |
| | | | | 65 | | | | | 70 | | | | | 75 |
| Glu | Asn | Leu | Lys | Asp | Asn | Gln | Val | Ile | Gln | Lys | Ser | Met | Asp | Thr |
| | | | | 80 | | | | | 85 | | | | | 90 |
| Ile | Lys | Glu | Asp | Leu | Phe | Val | Lys | Phe | Phe | Asn | Ser | Ser | Thr | Ser |
| | | | | 95 | | | | | 100 | | | | | 105 |
| Lys | Leu | Glu | Asp | Phe | Gln | Lys | Leu | Ile | Gln | Ile | Pro | Val | Asn | Asp |
| | | | | 110 | | | | | 115 | | | | | 120 |
| Leu | Lys | Val | Gln | Arg | Lys | Ala | Ile | Ser | Glu | Leu | Ile | Lys | Val | Met |
| | | | | 125 | | | | | 130 | | | | | 135 |
| Asn | Asp | Leu | Ser | Pro | Lys | Ala | Asn | Leu | Arg | Lys | Arg | Lys | Arg | Ser |
| | | | | 140 | | | | | 145 | | | | | |
| Gln | Asn | Pro | Phe | Arg | Gly | Arg | Arg | Ala | Leu | Gln | | | | |

Formel V

bzw.

| | | | | | | | | | | | | | | |
|---|---|---|---|---|---|---|---|---|---|---|---|---|---|---|
| | | | Gln | Ala | Ala | Phe | Phe | Lys | Glu | Ile | Glu | Asn | Leu | Lys |
| Glu | Tyr | Phe | Asn | Ala | Arg | Asn | Pro | Asp | Val | Gly | Asp | Gly | Gly | Pro |
| Leu | Phe | Leu | Asp | Ile | Leu | Lys | Asn | Trp | Lys | Glu | Asp | Ser | Asp | Lys |
| Lys | Ile | Ile | Gln | Ser | Gln | Ile | Val | Ser | Phe | Tyr | Phe | Lys | Leu | Phe |
| Glu | Asn | Leu | Lys | Asp | Asn | Gln | Val | Ile | Gln | Lys | Ser | Met | Asp | Thr |
| Ile | Lys | Glu | Asp | Leu | Phe | Val | Lys | Phe | Phe | Asn | Ser | Ser | Thr | Ser |
| Lys | Leu | Glu | Asp | Phe | Gln | Lys | Leu | Ile | Gln | Ile | Pro | Val | Asn | Asp |
| Leu | Lys | Val | Gln | Arg | Lys | Ala | Ile | Ser | Glu | Leu | Ile | Lys | Val | Met |
| Asn | Asp | Leu | Ser | Pro | Lys | Ala | Asn | Leu | Arg | Lys | Arg | Lys | Arg | Ser |
| Gln | Asn | Pro | Phe | Arg | Gly | Arg | Arg | Ala | Leu | Gln | | | | |

Formel Va

Bei Verwendung der chromosomalen Sequenz (Formel II) erhält man nach Transformation von Säugetierzellen das EqIFN-γ in der natürlich vorkommenden, glykosylierten Form (authentisches EqIFN-γ).
Die Sequenzen gemäß Formel II, III bzw. IIIa sind besonders geeignet bei der Herstellung des EqIFN-γ's in Mikroorganismen, insbesondere bei der Herstellung des EqIFN-γ's in E.coli, wobei das Polypetid in nichtglykosylierter Form exprimiert wird.

Eine weitere Aufgabe der vorliegenden Erfindung bestand darin, Modifikationen des natürlichen EqIFN-γ's bereitzustellen.

Modifikationen eines Proteins kann man herstellen, indem beispielsweise entweder das Protein derivatisiert oder durch Enzymverdau fragmentiert wird oder die das Protein codierende DNA-Sequenz durch Deletion oder Fragmentierung modifiziert und diese Sequenz in einem geeigneten Wirtsorganismus zur Expression gebracht wird.

Erfindungsgemäß werden die für Modifikationen des EqIFN-γ codierenden DNA-Sequenzen chemisch synthetisiert. Um eine einfache Manipulation des Gens für Veränderungen einzelner Abschnitte möglich zu machen, werden in die vollständige DNA-Sequenz mehrere singuläre Restriktionsenzym-Schnittstellen eingebaut.

Weitere modifizierte Formen des EqIFN-γ's erhält man erfindungsgemäß dadurch, daß die verschiedenen Oligonukleotide allein oder in verschiedenen Kombinationen, mit den entsprechenden Kontrollsequenzen versehen in geeignete Vektoren eingebaut werden, damit transformierte Wirtsorganismen kultiviert und die gebildeten Proteine isoliert und gereinigt werden.

Zur Lösung der gestellten Aufgaben wurde hochmolekulare DNA aus einem Gewebe von Pferden, vorzugsweise aus der Leber, nach einem modifizierten Verfahren gemäß Blin und Stafford (Blin, N.; Stafford, P.W. Nucl. Acids Res. (1976), 3 2303-2308) isoliert und mit Hilfe spezieller Endonucleasen statistisch fragmentiert. Die so erhaltenen unterschiedlich großen Fragmente wurden nach der Größe fraktioniert, vorzugsweise zur Bildung von 10-23 kb Fragmenten, um in einem Vektor, beispielsweise in einem Lambda-Vektor beispielsweise Lambda EMBL3A geklont zu werden. Anschließend wurden diese Vektoren nach Transformation in eimen Wirtsorganismus, beispielsweise E.coli, vermehrt. Diese equine DNA-Bibliothek wurde mit Hilfe einer humanen gamma-Interferon "probe" unter nicht stringenten Hybridisierungsbedingungen durchsucht. Die niedrige Stringenz ermöglicht das Auffinden von DNA Sequenzen, die Unterschiede zu der "probe" aufweisen.

Sie kann entweder durch Verdauung literaturbekannter Plasmide mit Hilfe von Restriktionsenzymen oder durch chemische Synthese mittels an sich bekannter Oligonukleotidsyntheseverfahren hergestellt werden. Diese "probe" weist die für HuIFN-γ kodierende Sequenz auf. Fünf Lambda Klone konnten identifiziert werden, die positive Hybridisierungssignale ergaben.

Aus diesen isolierten rekombinanten Phagen wurde nach üblichen Methoden die DNA gereinigt. Die Phagen-DNAs wurden nach Verdauung mit verschiedenen Restriktionsenzymen und nachfolgender Southern-Analyse (Southeren, J.Mol. Biol. 98: 503-517, 1975) durch Hybridisierung mit der HuIFN-γ "probe" charakterisiert. Ein singuläres hybridisierendes 4,6 kb langes BamHI Fragment des Klons Lambda Eq-γ2 wurde isoliert und in die BamHI Schnittstelle des Plasmids pUC9 (Vieira und Messing, Gene 19: 259-268, 1982) geklont.

Nach Transformation von E.coli beispielsweise JM101, wurde aus erhaltenen Kolonien im einem Minipräparationsverfahren (Birnboim und Doly, Nucl. Acids Res. 7: 1513-1523, 1979) Plasmid-DNA hergestellt und durch Verdauung mit Restriktionsenzymen charakterisiert. Ein Plasmid mit dem gewünschten BamHI Insert wurde pAH111 benannt. Die Ränder des BamHI Inserts von Plasmid pAH111 wurden nach Einbringung in die M13mp8 bzw. M13mp9 Vektoren (Vieira und Messing, Gene 19, 259-268, 1982) nach der Didesoxy-Methode sequenziert (Sanger et al., Proc. Natl. Acad. Sci. USA 74: 5463-5467, 1977). Ein Sequenzvergleich mit dem humanen gamm-Interferon-Gen (Gray und Goeddel, Nature 298: 859-863, 1982) zeigte hohe Homologie mit den nichtkodierenden 5′- und 3′-Regionen. Daraus wurde geschlossen, daß das vollständige EqIFN-γ Gen isoliert wurde.

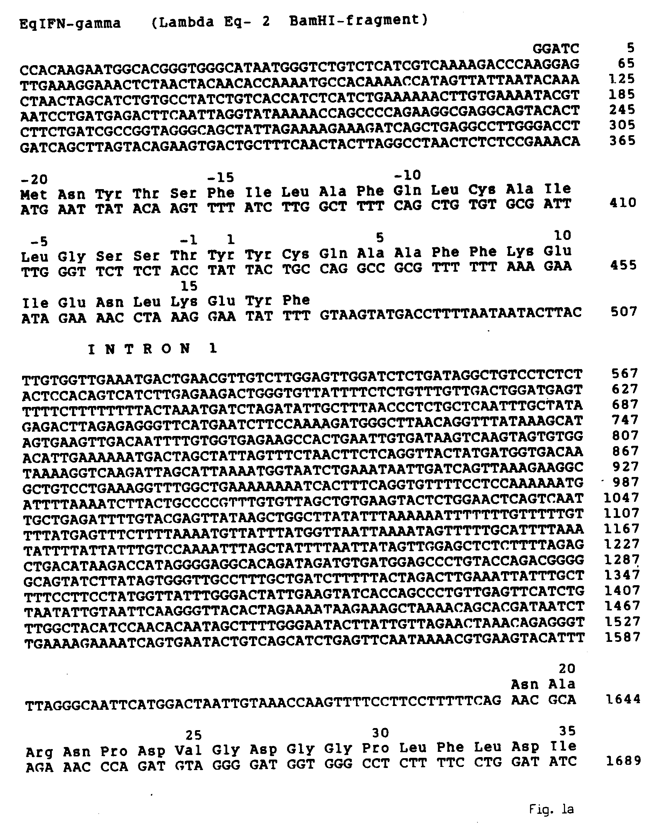

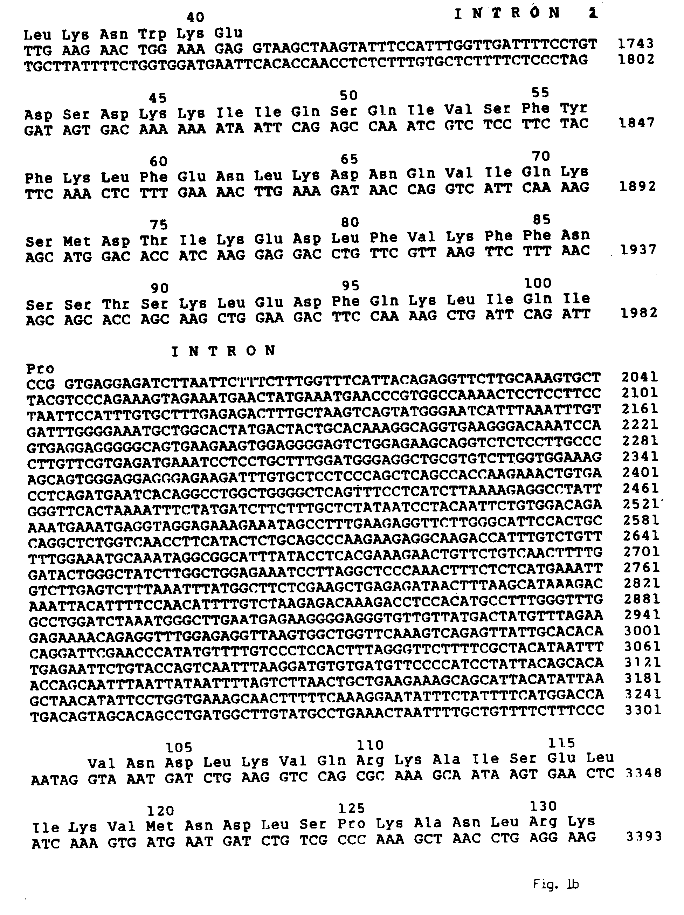

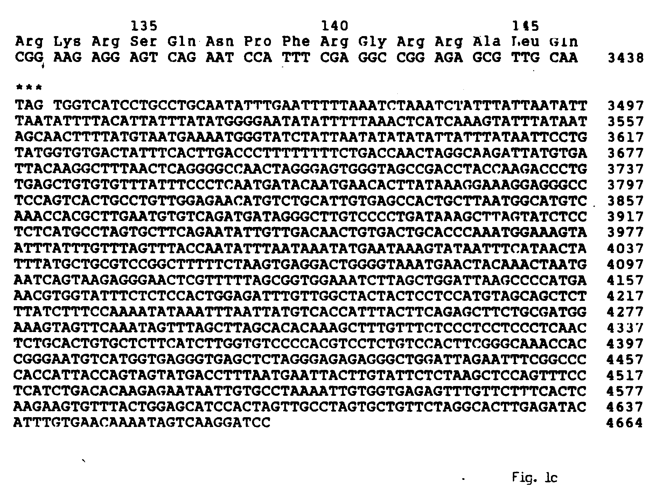

Das 4,6 kb lange BamHI Insert von Plasmid pAH111 wurde nach der Didesoxy-Methode vollständig sequenziert. Die Gesamtsequenz des BamHI-Fragments wurde durch Kombination von Teilsequenzen von M13-Subklonen ermittelt, die durch gerichtete Klonierung von Restriktionsfragmenten (EcoRI, HindIII, PstI, PstI-BglII, HindIII-BamHI) in entsprechend geschnittene M13mp8 oder M13mp9 Vektoren erhalten wurden. Weitere Teilsequenzen wurden erhalten, indem das 2,0 kb lange BamHI-BglII Fragment, bzw. das 2,0 kb lange PstI Fragment nach der "Shotgun-Methode" in den M13mp8 Vektor geklont wurde.
Die erhaltenen Teilsequenzen wurden mittels eines Computerprogrammes zu der 4664 bp langen Totalsequenz vereint, die in Fig. 1 dargestellt ist.

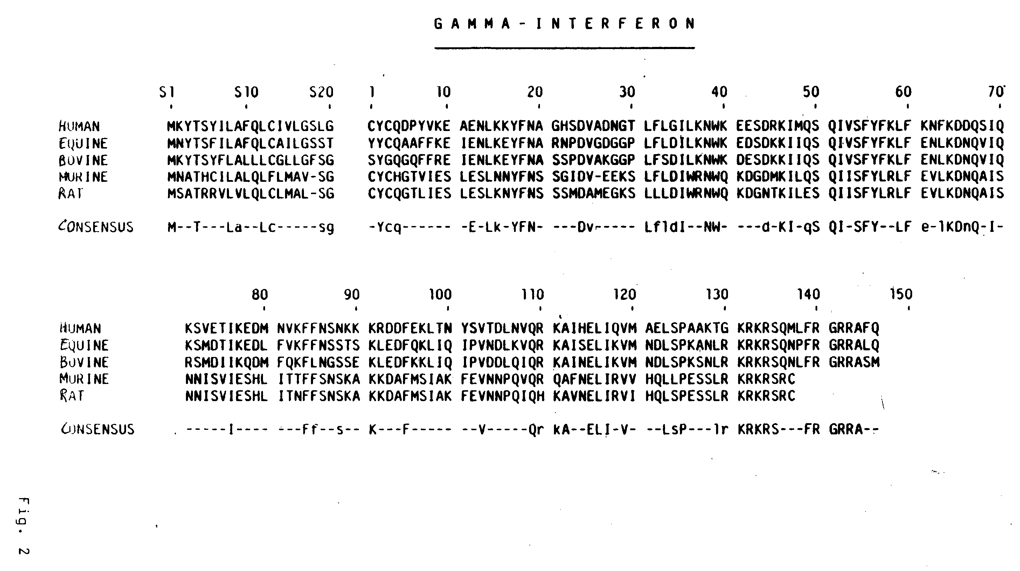

Durch computerunterstützte Analyse der offenen Leserahmen und Vergleich mit gamma-Interferongenen anderer Spezies (Gray und Goeddel, Nature 298: 859-863; Gray und Goeddel, Proc. Natl.Acad.Sci.USA 80: 5842-5846, 1983; Dijkema et al., EMBO J. 4: 761-767, 1985; Cerretti et al., J. Immunology 136: 4561-4564, 1986) wurde der proteinkodierende Bereich des equinen gamma-Interferongens ermittelt. Die proteinkodierende Region ist durch drei Introns unterbrochen, wobei im ersten Exon das 20 Aminosäuren lange, hydrophobe Signalpeptid und 18 Aminosäuren des reifen EqIFN-γ Polypeptides kodiert sind (Basen 366-479). Das zweite Exon kodiert für die Aminosäuren 19-41 (Basen 1639-1707), das dritte Exon kodiert für die Aminosäuren 42-102 (Basen 1803-1985), das vierte Exon kodiert den Carboxy-Terminus mit den Aminosäuren 103-146 (Basen 3307-3441). An den Positionen 4010 und 4020 befinden sich zwei Signalsequenzen (AATAAA) für die Polyadenylierung der mRNA. An den Positionen 86-88 des reifen EqIFN-γ Polypeptides befindet sich die einzige potentielle N-Glykosylierungsstelle (ASN-Ser-Ser), welche mit der zweiten N-Glykosylierungsstelle des bovinen gamma-Interferons (Asn-Gly-Ser) zusammenfällt (Fig. 2). Das reife EqIFN-γ Polypeptid enthält überraschenderweise nur einen einzigen Cysteinrest an Position 3, wobei in Analogie zu den natürlichen humanen und murinen gamma-Interferonen wahrscheinlich die ersten drei amino-terminalen Aminosäuren (in diesem Fall Tyr-Tyr-Cys) im Körper proteolytisch abgespalten werden.

Um rekombinantes EqIFN-γ in seiner reifen Form in Eschereichia coli zu exprimieren, wurde ein synthetisches Gen aus Oligonukleotiden konstruiert. Es kodiert für dieselbe Aminosäuresequenz wie das natürliche EqIFN-γ Gen, enthält jedoch ausschließlich solche Codons für die einzelnen Aminosäuren, die in von E.coli hoch exprimierten, zelleigenen Genen verwendet werden (Gouy und Gautier, Nucl. Acids Res. 10: 7055-7074, 1982). Zusätzlich wurden mehrere singuläre Restriktionsenzym-Schnittstellen eingebaut, die eine einfache Manipulation des Gens für die Veränderung einzelner Abschnitte möglich macht. Das synthetische Gen für EqIFN-γ wurde in zwei Varianten aus insgesamt 16 verschiedenen Oligonukleotiden konstruiert. Die erste Variante kodiert für reifes EqIFN-γ mit 146 Aminosäuren plus Start-Methionin, die zweite Form für ein am Amino-Terminus um 3 Aminosäuren (Tyr-Tyr-Cys) verkürztes Polypeptid plus Startmethionin, wie es vermutlich im natürlichen Organismus auftreten dürfte.

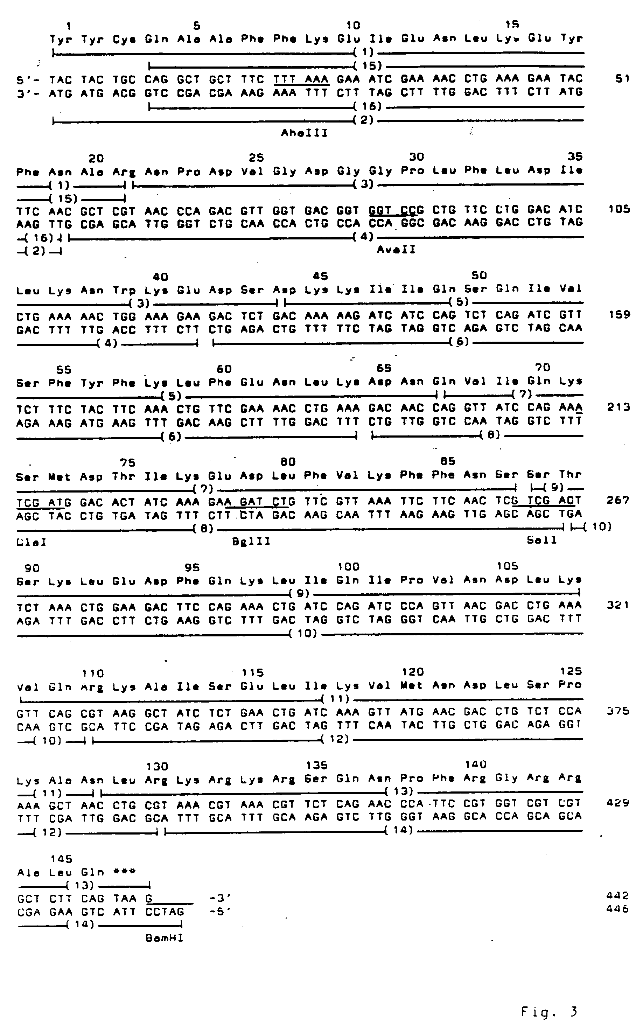

Die Struktur des synthetischen EqIFN-γ Gens ist in Fig. 3 dargestellt. Die für die Herstellung verwendeten Oligonukleotide wurden mit Hilfe eines Applied Biosystems Model 381A DNA-Synthesizer synthetisiert, durch Elektrophorese gereinigt und entsalzt. Die in Fig. 3 gekennzeichneten Oligonukleotide haben folgende Struktur:

| | | |
|---|---|---|
| EG-1 | 5'-TACTACTGCC AGGCTGCTTT CTTTAAAGAA ATCGAAAACC TGAAAGAATA CTTCAACGCT CG-3' | |
| EG-2 | 5'-TTGAAGTATT CTTTCAGGTT TTCGATTTCT TTAAAGAAAG CAGCCTGGCA GTAGTA-3' | |
| EG-3 | 5'-TAACCCAGAC GTTGGTGACG GTGGTCCGCT GTTCCTGGAC ATCCTGAAAA ACTGGAAAGA AGACTCTG-3' | |
| EG-4 | 5'-TTCTTTCCAG TTTTTCAGGA TGTCCAGGAA CAGCGGACCA CCGTCACCAA CGTCTGGGTT ACGAGCG-3' | |
| EG-5 | 5'-ACAAAAAGAT CATCCAGTCT CAGATCGTTT CTTTCTACTT CAAACTGTTC GAAAACCTGA AAGACAACC-3' | |
| EG-6 | 5'-TTTCAGGTTT TCGAACAGTT TGAAGTAGAA AGAAACGATC TGAGACTGGA TGATCTTTTT GTCAGAGTC-3' | |
| EG-7 | 5'-AGGTTATCCA GAAATCGATG GACACTATCA AAGAAGATCT GTTCGTTAAA TTCTTCAACT CG-3' | |
| EG-8 | 5'-TCGACGAGTT GAAGAATTTA ACGAACAGAT CTTCTTTGAT AGTGTCCATC GATTTCTGGA TAACCTGGTT GTC-3' | |
| EG-9 | 5'-TCGACTTCTA AACTGGAAGA CTTCCAGAAA CTGATCCAGA TCCCAGTTAA CGACCTGAAA-3' | |
| EG-10 | 5'-GCTGAACTTT CAGGTCGTTA ACTGGGATCT GGATCAGTTT CTGGAAGTCT TCCAGTTTAG AAG-3' | |
| EG-11 | 5'-GTTCAGCGTA AGGCTATCTC TGAACTGATC AAAGTTATGA ACGACCTGTC TCCAAAAGCT AA-3' | |
| EG-12 | 5'-CGCAGGTTAG CTTTTGGAGA CAGGTCGTTC ATAACTTTGA TCAGTTCAGA GATAGCCTTA C-3' | |
| EG-13 | 5'-CCTGCGTAAA CGTAAACGTT CTCAGAACCC ATTCCGTGGT CGTCGTGCTC TTCAGTAAG-3' | |
| EG-14 | 5'-GATCCTTACT GAAGAGCACG ACGACCACGG AATGGGTTCT GAGAACGTTT ACGTTTA-3' | |
| EG-15 | 5'-CAGGCTGCTT TCTTTAAAGA AATCGAAAAC CTGAAAGAAT ACTTCAACGC TCG-3' | |
| EG-16 | 5'-TTGAAGTATT CTTTCAGGTT TTCGATTTCT TTAAAGAAAG CAGCCTG-3' | |

Der Zusammenbau des synthetischen EqIFN-$\gamma$ Gens erfolgte in zwei Aubschnitten. Der erste Teil des Gens wurde mit Hilfe der achte Oligonukleotide EG-1 bis EG-8 bis zur SalI Schnittstelle hergestellt, die zweite Hälfte des Gens aus den sechs Oligonukleotiden EG-9 bis EG-14 von der SalI Schnittstelle bis zur BamHI Schnittstelle. Für die am Amino-Terminus um drei Aminosäuren verkürzte Form des EqIFN-$\gamma$ wurden statt der Oligonukleotide EG-1 und EG-2 die Oligonukleotide EG-15 und EG-16 verwendet.

Gegenstand der Erfindung sind nicht nur Gen-Sequenzen, die spezifisch für die erfindungsgemäßen Interferone codieren, sondern ebenfalls Modifikationen, die leicht und routinemäßig durch Mutation, Abbau, Transposition oder Addition erhalten werden können. Jede Sequenz, die für die erfindungsgemäßen Interferone codiert (d.h. die das biologische Aktivitätsspektrum aufweist, das hier beschrieben ist) und im Vergleich mit den gezeigten degeneriert ist, ist ebenfalls eingeschlossen; Fachleute auf diesem Gebiet sind in der Lage, DNA-Sequenzen der codierenden Regionen zu degenerieren. Ebenso ist jede Sequenz, die für ein Polypeptid mit dem Aktivitätsspektrum der erfindungsgemäßen Interferone codiert, und die mit den gezeigten Sequenzen (oder Teilen davon) unter stringenten Bedingungen hybridisiert (beispielsweise Bedingungen, die für mehr als 85 %, bevorzugt mehr als 90 % Homologie selektieren) beinhaltet.

Die Hybrdisierungen werden in 6xSSC/5x Denhardt's Lösung/0,1% SDS bei 65°C durchgeführt. Der Grad der Stringenz wird im Waschschritt festgelegt. So sind für eine Selektionierung auf DNA-Sequenzen mit ca. 85 % oder mehr Homologie die Bedingungen 0,2xSSC/0.01% SDS/65°C und für eine Selektionierung auf DNA-Sequenzen mit ca. 90 % oder mehr Homologie die Bedingungen 0,1x SSC/0,01% SDS 65°C geeignet.

Erfindungsgemäße Interferon-Gene können unter Bedingungen in jeden Organismus eingebracht werden, die zu hohen Ausbeuten führen. Geeignete Wirte und Vektoren sind dem Fachmann bestens bekannt; als Beispiel sei auf die EP-A-0.093.619 hingewiesen.

Insbesondere sind Prokaryoten für die Expression bevorzugt, beispielsweise E. coli K 12, Stamm 294 (ATCC Nr. 31 446) oder E. coli X1776 (ATCC Nr. 31.537). Ebenso wie die vorerwähnten Stämme können auch E. coli W 3110 ($F^-$, $Lambda^-$, Prototroph, ATCC Nr. 27325), Bazillen wie Bacillus subtilis, und andere Enterobacteriaceae, wie Salmonella typhimurium oder Serratia marcescens und verschiedene Pseudomonaden verwendet werden.

Im allgemeinen können Plasmid-Vektoren, die Kontrollsequenzen, die aus Spezies stammen, die kompatibel mit den Wirtszellen sind, enthalten, in Verbindung mit diesen Wirten verwendet werden. Der Vektor trägt üblicher- weise neben einer Replikationsstelle Erkennungssequenzen, die es ermöglichen, die transformierten Zellen phenotypisch zu selektieren. Zum Beispiel wird E. coli üblicherweise mit pBR322 transformiert, ein Plasmid, das aus E. coli Spezies stammt (Bolivar, et al., Gene 2, 95 (1977)). pBR322 enthält Gene für Ampicillin- und Tetracyclin-Resistenz und bietet damit die Möglichkeit, transformierte Zellen zu identifizieren. Das pBR322-Plasmid oder auch andere Plasmide müssen außerdem von sich aus Promotoren enthalten oder müssen mit Promotoren versehen werden, die vom mikrobiellen Organismus zur Expression ihrer eigenen Proteine verwendet werden können. Die Promotoren, die am häufigsten bei der Herstellung rekombinanter DNA verwendet werden, beinhalten die Beta-Lactamase-(Penicillinase) und Lactose-Promotor-Systeme (Chang et al., Nature 275, 615 (1978); Itakura et al., Science 198, 1056 (1977); Goeddel et al., Nature 281, 544 (1979)) und Tryptophan(trp) Promotor- Systeme (Goeddel et al, Nucleic Acids, Res. 8, 4057 (1980); EP-A-0.036.776). Neben diesen besonders gebräuchlichen Promotoren sind auch andere mikrobielle Promotoren entwickelt und benutzt worden. Die Gen-Sequenz für die erfindungsgemäßen Interferone kann beispielsweise unter der Kontrolle des Leftward-Promotors des Bakteriophagen Lambda ($P_L$) eingesetzt werden. Dieser Promotor ist ein besonders effektiver steuerbarer Promotor. Die Steuerung wird möglich durch den Lambda-Repressor, von dem benachbarte Restriktionsschnittstellen bekannt sind. Ein temperaturempfindliches Allel dieses Repressor-Gens kann in einen Vektor, der eine EqIFN-$\gamma$-Sequenz enthält, eingefügt werden. Wird die Temperatur auf 42°C erhöht, wird der Repressor inaktiviert und der Promotor aktiviert. Durch die Verwendung dieses Systems ist es möglich, eine Clon-Bank zu etablieren, in der eine funktionelle IFN-Sequenz in Nachbarschaft zu einer Ribosom-Bindungsstelle in variierenden Abständen zu dem Lambda-$P_L$-Promotor plaziert wird. Diese Klone können dann überprüft und der mit der höchsten Ausbeute selektiert werden.

Die Expression und Translation einer Sequenz codierend für die erfindungsgemäßen Proteine kann auch unter Kontrolle anderer Regulationssysteme, die als "homolog" zu dem Organismus in seiner untransformierten Form gelten können, ablaufen. So enthält z.B. chromosomale DNA von einem Lactose-abhängigen E.coli ein Lactose oder Lac-Operon, das durch Ausschüttung des Enzyms Beta-Galactosidase den Lactose-Abbau ermöglicht.

Die Lac-Kontrollelemente können aus dem Bacteriophagen Lambda-plac5, der infektiös für E. coli ist, erhalten werden. Das Lac-Operon des Phagen kann durch Transduktion aus derselben Bakterien-Spezies stammen. Regulationssysteme, die bei dem erfindungsgemäßen Verfahren Verwendung finden können, können auch aus plasmidischer DNA stammen, die dem Organismus eigen ist. Das Lac-Promotor-Operator-System kann durch IPTG induziert werden.

Andere Promotor-Operator-Systeme oder Teile hiervon können genausogut verwendet werden: beispielsweise Arabinose-Operator, Colicine $E_1$-Operator, Galactose-Operator, alkalischer Phosphatase-Operator, trp-Operator, Xylose-A-Operator, tac- Promotor u.ä..

Zusätzlich zu Prokaryoten können auch eukaryotische Mikro- organismen, wie Hefekulturen verwendet werden. Saccharomyces cerevisiae ist die am meisten verwendete von den eukaryotischen Mikroorganismen, obwohl eine Anzahl anderer Spezies allgemein erhältlich ist. Zur Expression in Saccharomyces wird beispielsweise das Plasmid YRp7 (Stinchcomb et al. Natur 282, 39 (1979); Kingsman et al., Gene 7, 141 (1979); Tschumper et al., Gene 10, 157 (1980)) und das Plasmid YEp13 (Bwach et al., Gene 8, 121-133( 1979)) verwendet. Das Plasmid YRp7 enthält das TRP1-Gen, das eine Selektionierungsmarkierung für eine Hefemutante, die unfähig ist, in trypthophanfreiem Medium zu wachsen, bereitstellt; beispielsweise ATCC Nr. 44076.

Das Vorhandensein des TRP1 Schandens als Charakteristikum des Hefe-Wirts Genoms stellt. dann ein wirksames Hilfsmittel dar, um Transformation nachzuweisen, wenn ohne Tryptophan kultiviert wird.

Ganz ähnlich verhält es sich bei dem Plasmid YEp13, das das Hefe-Gen LEU 2, das zur Ergänzung einer LEU-2-minus-Mutante verwendet werden kann, enthält. Geeignete Promotor-Sequenzen für Hefe Vektoren beinhalten die 5′-flankierende Region der Gene des ADH I (Ammerer G., Methods of Enzymology 101, 192-201 (1983)), 3-Phosphoglycerate-Kinase (Hitzeman et al., J. Biol. Chem. 255, 2073 (1980)) oder andere glykolytische Enzyme (Kawaski und Fraenkel, BBRC 108, 1107-1112 (1982)) wie Enolase, Glycerinaldehyd-3-phosphat-Dehydrogenase, Hexokinase, Pyruvat-Decarboxylase, Phosphofructokinase, Glucose-6- Phosphat-Isomerase, Phosphoglucose-Isomerase und Glucokinase. Bei der Konstrucktion geeigneter Expressionsplasmide können die mit diesen Genen assoziierten Terminationssequenzen ebenfalls in den Expressions-Vektor am 3′-Ende der zu exprimierenden Sequenz eingesetzt werden, um Polyadenylierung und Termination der mRNA zu ermöglichen.

Andere Promotoren, die zudem noch den Vorteil der durch Wachstumsbedingungen kontrollierten Transkription besitzen, sind die Promotor-Regionen der Gene für Alkohol-Dehydrogenase-2, Isocytochrom C, Saure-Phosphatase, abbauende Enzyme, die mit dem Stickstoff-Metabolismus gekoppelt sind, die oben erwähnte Glycerinaldehyd-3-Phosphat-Dehydrogenase und Enzyme, die für die Verarbeitung von Maltose und Galaktose verantwortlich sind. Promotoren, die durch den Hefe Mating Typ Locus reguliert werden, beispielsweise Promotoren der Gene BARI, MFαI, STE2, STE3, STE5 können bei temperaturregulierten Systemen durch die Verwendung von temperaturabhänigigen sir Mutationen eingesetzt werden. (Rhine Ph.D. Thesis, University of Oregon, Eugene, Oregon (1979), Herskowitz and Oshima, The Molecular Biology of the Yeast Saccharomyces, part I, 181-209 (1981), Cold Spring Harbor Laboratory). Diese Mutationen beeinflussen die Expression der ruhenden Mating-Typ Kassetten von Hefen und dadurch indirekt die Mating-Typ abhängigen Promotoren. Generell ist jedoch jeder Plasmid Vektor, der einen Hefe-kompatiblen Promotor, originäre Replikations- und Terminationssequenzen enthält, geeignet.

Zusätzlich zu Mikroorganismen sind Kulturen multizellueärer Organismen ebenfalls geeignete Wirtsorganismen. Im Prinzip ist jede dieser Kulturen einsetzbar, ob von Wirbeltier- oder wirbellosen Tierkulturen. Größtes Interesse besteht jedoch an Wirbeltier-Zellen, so daö die Vermehrung von Wirbeltier- zellen in Kultur (Gewebe-Kultur) in den letzten Jahren zu einer routinemäßigen Methode wurde (Tissue Culture, Academic Press, Kruse and Patterson, Editors (1973)). Beispiele solch nützlicher Wirtszellinien sind VERO- und HeLa-Zellen, CHO-Zellen und WI38, BHK, COS-7 und MDCK-Zellinien. Expressionsvektoren für diese Zellen enthalten üblicherweise (wenn nötig) einen Replikationsstartpunkt, einen Promotor, der vor dem zu exprimierenden Gen lokalisiert ist, gemeinsam mit allen notwendigen Ribosomenbindungsstellen, RNA-Splicing-Stelle, Polyadenylierungsstelle und transkriptionelle Terminations-Sequenzen.

Bei der Verwendung in Säugetierzellen stammen die Kontroll- funktionen auf den Expressions-Vektoren oftmals aus viralem Material. Beispielsweise stammen die üblicherweise verwendeten Promotoren aus Polyoma Adenovirus 2, und besonders häufig aus Simian Virus 40 (SV 40). Die frühen und späten Endpromotoren des SV 40 sind besonders nützlich, da beide leicht aus dem Virus als Fragment zu erhalten sind, das auch noch die virale Replikationsstelle des SV 40 enthält. (Fiers et al., Nature 273, 113 (1978)). Auch können kleinere oder größere Fragmente des SV 40 verwendet werden, vorausgesetzt, sie enthalten die annähernd 250 bp lange Sequenz, die von der HindIII Schittstelle bis zur BglI Schnittstelle in dem viralen Replikationsstartpunkt reicht. Außerdem ist es ebenfalls möglich und oft empfehlenswert, Promotor- oder Kontroll-Sequenzen zu verwenden, die normalerweise mit den gewünschten Gensequenzen verknüpft sind, vorausgesetzt, diese Kontrol-Sequenzen sind kompatibel zu den Wirtszellsystemen.

Ein Replikationsstartpunkt kann entweder durch entsprechende Vektorkonstruktion vorgesehen werden, um einen exogenen Startpunkt einzubauen, beispielsweise aus SV 40 oder anderen viralen Quellen (z.B. Polyoma, Adeno, VSV, PBV, etc.) oder kann durch die chromosomalen Replikationsmechanismen der Wirtszelle vorgesehen werden. Wird der Vektor in das Wirtszellenchromosom integriert, reicht die zuletztgenannte Maßnahme meistens aus.

Vorzugsweise können die erfindungsgemäßen DNA-Sequenzen auch in dem Expressionsplasmid pER103 (E. Rastl-Dworkin et al., Gene 21, 237-248 (1983) und EP-A-0.115-613 - hinterlegt bei der DSM unter der Nummer DSM 2773 am 20. Dezember 1983), in dem Plasmid parpER33 (EP-A-0.115-613) oder dem Plasmid pRH100 exprimiert werden, da diese Vektoren alle Regulationselemente enthalten, die zu einer hohen Expressionsrate der klonierten Gene führen.

Erfindungsgemäß wird als Expressionsvektor für das synthetische EqIFN-γ Gen das Plasmid pRH100 verwendet, das den regulierbaren Tryptophanpromotor aus Serratia marcescens und eine artifizielle Ribosomenbindungsstelle enthält. Zur Herstellung des Expressionsplasmides pRH100 wurde das Plasmid pER103 (Eva Dworkin-Rastl et al., Gene 21 (1983) 237-248, EP-A-0.115-613) mit der Restriktionsendonuklease HindIII linearisiert und die 5'terminalen Phosphatreste wurden entfernt.

Diese Plasmid-DNA wurde mit den phosphorylierten Oligonukleotiden d(AGCTTAAAGATGAGCT) und d-(CATCTTTA) gemischt und ligiert. Die Ligasereaktion wurde mit der Restriktionsendonuklease SacI verdaut und durch Zugabe von T4-PNK ligiert. Die Oligonukleotide wurden analog der in der EP-A-0.115-613 beschriebenen Methode hergestellt. Kompetenter E.coli HB101 wurde mit dieser Ligasereaktion versetzt und inkubiert. Von den entstandenen Bakterienkolonien wurden 12 willkürlich ausgesucht und daraus im Mikromaßstab die Plasmide isoliert (Birnboim und Doly, Nucl. Acids Res. 7 (1979) 1513-1523). Die resultierende DNA wurde mit der Restriktionsendonuklease SacI geschnitten und die DNA auf einem Agarosegel (1%, 1x TBE-Puffer) aufgetrennt. Die Wanderung der DNA als lineares Molekül der Größe von etwa 4.400 bp bestätigte die Einführung einer SacI-Erkennungsstelle in das Plasmid. Eines dieser Plasmide wurde willkürlich ausgesucht. Mit der DNA aus der dazugehörigen Minipräparation wurde nochmals E.coli HB101 transformiert. Von den resultierenden transformierten Bakterien wurde eine Kolonie ausgewählt und in größerem Maßstab angezüchtet. Das daraus isolierte Plasmid wurde mit den Restriktionsendonukleasen EcoRI und BamHI geschnitten, die DNA auf einem 1%igem-Agarosegel aufgetrennt, und das kleinere Fragment aus dem Gel durch Elektroelution isoliert. Dieses etwa 460 bp lange EcoRI-BamHI DNA-Fragment wurde nach Sanger sequenziert. (F. Sanger et al., Proc. Natl. Acad. Sci. (1977) 5463-5467). Das dermaßen analysierte Plasmid wurde pRH100 genannt.

Das Plasmid wurde mit SacI vollständig geschnitten und die überhängenden DNA-Enden wurden durch Behandlung mit Klenow-Fragment (Amersham) in Gegenwart aller vier Desoxynukleotidtriphosphate begradigt. Die Reaktion wird durch Phenol/Chloroform-Extraktion gestoppt und die DNA durch Äthanolfällung konzentriert. Durch diese Behandlung entsteht im Anschluß an den Tryptophanpromotor ein stumpfes DNA-Ende, das mit dem Translationsstartcodon "ATG" endet. Die linearisierte Plasmid-DNA wird mit BamHI nachgeschnitten und der Vektoranteil isoliert.

Der so vorbereitete pRH100 Plasmid-Vektor wird mit den ligierten Oligonukleotiden EG-1 bis EG-8 und EG-9 bis EG-14 gemischt und in Ligationspuffer mit T4 DNA-Ligase inkubiert. Kompetent gemachter E.coli, vorzugsweise JM101, wird mit diesem Ligationsgemisch transformiert und über Nacht inkubiert. Von den erhaltenen Transformanten wird im Minipräparationsverfahren Plasmid-DNA isoliert und durch Restriktionsanalyse und Sequenzierung des HindIII-BamHI Inserts die Struktur ermittelt. Ein Plasmid der gewünschten Struktur für die Expression von reifem EqIFN-$\gamma$ wird pEqG-YYCl benannt. In völlig analoger Weise werden die Oligonukleotide EG-15,16 EG-3 bis EG-8 und EG-9 bis EG-14 in den pRH100 Vektor geklont, um das um drei Aminosäuren verkürzte EqIFN-$\gamma$ zu erhalten. Ein Plasmid der gewünschten Struktur wird pEqG-QAA1 benannt.

Transformation der Zellen mit den Vehikeln kann durch eine Vielzahl an Verfahren erreicht werden.

Beispielsweise kann sie durch Kalzium erfolgen, wobei entweder die Zellen in Magnesium gewaschen und die DNA den in Kalzium suspendierten Zellen zugegeben oder die Zellen einem Kopräzipitat von DNA und Kalziumphosphat ausgesetzt werden. Bei nachfolgender Genexpression werden die Zellen auf Medien übertragen, die für transformierte Zellen selektieren.

Zum Nachweis der Expression der Interferonaktivität durch E.coli JM101, die das Plasmid pEqG-YYC1 oder pEqG-QAA1 enthalten, werden die Bakterien nach der Inkubation in einem geeigneten Kulturmedium aufgebrochen und der Überstand nach Sterilfiltration auf Interferonaktivität in einem Assay, der den cytopathischen Effekt (CPE) von VSV oder EMCV mißt, getestet. Verwendet werden dazu NBL-6 Zellen (ATCC CCL 57, Pferdehaut-Epidermis-Zellen), die mit Vesicular-Stomatitis-Virus (VSV) infiziert worden waren und/oder A549 (ATCC CCL185, humane Lungencarcinom-Zellinie) die mit Encephalomyocarditis Virus (EMCV) infiziert worden waren.

Der Nachweise der exprimierten Pferde-Interferone wird durch Markierung der Proteine in Maxizellen erbracht. Plasmidcodierte Proteine können durch Verwendung der Maxizelltechnik (Sancar, A. et al., J. Bacteriol, 137, 692-693 (1979) selektiv in vivo markiert werden. Der E.coli Stamm CSR603 (CGSC 5830) besitzt keine Mechanismen für die Reparatur von durch UV-Bestrahlung entstandene Schäden der DNA. Durch Bestrahlung mit einer geeigneten UV-Strahlendosis wird das bakterielle Chromosom zerstört, einige der wesentlich kleineren und in mehreren Kopien pro Zelle vorhandenen Plasmid-DNAs bleiben dagegen funktionell. Nach Abtöten aller nicht geschädigten, sich vermehrenden Zellen durch das Antibiotikum D-Cylcoserin und Aufbrauchen der endogenen mRNA werden in den verbleibenden Zellen nur noch am Plasmid codierte Gene transkribiert und translatiert. Die gebildeten Proteine können durch Einbau von $^{35}$S-Methionin radioaktiv markiert und nachgewiesen werden. E.coli CSR603 wird nach üblichen Verfahren mit den Expressionsplasmiden transformiert und auf ampicillinhaltigen Agarplatten auf transformierte Bakterien selektioniert. Die Präparation der Maxizellen und das Markieren der Proteine erfolgt nach der Vorschrift von A. Sancar. Als Molekulargewichtsstandard wird ein $^{14}$C-methyliertes Proteingemisch (Amersham) verwendet.

Als Kontrollen werden das Plasmid pER103, das nur den Promotor ohne Interferongen enthält und das Plasmid pER21/1 eingesetzt, das zwei Kopien des humanen IFN-α2arg Genes enthält.

Zur Charakterisierung der erfindungsgemäßen Produkte sind die bekannten biologischen und immunologischen Assays für Interferone geeignet. Da sowohl IFN-α, -β und γ die in den PFU- und CPE-Assays festzustellende antivirale Eigenschaft eigen ist, wird zur Unterschiedung des erfindungsgemäßen EqIFN-γ's von EqIFN-α und/oder -β- die unterschiedliche Antigenität der Interferone ausgenutzt. Die erfindungsgemäßen Polypeptide werden nicht durch Antiseren gegen EqIFN-α und/oder EqIFN-β neutralisiert.

Als weiteres Unterscheidungskriterium ist die Säurelabilität der erfindungsgemäßen Polypeptide, sowie die Sensitivität auf Natriumdodecylsulfat (SDS) geeignet. Sowohl die Inkubation mit 0,2%iger SDS-Lösung als auch die Inkubation der Polypeptide bei pH2 über einige Stunden bei 4°C führt zu einem fast völligen Verlust der antiviralen Aktivität. EqIFN-α und EqIFN-β- sind unter gleichen Bedingungen stabil.

Zum Nachweis der Gesamtanzahl von Sequenzen im Pferdegenom, die hohe Homologie mit dem Interferongen aufweisen, wird hochmolekulare Pferde-DNA mit den entsprechenden Restriktionsenzymen vollständig verdaut und diese geschnittene DNA nach der Größe aufgetrennt. Nach Southern-Transfer auf Nitrozellulosefilter, denatuieren und fixieren der DNA wird jedes Filter mit nicktranslatierter Probe hybridisiert. Als Probe für EqIFN-γ wird ein Fragment aus dem Plasmid pEqG-YYC1 verwendet, das die codierende Sequenz für das gesamte reife Interferon enthält. Die Filter werden anschließend unter stringenten Bedingungen gewaschen. Die Autoradiographie erfolgt auf DuPont Cronex X-ray Film unter Verwendung von Kodak Lanex-Regular Verstärkerfolie 7 Tage bei -80°C.

Nach erfolgter Transformation des Wirtes, Expression des Gens und Fermentation oder Zellkultivierung unter Bedingungen, bei denen die erfindungsgemäßen Proteine exprimiert wird, kann das Produkt üblicherweise durch bekannte chromatographische Trennmethoden extrahiert werden, um so ein Material zu erhalten, das die Proteine mit oder ohne Leader und Tailing-Sequenzen enthält. Die erfindungsgemäßen Interferone können mit einer Leader-Sequenz am N-Terminus exprimiert werden (Pre-IFN), die von einigen Wirtszellen entfernt werden kann. Wenn nicht, so ist eine Abspaltung des Leader-Polypeptids (wenn vorhanden) erforderlich, um reifes IFN zu erhalten. Alternativ kann der IFN Klon so modifiziert werden, daß das reife Protein im Mikroorganismus direkt statt des Pre-IFN produziert wird. Für diesen Fall kann die Precursor-Sequenz des Hefe-Mating-Pheromons MF-alpha-1 verwendet werden, um eine korrekte "Reifung" des fusionierten Proteins und die Ausscheidung der Produkte in das Wachstumsmedium oder den periplasmischen Raum zu gewährleisten. Die DNA-Sequenz für funktionelles oder reifes IFN kann mit MF-alpha-1 an der vermuteten kathepsinähnlichen Schnittstelle (nach Lys-Arg) an Position 256 von Initiationscodon ATG aus (Kurjan, Herskowitz, Cell 30, 933-943 (1982) verbunden sein.

Ein Verfahren, nach dem EqIFN-γ z.B. aus Bakterien gereinigt werden kann, ist in dem folgenden allgemeinen Schema beschrieben.

1. Extraktion der Zellen in einem Lysepuffer (etwa pH 8) von hoher Leitfähigkeit mittels Passage durch einen Homogenisator bei hohem Druck; Abkühlen des Abstroms in einem Eisbad.
2. Ausfällung der DNA durch Zugabe von Polyethylenimin unter Rühren z.B. bei 4°C.
3. pH-Ausfällung der bakteriellen Proteine, wobei wiederum EqIFN-γ in Lösung bleibt.
4. Abzentrifugieren der Feststoffe bei 4°C.
5. Konzentrieren des Überstands (nach erneuter Einstellung des pH) z.B. durch Ultrafiltration.
6. Dialyse des Konzentrats gegen einen Puffer von niedriger Leitfähigkeit.
7. Abzentrifugieren der Feststoffe, wobei EqIFN-γ in Lösung bleibt.
8. Ionenaustauschchromatographie an Carboxymethylcellulose, Elution mit einem Gradienten an zunehmender Ionenstärke.
9. Chromatographie an Calciumphosphat-Gel und Elution mit einem Gradienten an zunehmender Ionenstärke.
10. Ionenaustauschchromatographie an Carboxymethylcellulose unter schwach denaturierenden Bedingungen und Elution mit einem Gradienten an zunehmender Ionenstärke.
11. Abtrennung durch Gelfiltrationschromatographie.

Das genannte Verfahren ermöglicht Materialausbeuten mit einer Reinheit von mehr als 95%.

An dieser Stelle sei erwähnt, daß es sich bei den erfindungsgemäßen Interferonen nicht nur um die Interferone, die im einzelnen beschrieben sind, handelt, sondern ebenfalls um jedwede Modifikation dieser Polypeptide, die die Pferde-γ-IFN-Aktivität nicht wesentlich verändert. Diese Modifikationen beinhalten z.B. Verkürzung des Moleküls z.B. am N- oder C-terminalen Ende, Austausch von Aminosäuren durch andere Reste, chemische oder biochemische Bindungen des Moleküls an andere Moleküle, die inert oder aktiv sind. Bei den letztgenannten Modifikationen kann es sich beispielsweise um Hybridomoleküle aus einem oder mehreren erfindungsgemäßen Interferonen und/oder bekannten α- oder β-Interferonen handeln.

Basierend auf ihrem biologischen Wirkungsspektrum sind die neuen, erfindungsgemäßen Interferone verwendbar für jede Art der Behandlung für die auch die bekannten Interferone eingesetzt werden. Diese beinhalten beispielsweise Herpes, Rhinovirus, Equine-Abortion-Virus, verschiedene Krebsarten und ähnliches. Die neuen Interferone können alleine oder in Kombination mit anderen bekannten Interferonen oder biologisch aktiven Produkten eingesetzt werden, beispielsweise mit IFN-alpha, IL-2, anderen Immun-Modulatoren und ähnlichen.

Die erfindungsgemäßen Interferone können parenteral verabreicht werden in Fällen, in denen Antitumor oder antivirale Behandlung erforderlich ist und in Fällen, in denen sich immunosuppressive Eigneschaften zeigen. Dosierung und Dosierungsrate können ähnlich denen sein, die zur Zeit bei klinischen Untersuchungen für IFN-Materialien gelten z.B. ca. (1-10) x $10^6$ Einheiten täglich und bei Präparaten, die zu mehr als 1 % rein sind, bis zu beispielsweise 5 x $10^7$ Einheiten täglich. Beispielsweise können für eine zweckmäßige Dosierungsform bei einem im wesentlichen homogenen, bakterielle produzierten erfindungsgemäßen IFN, bei parenteraler Verwendung 3 mg EqIFN-$\gamma$ in 25 ml 5%igem tierischem Serum Albumin vorzugsweise Pferde/Hunde Serum Albumin gelöst werden. Diese Lösung wird dann durch ein bakteriologisches Filter gegeben und die gefilterte Lösung aseptisch auf 100 Fläschchen verteilt, von dem jedes 6 x $10^6$ Einheiten reines IFN zur parenteralen Applikation geeignet, enthält. Die Gläschen werden vor Verwendung vorzugsweise in der Kälte (-20 °C) aufbewahrt. Die erfindungsgemäßen Substanzen können in bekannter Weise formuliert werden, um pharmazeutisch verwendbare Mittel zu erhalten, wobei das erfindungsgemäße Polypeptid mit einer pharmazeutischen, akzeptablen Trägersubstanz vermischt wird. Gebräuchliche Trägerstoffe und ihre Formulierung sind bei E.W. Martin in Remington's Pharmaceutical Sciences beschrieben, worauf ausdrücklich Bezug genommen wird. Die erfindungsgemäßen Interferone werden mit einer angemessenen Menge des Trägerstoffes vermischt, um geeignete pharmazeutische Mittel zu schaffen, die für eine effektive Anwendung beim Empfänger (Patienten) geeignet sind. Bevorzugt wird eine parenterale Applikation vorgenommen.

Gegenstand der Erfindung im einzelnen sind:

Polypeptide in im wesentlichen reiner Form

- mit den biologischen und immunologischen Eigenschaften von Pferde-Interferon-gamma (EqIFN-gamma);
- im wesentlichen frei von anderen Proteinen tierischer Herkunft;
- frei von nativer Glykosylierung;
- die Aminosäure Methionin vor der 1. Aminosäure des N-Terminus aufweisend;
- ein Leader-Peptid enthaltend;
- die Aminosäuresequenz

| | | | | | | | | | | | | | | |
|---|---|---|---|---|---|---|---|---|---|---|---|---|---|---|
| 1 | | | | 5 | | | | | 10 | | | | | 15 |
| Tyr | Tyr | Cys | Gln | Ala | Ala | Phe | Phe | Lys | Glu | Ile | Glu | Asn | Leu | Lys |
| | | | | 20 | | | | | 25 | | | | | 30 |
| Glu | Tyr | Phe | Asn | Ala | Arg | Asn | Pro | Asp | Val | Gly | Asp | Gly | Gly | Pro |
| | | | | 35 | | | | | 40 | | | | | 45 |
| Leu | Phe | Leu | Asp | Ile | Leu | Lys | Asn | Trp | Lys | Glu | Asp | Ser | Asp | Lys |
| | | | | 50 | | | | | 55 | | | | | 60 |
| Lys | Ile | Ile | Gln | Ser | Gln | Ile | Val | Ser | Phe | Tyr | Phe | Lys | Leu | Phe |
| | | | | 65 | | | | | 70 | | | | | 75 |
| Glu | Asn | Leu | Lys | Asp | Asn | Gln | Val | Ile | Gln | Lys | Ser | Met | Asp | Thr |
| | | | | 80 | | | | | 85 | | | | | 90 |
| Ile | Lys | Glu | Asp | Leu | Phe | Val | Lys | Phe | Phe | Asn | Ser | Ser | Thr | Ser |
| | | | | 95 | | | | | 100 | | | | | 105 |
| Lys | Leu | Glu | Asp | Phe | Gln | Lys | Leu | Ile | Gln | Ile | Pro | Val | Asn | Asp |
| | | | | 110 | | | | | 115 | | | | | 120 |
| Leu | Lys | Val | Gln | Arg | Lys | Ala | Ile | Ser | Glu | Leu | Ile | Lys | Val | Met |
| | | | | 125 | | | | | 130 | | | | | 135 |
| Asn | Asp | Leu | Ser | Pro | Lys | Ala | Asn | Leu | Arg | Lys | Arg | Lys | Arg | Ser |
| | | | | 140 | | | | | 145 | | | | | |
| Gln | Asn | Pro | Phe | Arg | Gly | Arg | Arg | Ala | Leu | Gln | | | | |

oder

Gln Ala Ala Phe Phe Lys Glu Ile Glu Asn Leu Lys
Glu Tyr Phe Asn Ala Arg Asn Pro Asp Val Gly Asp Gly Gly Pro
Leu Phe Leu Asp Ile Leu Lys Asn Trp Lys Glu Asp Ser Asp Lys
Lys Ile Ile Gln Ser Gln Ile Val Ser Phe Tyr Phe Lys Leu Phe
Glu Asn Leu Lys Asp Asn Gln Val Ile Gln Lys Ser Met Asp Thr
Ile Lys Glu Asp Leu Phe Val Lys Phe Phe Asn Ser Ser Thr Ser
Lys Leu Glu Asp Phe Gln Lys Leu Ile Gln Ile Pro Val Asn Asp
Leu Lys Val Gln Arg Lys Ala Ile Ser Glu Leu Ile Lys Val Met
Asn Asp Leu Ser Pro Lys Ala Asn Leu Arg Lys Arg Lys Arg Ser
Gln Asn Pro Phe Arg Gly Arg Arg Ala Leu Gln

enthaltend.

DNA-Moleküle, codierend:

- für ein Polypeptid mit den biologischen und immunologischen Eigenchaften des EqIFN-gamma;
- für ein Polypeptid wie oben angegeben;
- für reifes Eq IFN-gamma.

DNA-Moleküle,

- das vollständige, natürliche Gen für EqIFN-gamma;

- die Nukleotide

```
ATG AAT TAT ACA AGT TTT ATC TTG GCT TTT CAG CTG TGT GCG ATT

TTG GGT TCT TCT ACC TAT TAC TGC CAG GCC GCG TTT TTT AAA GAA

                                   ---------I N T R O N 1---------
ATA GAA AAC CTA AAG GAA TAT TTT GTAAGTATGACCTTTTAATAATACTTAC
TTGTGGTTGAAATGACTGAACGTTGTCTTGGAGTTGGATCTCTGATAGGCTGTCCTCTCT
ACTCCACAGTCATCTTGAGAAGACTGGGTGTTATTTTCTCTGTTTGTTGACTGGATGAGT
TTTTCTTTTTTTTACTAAATGATCTAGATATTGCTTTAACCCTCTGCTCAATTTGCTATA
GAGACTTAGAGAGGGTTCATGAATCTTCCAAAAGATGGGCTTAACAGGTTTATAAAGCAT
AGTGAAGTTGACAATTTTGTGGTGAGAAGCCACTGAATTGTGATAAGTCAAGTAGTGTGG
ACATTGAAAAAATGACTAGCTATTAGTTTCTAACTTCTCAGGTTACTATGATGGTGACAA
TAAAAGGTCAAGATTAGCATTAAAATGGTAATCTGAAATAATTGATCAGTTAAAGAAGGC
GCTGTCCTGAAAGGTTTGGCTGAAAAAAAATCACTTTCAGGTGTTTTCCTCCAAAAAATG
ATTTTAAAATCTTACTGCCCCGTTTGTGTTAGCTGTGAAGTACTCTGGAACTCAGTCAAT
TGCTGAGATTTTGTACGAGTTATAAGCTGGCTTATATTTAAAAAATTTTTTTGTTTTTGT
TTTATGAGTTTCTTTTAAAATGTTATTTATGGTTAATTAAAATAGTTTTTGCATTTTAAA
TATTTTATTATTTGTCCAAAATTTAGCTATTTTAATTATAGTTGGAGCTCTCTTTTAGAG
CTGACATAAGACCATAGGGGAGGCACAGATAGATGTGATGGAGCCCTGTACCAGACGGGG
GCAGTATCTTATAGTGGGTTGCCTTTGCTGATCTTTTTACTAGACTTGAAATTATTTGCT
TTTCCTTCCTATGGTTATTTGGACTATTGAAGTATCACCAGCCCTGTTGAGTTCATCTG
TAATATTGTAATTCAAGGGTTACACTAGAAAATAAGAAAGCTAAAACAGCACGATAATCT
TTGGCTACATCCAACACAATAGCTTTTGGGAATACTTATTGTTAGAACTAAACAGAGGGT
TGAAAAGAAAATCAGTGAATACTGTCAGCATCTGAGTTCAATAAAACGTGAAGTACATTT
-----------------------------------------------------
TTAGGGCAATTCATGGACTAATTGTAAACCAAGTTTTCCTTCCTTTTTCAG AAC GCA

AGA AAC CCA GAT GTA GGG GAT GGT GGG CCT CTT TTC CTG GAT ATC

                        ---------------------I N T R O N 2-
TTG AAG AAC TGG AAA GAG GTAAGCTAAGTATTTCCATTTGGTTGATTTTCCTGT
------------------------------------------------------------
TGCTTATTTTCTGGTGGATGAATTCACACCAACCTCTCTTTGTGCTCTTTTCTCCCTAG

GAT AGT GAC AAA AAA ATA ATT CAG AGC CAA ATC GTC TCC TTC TAC

TTC AAA CTC TTT GAA AAC TTG AAA GAT AAC CAG GTC ATT CAA AAG
```

AGC ATG GAC ACC ATC AAG GAG GAC CTG TTC GTT AAG TTC TTT AAC

AGC AGC ACC AGC AAG CTG GAA GAC TTC CAA AAG CTG ATT CAG ATT

-----------I N T R O N 3-------------------------------
CCG GTGAGGAGATCTTAATTCTTTCTTTGGTTTCATTACAGAGGTTCTTGCAAAGTGCT
TACGTCCCAGAAAGTAGAAATGAACTATGAAATGAACCCGTGGCCAAAACTCCTCCTTCC
TAATTCCATTTGTGCTTTGAGAGACTTTGCTAAGTCAGTATGGGAATCATTTAAATTTGT
GATTTGGGGAAATGCTGGCACTATGACTACTGCACAAAGGCAGGTGAAGGGACAAATCCA
GTGAGGAGGGGGCAGTGAAGAAGTGGAGGGGAGTCTGGAGAAGCAGGTCTCTCCTTGCCC
CTTGTTCGTGAGATGAAATCCTCCTGCTTTGGATGGGAGGCTGCGTGTCTTGGTGGAAAG
AGCAGTGGGAGGAGGGAGAAGATTTGTGCTCCTCCCAGCTCAGCCACCAAGAAACTGTGA
CCTCAGATGAATCACAGGCCTGGCTGGGGCTCAGTTTCCTCATCTTAAAAGAGGCCTATT
GGGTTCACTAAAATTTCTATGATCTTCTTTGCTCTATAATCCTACAATTCTGTGGACAGA
AAATGAAATGAGGTAGGAGAAAGAAATAGCCTTTGAAGAGGTTCTTGGGCATTCCACTGC
CAGGCTCTGGTCAACCTTCATACTCTGCAGCCCAAGAAGAGGCAAGACCATTTGTCTGTT
TTTGGAAATGCAAATAGGCGGCATTTATACCTCACGAAAGAACTGTTCTGTCAACTTTTG
GATACTGGGCTATCTTGGCTGGAGAAATCCTTAGGCTCCCAAACTTTCTCTCATGAAATT
GTCTTGAGTCTTTAAATTTATGGCTTCTCGAAGCTGAGAGATAACTTTAAGCATAAAGAC
AAATTACATTTTCCAACATTTTGTCTAAGAGACAAAGACCTCCACATGCCTTTGGGTTTG
GCCTGGATCTAAATGGGCTTGAATGAGAAGGGGAGGGTGTTGTTATGACTATGTTTAGAA
GAGAAAACAGAGGTTTGGAGAGGTTAAGTGGCTGGTTCAAAGTCAGAGTTATTGCACACA
CAGGATTCGAACCCATATGTTTTGTCCCTCCACTTTAGGGTTCTTTTCGCTACATAATTT
TGAGAATTCTGTACCAGTCAATTTAAGGATGTGTGATGTTCCCCATCCTATTACAGCACA
ACCAGCAATTTAATTATAATTTTAGTCTTAACTGCTGAAGAAAGCAGCATTACATATTAA
GCTAACATATTCCTGGTGAAAGCAACTTTTTCAAAGGAATATTTCTATTTTCATGGACCA
TGACAGTAGCACAGCCTGATGGCTTGTATGCCTGAAACTAATTTTGCTGTTTTCTTTCCC
-----
AATAG GTA AAT GAT CTG AAG GTC CAG CGC AAA GCA ATA AGT GAA CTC

ATC AAA GTG ATG AAT GAT CTG TCG CCC AAA GCT AAC CTG AGG AAG

CGG AAG AGG AGT CAG AAT CCA TTT CGA GGC CGG AGA GCG TTG CAA

***
TAG

oder

TAT TAC TGC CAG GCC GCG TTT TTT AAA GAA

-----I N T R O N 1---------

ATA GAA AAC CTA AAG GAA TAT TTT GTAAGTATGACCTTTTAATAATACTTAC
TTGTGGTTGAAATGACTGAACGTTGTCTTGGAGTTGGATCTCTGATAGGCTGTCCTCTCT
ACTCCACAGTCATCTTGAGAAGACTGGGTGTTATTTTCTCTGTTTGTTGACTGGATGAGT
TTTTCTTTTTTTTACTAAATGATCTAGATATTGCTTTAACCCTCTGCTCAATTTGCTATA
GAGACTTAGAGAGGGTTCATGAATCTTCCAAAAGATGGGCTTAACAGGTTTATAAAGCAT
AGTGAAGTTGACAATTTTGTGGTGAGAAGCCACTGAATTGTGATAAGTCAAGTAGTGTGG
ACATTGAAAAAATGACTAGCTATTAGTTTCTAACTTCTCAGGTTACTATGATGGTGACAA
TAAAAGGTCAAGATTAGCATTAAAATGGTAATCTGAAATAATTGATCAGTTAAAGAAGGC
GCTGTCCTGAAAGGTTTGGCTGAAAAAAAATCACTTTCAGGTGTTTTCCTCCAAAAAATG
ATTTTAAAATCTTACTGCCCCGTTTGTGTTAGCTGTGAAGTACTCTGGAACTCAGTCAAT
TGCTGAGATTTTGTACGAGTTATAAGCTGGCTTATATTTAAAAAATTTTTTTGTTTTTGT
TTTATGAGTTTCTTTTAAAATGTTATTTATGGTTAATTAAAATAGTTTTTGCATTTTAAA
TATTTTATTATTTGTCCAAAATTTAGCTATTTTAATTATAGTTGGAGCTCTCTTTTAGAG
CTGACATAAGACCATAGGGGAGGCACAGATAGATGTGATGGAGCCCTGTACCAGACGGGG
GCAGTATCTTATAGTGGGTTGCCTTTGCTGATCTTTTTACTAGACTTGAAATTATTTGCT
TTTCCTTCCTATGGTTATTTGGGACTATTGAAGTATCACCAGCCCTGTTGAGTTCATCTG
TAATATTGTAATTCAAGGGTTACACTAGAAAATAAGAAAGCTAAAACAGCACGATAATCT
TTGGCTACATCCAACACAATAGCTTTTGGGAATACTTATTGTTAGAACTAAACAGAGGGT
TGAAAAGAAAATCAGTGAATACTGTCAGCATCTGAGTTCAATAAAACGTGAAGTACATTT

----------------------------------------------------

TTAGGGCAATTCATGGACTAATTGTAAACCAAGTTTTCCTTCCTTTTTCAG AAC GCA

AGA AAC CCA GAT GTA GGG GAT GGT GGG CCT CTT TTC CTG GAT ATC

---------------------I N T R O N 2-

TTG AAG AAC TGG AAA GAG GTAAGCTAAGTATTTCCATTTGGTTGATTTTCCTGT

------------------------------------------------------------

TGCTTATTTTCTGGTGGATGAATTCACACCAACCTCTCTTTGTGCTCTTTTCTCCCTAG

GAT AGT GAC AAA AAA ATA ATT CAG AGC CAA ATC GTC TCC TTC TAC

TTC AAA CTC TTT GAA AAC TTG AAA GAT AAC CAG GTC ATT CAA AAG

AGC ATG GAC ACC ATC AAG GAG GAC CTG TTC GTT AAG TTC TTT AAC

AGC AGC ACC AGC AAG CTG GAA GAC TTC CAA AAG CTG ATT CAG ATT

-----------I N T R O N 3-------------------------------

CCG GTGAGGAGATCTTAATTCTTTCTTTGGTTTCATTACAGAGGTTCTTGCAAAGTGCT
TACGTCCCAGAAAGTAGAAATGAACTATGAAATGAACCCGTGGCCAAAACTCCTCCTTCC
TAATTCCATTTGTGCTTTGAGAGACTTTGCTAAGTCAGTATGGGAATCATTTAAATTTGT
GATTTGGGGAAATGCTGGCACTATGACTACTGCACAAAGGCAGGTGAAGGGACAAATCCA
GTGAGGAGGGGGCAGTGAAGAAGTGGAGGGGAGTCTGGAGAAGCAGGTCTCTCCTTGCCC
CTTGTTCGTGAGATGAAATCCTCCTGCTTTGGATGGGAGGCTGCGTGTCTTGGTGGAAAG
AGCAGTGGGAGGAGGGAGAAGATTTGTGCTCCTCCCAGCTCAGCCACCAAGAAACTGTGA
CCTCAGATGAATCACAGGCCTGGCTGGGGCTCAGTTTCCTCATCTTAAAAGAGGCCTATT
GGGTTCACTAAAATTTCTATGATCTTCTTTGCTCTATAATCCTACAATTCTGTGGACAGA
AAATGAAATGAGGTAGGAGAAAGAAATAGCCTTTGAAGAGGTTCTTGGGCATTCCACTGC
CAGGCTCTGGTCAACCTTCATACTCTGCAGCCCAAGAAGAGGCAAGACCATTTGTCTGTT
TTTGGAAATGCAAATAGGCGGCATTTATACCTCACGAAAGAACTGTTCTGTCAACTTTTG
GATACTGGGCTATCTTGGCTGGAGAAATCCTTAGGCTCCCAAACTTTCTCTCATGAAATT
GTCTTGAGTCTTTAAATTTATGGCTTCTCGAAGCTGAGAGATAACTTTAAGCATAAAGAC
AAATTACATTTTCCAACATTTTGTCTAAGAGACAAAGACCTCCACATGCCTTTGGGTTTG
GCCTGGATCTAAATGGGCTTGAATGAGAAGGGGAGGGTGTTGTTATGACTATGTTTAGAA
GAGAAAACAGAGGTTTGGAGAGGTTAAGTGGCTGGTTCAAAGTCAGAGTTATTGCACACA
CAGGATTCGAACCCATATGTTTTGTCCCTCCACTTTAGGGTTCTTTTCGCTACATAATTT
TGAGAATTCTGTACCAGTCAATTTAAGGATGTGTGATGTTCCCCATCCTATTACAGCACA
ACCAGCAATTTAATTATAATTTTAGTCTTAACTGCTGAAGAAAGCAGCATTACATATTAA
GCTAACATATTCCTGGTGAAAGCAACTTTTTCAAAGGAATATTTCTATTTTCATGGACCA
TGACAGTAGCACAGCCTGATGGCTTGTATGCCTGAAACTAATTTTGCTGTTTTCTTTCCC

-----

AATAG GTA AAT GAT CTG AAG GTC CAG CGC AAA GCA ATA AGT GAA CTC

ATC AAA GTG ATG AAT GAT CTG TCG CCC AAA GCT AAC CTG AGG AAG

CGG AAG AGG AGT CAG AAT CCA TTT CGA GGC CGG AGA GCG TTG CAA

***

TAG

oder

```
  1                       5                       10                      15
 TAT TAC TGC CAG GCC GCG TTT TTT AAA GAA ATA GAA AAC CTA AAG
                         20                      25                      30
 GAA TAT TTT AAC GCA AGA AAC CCA GAT GTA GGG GAT GGT GGG CCT
                         35                      40                      45
 CTT TTC CTG GAT ATC TTG AAG AAC TGG AAA GAG GAT AGT GAC AAA
                         50                      55                      60
 AAA ATA ATT CAG AGC CAA ATC GTC TCC TTC TAC TTC AAA CTC TTT
                         65                      70                      75
 GAA AAC TTG AAA GAT AAC CAG GTC ATT CAA AAG AGC ATG GAC ACC
                         80                      85                      90
 ATC AAG GAG GAC CTG TTC GTT AAG TTC TTT AAC AGC AGC ACC AGC
                         95                      100                     105
 AAG CTG GAA GAC TTC CAA AAG CTG ATT CAG ATT CCG GTA AAT GAT
                         110                     115                     120
 CTG AAG GTC CAG CGC AAA GCA ATA AGT GAA CTC ATC AAA GTG ATG
                         125                     130                     135
 AAT GAT CTG TCG CCC AAA GCT AAC CTG AGG AAG CGG AAG AGG AGT
                         140                     145
 CAG AAT CCA TTT CGA GGC CGG AGA GCG TTG CAA TAG
```

oder

```
 -1   1                       5                       10                      15
 ATG TAC TAC TGC CAG GCT GCT TTC TTT AAA GAA ATC GAA AAC CTG AAA
                         20                      25                      30
 GAA TAC TTC AAC GCT CGT AAC CCA GAC GTT GGT GAC GGT GGT CCG
                         35                      40                      45
 CTG TTC CTG GAC ATC CTG AAA AAC TGG AAA GAA GAC TCT GAC AAA
                         50                      55                      60
 AAG ATC ATC CAG TCT CAG ATC GTT TCT TTC TAC TTC AAA CTG TTC
                         65                      70                      75
 GAA AAC CTG AAA GAC AAC CAG GTT ATC CAG AAA TCG ATG GAC ACT
                         80                      85                      90
 ATC AAA GAA GAT CTG TTC GTT AAA TTC TTC AAC TCG TCG ACT TCT
                         95                      100                     105
 AAA CTG GAA GAC TTC CAG AAA CTG ATC CAG ATC CCA GTT AAC GAC
                         110                     115                     120
 CTG AAA GTT CAG CGT AAG GCT ATC TCT GAA CTG ATC AAA GTT ATG
                         125                     130                     135
 AAC GAC CTG TCT CCA AAA GCT AAC CTG CGT AAA CGT AAA CGT TCT
                         140                     145
 CAG AAC CCA TTC CGT GGT CGT CGT GCT CTT CAG TAA
```

oder

```
-1   1               5                   10                  15
ATG CAG GCT GCT TTC TTT AAA GAA ATC GAA AAC CTG AAA GAA TAC TTC
                 20                  25                  30
AAC GCT CGT AAC CCA GAC GTT GGT GAC GGT GGT CCG CTG TTC CTG
                 35                  40                  45
GAC ATC CTG AAA AAC TGG AAA GAA GAC TCT GAC AAA AAG ATC ATC
                 50                  55                  60
CAG TCT CAG ATC GTT TCT TTC TAC TTC AAA CTG TTC GAA AAC CTG
                 65                  70                  75
AAA GAC AAC CAG GTT ATC CAG AAA TCG ATG GAC ACT ATC AAA GAA
                 80                  85                  90
GAT CTG TTC GTT AAA TTC TTC AAC TCG TCG ACT TCT AAA CTG GAA
                 95                  100                 105
GAC TTC CAG AAA CTG ATC CAG ATC CCA GTT AAC GAC CTG AAA GTT
                 110                 115                 120
CAG CGT AAG GCT ATC TCT GAA CTG ATC AAA GTT ATG AAC GAC CTG
                 125                 130                 135
TCT CCA AAA GCT AAC CTG CGT AAA CGT AAA CGT TCT CAG AAC CCA
                 140         143
TTC CGT GGT CGT CGT GCT CTT CAG TAA
```

gegebenenfalls zusätzlich die Leader-Sequenz

```
ATG AAT TAT ACA AGT TTT ATC TTG GCT TTT CAG CTG TGT GCG ATT

TTG GGT TCT TCT ACC
```

oder ATG enthaltend;

- die mit einem der obengennanten DNA-Moleküle unter Bedingungen, die eine Homologie größer als 85%, vorzugsweise größer als 90% erkennen lassen, hybridisieren, wobei diese DNA-Moleküle natürlichen, synthetischen oder halbsynthetischen Ursprungs sein können und mit diesen DNA-Molekülen durch Mutation, Nukleotid-Substitutionen, Nukleotid-Deletionen, Nukleotid-Insertion und Inversionen von Nukleotiden verwandt sein können und für Polypeptide mit der biologischen und immunologischen Aktivität von EqIFN-gamma codieren.

DNA-Moleküle:

- die Nukleotide

```
EG-1   5'-TACTACTGCC AGGCTGCTTT CTTTAAAGAA ATCGAAAACC TGAAAGAATA
          CTTCAACGCT CG-3'
```

oder

```
EG-2   5'-TTGAAGTATT CTTTCAGGTT TTCGATTTCT TTAAAGAAAG CAGCCTGGCA
          GTAGTA-3'
```

oder

EG-3 5'-TAACCCAGAC GTTGGTGACG GTGGTCCGCT GTTCCTGGAC ATCCTGAAAA ACTGGAAAGA AGACTCTG-3'

oder

EG-4 5'-TTCTTTCCAG TTTTTCAGGA TGTCCAGGAA CAGCGGACCA CCGTCACCAA CGTCTGGGTT ACGAGCG-3'

oder

EG-5 5'-ACAAAAAGAT CATCCAGTCT CAGATCGTTT CTTTCTACTT CAAACTGTTC GAAAACCTGA AAGACAACC-3'

oder

EG-6 5'-TTTCAGGTTT TCGAACAGTT TGAAGTAGAA AGAAACGATC TGAGACTGGA TGATCTTTTT GTCAGAGTC-3'

oder

EG-7 5'-AGGTTATCCA GAAATCGATG GACACTATCA AAGAAGATCT GTTCGTTAAA TTCTTCAACT CG-3'

oder

EG-8 5'-TCGACGAGTT GAAGAATTTA ACGAACAGAT CTTCTTTGAT AGTGTCCATC GATTTCTGGA TAACCTGGTT GTC-3'

oder

EG-9 5'-TCGACTTCTA AACTGGAAGA CTTCCAGAAA CTGATCCAGA TCCCAGTTAA CGACCTGAAA-3'

oder

**EG-10 5'-GCTGAACTTT CAGGTCGTTA ACTGGGATCT GGATCAGTTT CTGGAAGTCT TCCAGTTTAG AAG-3'**

oder

**EG-11 5'-GTTCAGCGTA AGGCTATCTC TGAACTGATC AAAGTTATGA ACGACCTGTC TCCAAAAGCT AA-3'**

oder

**EG-12 5'-CGCAGGTTAG CTTTTGGAGA CAGGTCGTTC ATAACTTTGA TCAGTTCAGA GATAGCCTTA C-3'**

oder

**EG-13 5'-CCTGCGTAAA CGTAAACGTT CTCAGAACCC ATTCCGTGGT CGTCGTGCTC TTCAGTAAG-3'**

oder

**EG-14 5'-GATCCTTACT GAAGAGCACG ACGACCACGG AATGGGTTCT GAGAACGTTT ACGTTTA-3'**

oder

**EG-15 5'-CAGGCTGCTT TCTTTAAAGA AATCGAAAAC CTGAAAGAAT ACTTCAACGC TCG-3'**

oder

**EG-16 5'-TTGAAGTATT CTTTCAGGTT TTCGATTTCT TTAAAGAAAG CAGCCTG-3'**

enthaltend, codierend für Teilbereiche des EqIFN-gamma, beliebige Kombinationen von mindestens zwei dieser DNA-Moleküle und die von diesen DNA-Molekülen codierten Polypeptide;

- die mit einem der obengenannten DNA-Moleküle unter Bedingungen, die eine Homologie größer als 85%, vorzugsweise größer als 90% erkennen lassen, hybridisieren, wobei diese DNA-Moleküle natürlichen, synthetischen oder halbsynthetischen Ursprungs sein können und mit diesen DNA-Molekülen durch Mutation, Nukleotid-Substitutionen, Nukleotid-Deletionen, Nukleotid-Insertionen und Inversionen von Nukleotiden verwandt sein können und die für Teilbereiche des EqIFN-gamma codieren, sowie die von diesen DNA-Molekülen codierten Polypeptide.

Rekombinante DNA-Moleküle, beispielsweise Vektoren, vorzugsweise Plasmide, die:

- ein für ein obenerwähntes Polypeptid codierendes Insert;
- ein obenerwähntes DNA-Molekül;
- ein obenerwähntes DNA-Molekül in funktioneller Verbindung mit einer Expressionskontrollsequenz enthalten, in Mikroorganismen, wie Prokaryoten, beispielsweise E.coli, Eukaryoten, beispielsweise Saccharomyces cerevisiae und Säugetierzellen, beispielsweise Pferdezellen replizierbar;
- mindestens zwei der als EG-1 bis EG-16 bezeichneten DNA-Moleküle in beliebiger Kombination miteinander verknüpft in funktioneller Verbindung mit einer Expressionskontrollsequenz enthalten, in Mikroorganismen, wie Prokaryoten, beispielsweise E.coli, Eukaryoten, beispielsweise Saccharomyces cerevisiae und Säugetierzellen, beispielsweise Pferdezellen replizierbar.

Rekombinante DNA-Moleküle, wie die Plasmide pAH111, pRH281/5, pRH282/5, pGN1, pGN3, pGN20, pEqG-QAA2 oder pEqG-QAA3.

Wirtsorganismen, mit einem der obenerwähnten rekombinanten DNA-Moleküle transformiert, beispielsweise Prokaryoten, vorzugsweise E.coli, insbesondere E.coli JM101 oder HB101, Eukaryoten, beispielsweise Saccharomyces cerevisiae oder Säugetierzellinien, vorzugsweise Pferdezellinien.

Verfahren zur Herstellung erfindungsgemäßer Polypeptide, wobei

a) geeignete Wirtsorganismen, beispielsweise die obenerwähnten, mit genetischen Informationen, codierend für die erfindungsgemäßen Polypeptide, vorzugsweise mit den obenerwähnten rekombinanten DNA-Molekülen transformiert,

b) die Information zur Produktion der erfindungsgemäßen Polypeptide im Wirtsorganismus exprimiert und

c) die erfindungsgemäße Polypeptide isoliert werden.

Polypeptide, die nach diesen Verfahren herstellbar sind.

Verwendung der erfindungsgemäßen Polypeptide zur therapeutischen Behandlung und/oder zur Immunisierung oder zur Herstellung pharmazeutischer Präparate.

Mittel für die therapeutische Behandlung, beispielsweise von Pferden, dadurch gekennzeichnet, daß es neben pharmazeutisch inerten Trägerstoffen eine wirksame Menge eines der erfindungsgemäßen Polypeptide enthält.

Legende zu den Figuren:

Figur 1: DNA-Sequenz des 4664 bp langen BamHI-Fragments aus Lambda Eq-γ2. Die codierte Aminosäuresequenz und die Lage der Introns ist angegeben. Negativ numerierte Aminosäuren zeigen das hydrophobe Signalpeptid an. Die einzige potentielle N-Glykosylierungsstelle des reifen EqIFN-γ an Position 86-88 ist unterstrichen. Die für die Bindung der RNA-Polymerase wichtigen Sequenzen CCATC und TATAAAA sind unterstrichen, ebenso zwei Signalsequenzen für die Polyadenylierung der mRNA (AATAAA).

Figur 2: Vergleich der Aminosäuresequenzen von gamma-Interferonen verschiedener Spezies. Die mit vorangestelltem "S" numerierten. Aminosäuren geben das Signalpeptid an. Die "Consensus"-Sequenz zeigt in Großbuchstaben jene Aminosäuren, die in allen gamma-Interferonen identisch, in Kleinbuchstaben die Aminosäuren, die in mehr als 75% der gamma-Interferone vorkommen.

Figur 3: Schematische Darstellung der für die Totalsynthese des Pferde gamma-Interferon Gens verwendeten Oligonukleotide. Die Länge der einzelnen Oligonukleotide, sowie deren Numerierung ist angegeben. Restriktionsschnittstellen, die innerhalb des synthetischen Gens nur ein einziges Mal auftreten, sind numeriert.

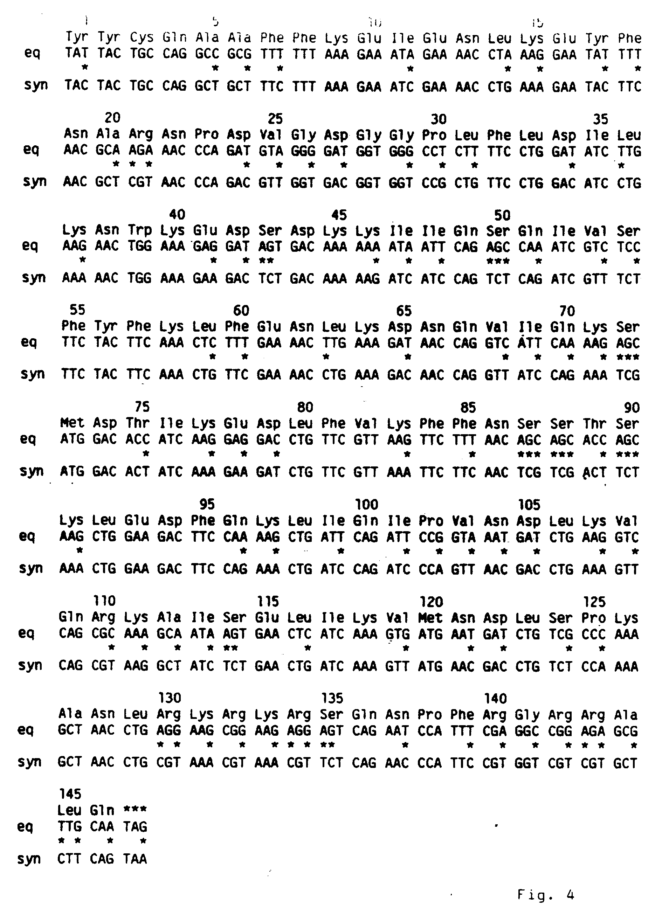

Figur 4: Vergleich der codierenden Sequenzen für reifes EqIFN-γ des natürlichen Gens (eq) und des synthetischen (syn), für die Expression in E.coli optimierten Gens. Sich unterscheidende Basen sind mit einem Stern markiert.

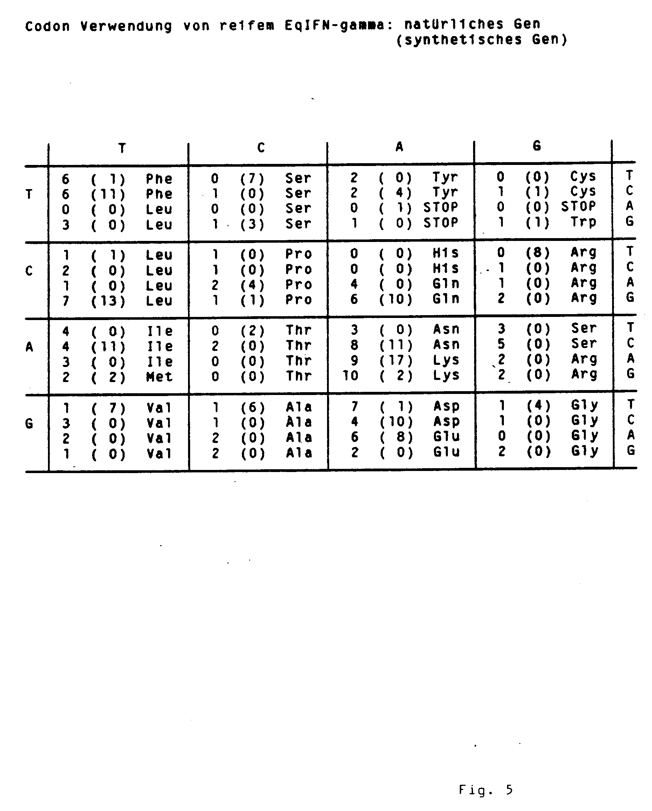

Figur 5: Tabelle für die verwendeten Codons für reifes EqIFN-γ. Am linken Rand ist die erste Base, in der Mitte die zweite Base und am rechten Rand die dritte Base des Codons angegeben. Die Tabelle zeigt die Anzahl der verwendeten Codons für die jeweilige Aminosäure im natürlichen Gen, sowie in Klammern die des synthetischen Gens.

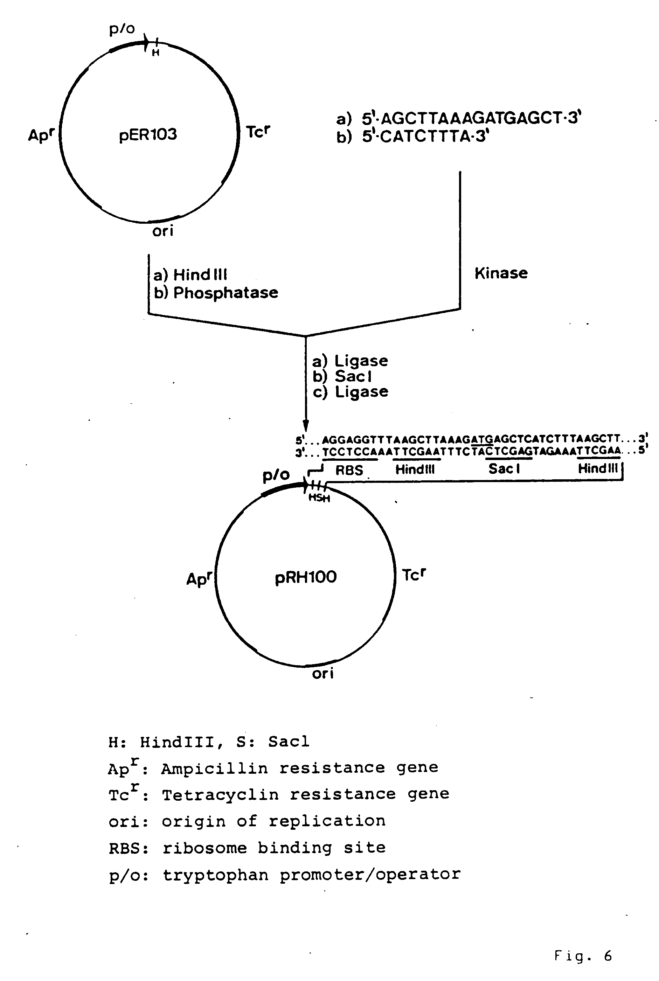

Figur 6: Konstruktion des Expressionsplasmides pRH100.

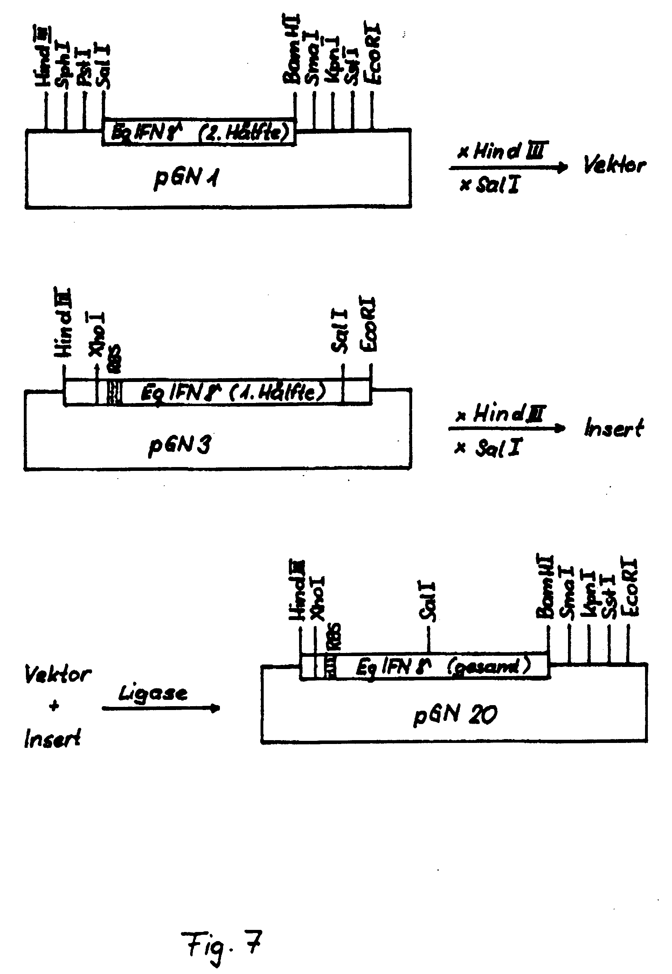

Figur 7: Konstruktion und Restriktionskarte des pGN20.

Die folgenden Beispiele, die die Erfindung nicht eingrenzen sollen, beschreiben die Erfindung im Detail.

Materialien

Die Ausgangsmaterialien wurden teilweise käuflich erworben, teilweise stammten sie vom EMBL in Heidelberg. E.Coli JM101, pUC9 und M13mp8 stammten von den Bethesda Research Laboratories, die E.coli Stämme mit dem Suppressorfaktor sup F, beispielsweise E.coli NM526, 538 und 539 und der Vektor lambda-EMBL3 oder 3A stammten vom EMBL und sind auch bei der Firma Stehelin/Basel (Schweiz) erhältlich.

1. Isolierung von Pferde-DNA

Gefrorenes Gewebe, z.B. Pferdeleber wurde in flüssigem Stickstoff zu feinem Pulver zermahlen und in 0,5M EDTA, 10 mM Tris-HCl pH 8,0, 0,5% SDS, 0,1 mg/ml Proteinase K (20 ml/g Gewebe) 3 Stunden bei 55 °C inkubiert. Die erhaltene, viskose Lösung wurde durch eine Phenolextraktion und 3-malige Extraktion mit Phenol/Chloroform/Isoamylalkohol (25/24/1 Vol) von Protein befreit, gegen 50mM Tris-HCl pH 8,0, 10mM EDTA, 10mM NaCl dialysiert und die DNA mit 2 Volumina Äthanol ausgefällt. Nach vollständiger Trocknung in Vakuum wurde die DNA in TE-Puffer (10mM Tris-HCl pH 8,0, 1mM EDTA) bei 4 °C in Lösung gebracht und mit 1,273 g CsCl/ml Lösung 62 Stunden mit 40.000 UpM bei 20 °C zentrifugiert (Sorvall 50Ti-Rotor). Der CsCl-Gradient wurde ausgetropft, die DNA enthaltenden Fraktionen gegen TE-Puffer dialysiert und die DNA anschließend mit 2 Volumina Äthanol gefällt, mit 70%igem Äthanol gewaschen, getrocknet und wieder in TE-Puffer gelöst (4 °C).

Das fertige DNA-Präparat war frei von RNA und länger als 50 kb (durch Elektrophorese an einem 0,45%igem Agarosegel bestimmt).

2. Partielle Endonuklease Verdauung und Größenfraktionierung von Pferde-DNA

Zweimal 50μg Pferde-DNA wurden mit 1,6 Einheiten Sau3A in 450μl Reaktionsmedium (10 mM Tris-HCl pH 7,5, 10 mM $MgCl_2$ 1mM Dithiothreitol) bei 37 °C inkubiert. Nach 15, 25 und 40 Minuten wurden 150 μl Aliquots entnommen und mit 15mM EDTA versetzt. Durch 10minütiges Erwärmen auf 70 °C wurde die Reaktion gestoppt. Nach Zusatz von 0,3M NaAcetat pH6,0 wurde die DNA mit 2,5 Volumina Äthanol gefällt. Nach dem Wiederauflösen in TE-Puffer wurde die DNA auf einem 0,45%igen Agarosegel in TBE-Puffer (10,8g/l Tris, 5,5g/l Borsäure, 0,93g/l $Na_2$EDTA) bei ca. 1 V/cm über Nacht elektrophoretisch der Größe nach getrennt. Anhand von Größenmarkern (mit EcoRI und HindIII doppelverdaute und mit HindIII verdaute Lambda-DNA) wurde das Gelstück mit DNA einer Länge von 10-23kb herausgeschnitten, die DNA aus dem Gel in einen Dialysenschlauch 3 Stunden bei 300V elektroeluiert (Puffer 0,1 x TBE), auf einer Elutip-D-Säule (Schleicher und Schüll) gemäß der Verwendungsvorschrift gereinigt und anschließend mit Äthanol gefällt.

Um die Selbstligation von Pferde-DNA-Fragmenten zu verhindern, was einerseits zu artifiziellen Hybriden von Pferde-DNA-Sequenzen, andererseits zu zu großen und damit nicht mehr in Lambda-Phagen verpackbaren DNA-Stücken führen kann, wurden die größenfraktionierten Pferde-DNA-Stücke dephosphoryliert.

Dazu wurde die DNA in 140μl Reaktionsmedium (50 mM Tris-HCl pH 9,5, 10mM $MgCl_2$, 0,1 mM ZnAcetat, 1 mM Spermidin) mit 5 Einheiten Bovine Intestinal Phosphatase 30 Minuten 37 °C inkubiert, weitere 5 Einheiten Enzym zugegeben und 30 Minuten inkubiert. Nach Zugabe von EDTA auf 25mM Endkonzentration wurde die DNA einmal mit Phenol/Chloroform/Isoamylalkohol (25/24/1 Vol), 2-mal mit Chloroform/Isoamylalkohol (24/1 Vol) und 3-mal mit Diäthyläther extrahiert mit Äthanol gefällt, getrocknet und in 0,1 x TE-Puffer gelöst.

3. Aufbau der Pferdegenom-DNA-Bibliothek

Die dephosphorylierten 10-23kb Pferde-DNA-Fragmente wurden in einem Lambda-Vektor, beispielsweise Lambda-EMBL3 oder -3A (Frischauf, A.M. et al. J. Mol. Biol., 170, 827-842 (1983)) mit G-A-T-C kohäsiven Enden, durch Entfernung des internen BamHI-Fragmentes der Phagen-DNA gewonnen, geklont.

Der Vektor wurde in einem E.coli Stamm mit Suppressorfaktor sup F, beispielsweise E.coli NM526, 538 oder 539 (Frischauf, A.M. et al. J. Mol. Biol., 170, 827-842 (1983)), in LB-Brühe (Miller; Experiments in Molecular Genetics; Cold Spring Harbor Lab. Cold Spring Harbor, New York) mit 5mM $MgSO_4$ gezüchtet, mit Polyethylenglykol gefällt und durch 2-malige CsCl-Dichtegradientenzentrifugation (0,71 g CsCl/ml Lösung, 40 Stunden 45.000 UpM, 20 °C) gereinigt. Nach Dialyse gegen TE-Puffer wurde die Phagen-DNA durch 2-malige Extraktion mit Phenol-Chloroform/Isoamylalkohol (25/24/1 Vol) und 2-malige Extraktion mit Chloroform/Isoamylalkohol (24/1 Vol) von Protein befreit und durch Äthanolfällung aufkonzentriert.

Zur Gewinnung der Endfragmente von EMBL3A wurden 50µg Phagen-DNA 2 Stunden bei 37°C in 450µl Reaktionsmedium (10 mM Tris-HCl pH 7,5, 10 mM $MgCl_2$, 1 mM Dithiothreitol) vollständig mit BamHI verdaut, mit 15mM EDTA 10 Minuten bei 70°C die Reaktion gestoppt und die DNA mit Äthanol gefällt.

Zur Vermeidung der Religation wurde das Mittelfragment mit EcoRI nachgeschnitten und das wegfallende Oligonukeotid mittels Isopropanolfällung entfernt.

Die BamHI-verdaute Lambda-DNA wurde hierzu 2 Stunden bei 37°C in 450µl 10mM Tris-HCl pH 7,5 100 mM NaCl, 10 mM $MgCl_2$ mit EcoRI vollständig verdaut und die Reaktion durch Zusatz von 15mM EDTA und 10minütiges Erwärmen auf 70°C gestoppt. Nach Zusatz von NaAcetat zu einer Endkonzentration von 0,3M wurden die 3 großen DNA-Fragmente mit 0,6 Volumina Isopropanol 15 Minuten bei 0°C gefällt, 2-mal mit 0,45M NaAcetat/0,6 Volumina Isopropanol und 1-mal mit 0,3M NaAcetat/2,5 Volumina Äthanol gewaschen und in 15µl 0,1x TE-Puffer gelöst. Die BamHI/EcoRI-Linker bleiben bei dieser Prozedur in Lösung. Die EMBL3A Fragmente (8µg) wurden mit ca. 5µg 10-23kb Pferde-DNA und 10 Einheiten T4-DNA-Ligase (NEN) vereinigt und über Nacht bei 14°C und einen Tag bei 4°C in 50µl Ligationsmedium (66mM Tris-HCl pH 7,2, 0,1M NaCl, 10mM $MgCl_2$, 1mM EDTA, 5mM Dithiothreitol, 0,5mM ATP) inkubiert. Das ligierte DNA-Gemisch wurde mittels eines in vitro-Lambda-Verpackungssystems (Scalenghe, F. et al; Chromosoma, 82, 205-216 (1981)) in reife Lambda-Phagenpartikel gepackt.

Die Komponenten dieses Systems, d.h. Ultraschallextrakt (SE), Gefrier-Auftau-Lysat (FTL), Puffer MI und A wurden gemäß (Scalenghe, F. et al; Chromosoma, 82, 205-216 (1981)) hergestellt. 10µl Aliquots des ligierten DNA-Gemisches wurden 2 Minuten bei Raumtemperatur mit 25µl SE inkubiert, das ebenso wie FTL 30 Minuten aus Eis aufgetaut worden war, mit 100µl FTL versetzt und 60 Minuten bei Raumtemperatur weiterinkubiert. Das Packungsgemisch wurde mit 150µl Lambda-Diluent (100 mM Tris-HCl pH 7,5, 10mM $MgSO_4$ 1mM EDTA) verdünnt und bei 4°C gelagert.

4. Klonung und Sequenzierung des Gens für Pferde gamma-Interferon (EqIFN-γ)

A. Isolierung eines vollständigen EqIFN-γ Gen Klons

Die equine DNA-Bibliothek wurde zur Infektion des E.coli Stammes NM528 (supF) verwendet. Eine über Nacht in LB-Nährlösung (10 g/l Trypton, 5 g/l Hefe Extrakt, 10 g/l NaCL, pH 7,4) mit 0,2% Maltose gewachsene Bakterienkultur wurde in 10 mM $MgSO_4$ auf eine optische Dichte (600 nm) von 2,0 eingestellt. Je 0,5 ml dieser Suspension wurden mit 50.000 pfu (plaquebildende Einheiten) Lambda-Phagen der DNA-bibliothek infiziert und mit einer LB-Weichagarschicht auf LB-Agar-Platten mit 10 mM $MgSO_4$ (13,5 cm Durchmesser) verteilt. Insgesamt wurden $1{,}5x10^6$ rekombinante Lambda-Phagen gescreent. Nach Bebrüten über Nacht bei 37°C wurden von den Phagen jeder Platte zweifache Replikas auf Nitrozellulose hergestellt (Benton und Davis, Science 196:180-182, 1977). Nach Denaturieren der Phagen-DNA (1 min in 0,5 N NaOH, 1,5 M NaCl), Neutralisieren (2-mal 3 min in 0,5 M Tris-HCl pH 7,5, 1,5 M NaCl) und Spülen (1 min in 2xSSC, 1xSSC, 0,15 M NaCl, 15 mM NaCitrat) wurden die Filter an der Luft getrocknet und die DNA durch 2-stündiges Backen bei 80°C fixiert. Die Filter wurden über Nacht bei 65°C in einer Lösung von 1,5 M NaCl, 10 mM Tris-Hcl pH 8,0, 0,1% SDS gewaschen und 4 bis 6 Stunden bei 65°C vorhybridisiert.
(Hybridisierlösung:
0,9 M NaCl, 50 mM $NaH_2PO_4$, pH 7,4, 5 mM EDTA, 0,1% Ficoll, 0,1% Polyvinylpyrrolidon, 0,1% Rinderserumalbumin, 0,1% SDS, 20 mg/ml beschallte und denatuierte Lachssperma-DNA). Die Hybridisierung erfolgte in einer frischen Lösung mit $10^6$ cpm pro Filter einer nach üblichen Methoden radioaktiv markierten HuIFN-γ "probe", 20 Stunden bei 65°C. Die Filter wurden unter nicht stringenten Bedingungen in 3xSSC, 0,1% SDS bei 65°C gewaschen, getrocknet und autoradiographiert. Nach drei Durchgängen von Plaque-Reinigung konnten 5 Lambda Klone identifiziert werden, die positive Hybridisierungssignale ergaben.

Aus diesen isolierten rekombinanten Phagen wurde nach üblichen Methoden (Maniatis et al., ibid.) die DNA gereinigt. Die Phagen-DNAs wurden nach Verdauung mit verschiedenen Restriktionsenzymen und nachfolgender Southern-Analyse (Southern, J.Mol. Biol. 98: 503-517, 1975) durch Hybridisierung mit der HuIFN-γ "probe" charakterisiert. Ein singuläres hybridisierendes 4,6 kb langes BamHI Fragment des Klons Lambda Eq-γ2 wurde isoliert und in die BamHI Schnittstelle des Plasmids pUC9 (Vieira und Messing, Gene 19: 259-268, 1982) geklont. Nach Transformation von E.coli JM101 wurde aus erhaltenen Kolonien in einem Minipräparationsverfahren (Birnboim und Doly, Nucl. Acids Res. 7: 1513-1523, 1979) Plasmid-DNA hergestellt und durch Verdauung mit Restriktionsenzymen charakterisiert. Ein Plasmid mit dem gewünschten BamHI Insert wurde pAH111 benannt. Die Ränder des BamHI Inserts von Plasmid paH111 wurden nach Einbringung in die M13mp8 bzw. M13mp9 Vektoren (Vieira und Messing, Gene 19, 259-268, 1982) nach der Didesoxy-Methode sequenziert (Sanger et al., Proc. Natl. Acad. Sci. USA 74: 5463-5467, 1977). Ein Sequenzvergleich mit dem humanen gamma-Interferon-Gen (Gray und Goeddel, Nature 298: 859-863, 1982) zeigte hohe Homologie mit den nichtkodierenden 5′- und 3′-Regionen. Daraus wurde geschlossen, daß das vollständige EqIFN-γ Gen isoliert wurde.

B. Sequenzierung des Pferde gamma-Interferongens aus Klon Lambda Eq-γ2

Das 4,6 kb lange BamHI Insert von Plasmid pAH111 wurde nach der Didesoxy-Methode vollständig sequenziert. Die Gesamtsequenz des BamHI-Fragments wurde durch Kombination von Teilsequenzen von M13-Subklonen ermittelt, die durch gerichtete Klonierung von Restriktionsfragmenten (EcoRI, HindIII, PstI, PstI-BglII, HindIII-BamHI) in entsprechend geschnittene M13mp8 oder M13mp9 Vektoren erhalten wurden. Weitere Teilsequenzen wurden erhalten, indem das 2,0 kb lange BamHI-BglII Fragment, bzw. das 2,0 kb lange PstI Fragment nach der "Shotgun-Methode" in den M13mp8 Vektor geklont wurde. Die beiden DNA-Fragmente wurden durch Ultraschall in kleinere Stücke zerteilt, die DNA-Enden durch Inkubation mit E.coli DNA-Polymerase I (Klenow-Fragment) in Gegenwart von je 0,1 mM aller vier Desoxynukleotidtriphosphate abgestumpft (Reaktionspuffer: 50 mM Tris-HCl pH 7,5, 10 mM $MgCl_2$, 1 mM Dithiothreit, 0,5 mg/ml Rinderserumalbumin: 1 h bei 25°C). Nach Größenfraktionierung in einem Agarosegel wurden DNA-Fragmente einer Länge von etwa 0,4 - 1,0 kb isoliert und in die SmaI Schnittstelle des M13mp8-Vektors ligiert.
Die erhaltenen Teilsequenzen wurden mittels eines Computerprogrammes zu der 4664 bp langen Totalsequenz vereint, die in Fig. 1 dargestellt ist.

Durch computerunterstützte Analyse der offenen Leserahmen und Vergleich mit von gamma-Interferongenen anderer Spezies (Gray und Goeddel, Nature 298: 859-863; Gray und Goeddel, Proc. Natl.Acad.Sci.USA 80: 5842-5846, 1983; Dijkema et al., EMBO J. 4: 761-767, 1985; Cerretti et al., J. Immunology 136: 4561-4564, 1986) wurde der proteinkodierende Bereich des equinen gamma-Interferongens ermittelt. Die proteinkodierende Region ist durch drei Introns unterbrochen, wobei im ersten Exon das 20 Aminosäuren lange, hydrophobe Signalpeptid und 18 Aminosäuren des reifen EqIFN-γ Polypeptides kodiert sind (Basen 366-479). Das zweite Exon kodiert für die Aminosäuren 19-41 (Basen 1639-1707), das dritte Exon kodiert für die Aminosäuren 42-102 (Basen 1803-1985), das vierte Exon kodiert den Carboxy-Terminus mit den Aminosäuren 103-146 (Basen 3307-3441). An den Positionen 4010 und 4020 befinden sich zwei Signalsequenzen (AATAAA) für die Polyadenylierung der mRNA. An den Positionen 86-88 des reifen EqIFN-γ Polypeptides befindet sich die einzige potentielle N-Glykosylierungsstelle (ASN-Ser-Ser), welche mit der zweiten N-Glykosylierungsstelle des bovinen gamms-Interferons (Asn-Gly-Ser) zusammenfällt (Fig. 2). Das reife EqIFN-γ Polypeptid enthält überraschenderweise nur einen einzigen Cysteinrest an Position 3, wobei in Analogie zu den natürlichen humanen und murinen gamma-Interferonen wahrscheinlich die ersten drei amino-terminalen Aminosäuren (in diesem Fall Tyr-Tyr-Cys) im Organismus proteolytisch abgespalten werden.

5. Herstellung eines synthetischen Gens für reifes EqIFN-γ

Um rekombinantes EqIFN-γ in seiner reifen Form in Escherichia coli zu exprimieren, wurde ein synthetisches Gen aus Oligonukleotiden konstruiert. Es kodiert für dieselbe Aminosäuresequenz wie das natürliche EqIFN-γ Gen, enthält jedoch ausschließlich solche Codons für die einzelnen Aminosäuren, die in von E.coli hoch exprimierten, zelleigenen Genen verwendet werden (Gouy und Gautier, Nucl. Acids Res. 10: 7055-7074, 1982). Zusätzlich wurden mehrere singuläre Restriktionsenzym-Schnittstellen eingebaut, die eine einfache Manipulation des Gens für die Veränderung einzelner Abschnitte möglich macht. Das synthetische Gen für EqIFN-γ wurde in zwei Varianten aus insgesamt 16 verschiedenen Oligonukleotiden konstruiert. Die erste Variante kodiert für reifes EqIFN-γ mit 146 Aminosäuren plus Start-Methionin, die zweite Form für ein am Amino-Terminus um 3 Aminosäuren (Tyr-Tyr-Cys) verkürztes Polypeptid plus Startmethionin, wie es vermutlich im natürlichen Organismus auftreten dürfte.

Die Struktur des synthetischen EqIFNγ Gens ist in Fig. 3 dargestellt. Die für die Herstellung verwendeten Oligonukleotide wurden mit Hilfe eines Applied Biosystems Model 381A DNA-Synthesizer synthetisiert, durch Elektrophorese in denaturierenden 12%-igen Polyacrylamidgelen (7 M Harnstoff) gereinigt und mittels Ausschlußchromatographie über Sephadex G-25 (Pharmacia) entsalzt.

Zusammenbau der Oligonukleotide zum synthetischen EqIFN-γ Gen

Der Zusammenbau des synthetischen EqIFN-γ Gens erfolgte in zwei Abschnitten. Der erste Teil des Gens wurde mit Hilfe der acht Oligonukleotide EG-1 bis EG-8 bis zur SalI Schnittstelle hergestellt, die zweite Hälfte des Gens aus den sechs Oligonukleotiden EG-9 bis EG-14 von der SalI Schnittstelle bis zur BamHI Schnittstelle. Für die am Amino-Terminus um drei Aminosäuren verkürzte Form des EqIFN-$\gamma$ wurden statt der Oligonukleotide EG-1 und EG-2 die Oligonukleotide EG-15 und EG-16 verwendet.
Die zueinander komplementären Oligonukleotide wurden jeweils paarweise am 5'-Ende phosphoryliert. Jeweils 100 pMol der beiden Oligonukleotide (z.B. EG-3 und EG-4, bzw. EG-5 und EG-6 usw.) wurden in 9 µl Kinase-Puffer (70mM Tris-HCl pH 7,6, 10 mM $MgCl_2$, 5mM Dithiothreit), 2µCi [$\gamma$-$^{32}$P]ATP (Amersham) mit 10 Einheiten T4-Polynukleotidkinase (New England Biolabs) 10 min bei 37 °C inkubiert, 1 µl einer 10 mM ATP-Lösung zugefügt und weitere 50 min bei 37 °C inkubiert. Die Reaktion wurde durch 10-minütiges Erhitzen auf 95 °C gestoppt. Um die spätere Verknüpfung der DNA-Enden zu verhindern, wurden die Oligonukleotide EG-1, EG-15, EG-9 und EG-14 nicht phosphoryliert. Sie wurden erst nach Inaktivierung der Polynukleotidkinase mit dem jeweils komplementären Oligonukleotide gemischt, 5 min auf 95 °C erhitzt und auf Raumtemperatur abgekühlt.

Die Mischungen der Oligonukleotide EG-1 + 2 (bzw. in einem zweiten Ansatz EG-15 + 16), EG-3 + 4, EG-5 + 6 und EG-7 + 8 wurden vereinigt, mit 1 µl 5 M NaCl versetzt, 5 min auf 70 °C erhitzt und auf Raumtemperatur abgekühlt. Zu dieser Lösung wurden 5µl 10 mM ATP, 2 µl Dithiothreit, 1,5 µl 10x Ligationspuffer (0,66 M Tris-HCl pH 7,2, 1 M NaCl, 100 mM $MgCl_2$, 10 mM EDTA, 50 mM Dithiothreit) und 80 Einheiten T4 DNA-Ligase (New England Biolabs) zugesetzt und 48 Stunden bei 4 °C inkubiert. Der Verlauf der Ligase-Reaktion wurde durch gelelektrophoretische Auftrennung der DNA-Fragmente eines kleinen Reaktionsanteils in einem 5%-igen, nicht denaturierenden Polyacrylamidgel und anschließender Autoradiographie verfolgt. In gleicher Weise wurden die sechs Oligonukleotide EG-9 bis EG-14 miteinander verknüpft. Die Reaktion wurde durch Phenol/Chloroform-Extraktion gestoppt und die DNA durch Äthanolfällung wiedergewonnen.

6. Konstruktion des Expressionsplasmides pRH 100

Alle Enzymreaktionen wurden unter den von den Herstellern angegebenen Bedingungen durchgeführt.

7 µg Plasmid pER103 (Eva Dworkin-Rastl et al., Gene 21 (1983) 237-248, EP-A-0.115-613) wurden in 50 µl Reaktionsmedium mit der Restriktionsendonuklease HindIII linearisiert. Nach einer einstündigen Inkubation bei 37 °C wurden 50 µl 2x CIP-Puffer zugesetzt (2x CIP-Puffer = 20 mM Tris, pH = 9,2, 0,2 mM EDTA). Durch Zugabe von 2 Einheiten alkalischer Phosphatase aus Kälberdarm (CIP) wurden die 5'terminalen Phosphatreste entfernt; inkubiert wurde bei 45 °C 30 Minuten lang. Die Reaktion wurde durch Zugabe von 4 µl 0,5 mM EDTA-Lösung sowie Zugabe von 10 µl 1M Tris, pH = 8,0 Lösung gestoppt. Die Proteine wurden durch zweimalige Phenol- und einmalige Phenol-/Chloroformextraktion entfernt. Die DNA wurde aus der wäßrigen Phase nach Zugabe von 0,1 vol 3M Natriumacetatlösung pH = 5,5 und 250 µl Äthanol ausgefällt, das DNA-Präzipitat nach Zentrifugieren einmal mit 70%-iger Äthanollösung gewaschen. Die DNA wurde getrocknet, und das Pellet anschließend in 20 µl TE-Puffer (10 mM Tris pH = 8,0, 1 mM EDTA) gelöst.

Jeweils 1 µg der synthetisch hergestellten Oligonukleotide d(AGCTTAAAGATGAGCT) und d-(CATCTTTA) wurden in je 10 µl Reaktionslösung unter Zugabe von 10 Einheiten T4-PNK (Polynukleotidkinase) sowie 1 mM rATP phosphoryliert. Die Reaktion fand bei 37 °C statt und dauerte 45 Minuten. Die Reaktion wurde durch 10-minütiges Erhitzen auf 70 °C abgebrochen.

Jeweils 5 µl der Plasmidlösung und der phosphorylierten Oligonukleotide wurden gemischt und 5 Minuten auf 70 °C erwärmt. Danach wurde die Lösung auf 0 °C abgekühlt, 2 µl 10x Ligasepuffer (500 mM Tris, pH = 7,5), 100 mM $MgCl_2$ 200 mM DTT (Dithiothreit), 1 mM rATP, 500 µg/ml BSA (Rinderserumalbumin), sowie 2 µl Wasser und 10 Einheiten T4-DNA Ligase zugesetzt. Die Reaktion dauerte 40 Stunden und wurde bei 4 °C durchgeführt. Sie wurde durch 10-minütiges Erhitzen auf 70 °C abgebrochen.

2 µl dieser Ligasereaktion wurden in insgesamt 30 µl Lösung mit 10 Einheiten der Restriktionsendonuklease SacI (New England Biolabs) 3 Stunden bei 37 °C verdaut. Die Reaktion wurde durch 10-minütiges Erhitzen auf 70 °C abgebrochen. 5 µl dieser Reaktionsmischung wurden in insgesamt 30 µl durch Zugabe von 10 Einheiten T4-PNK bei 14 °C 16 Stunden lang ligiert.

200 µl kompetenter E.coli Hb101 wurden mit 10 µl dieser Ligasereaktion versetzt. Die Bakterien wurden 45 Minuten auf Eis gehalten und anschließend 2 Minuten auf 42 °C erwärmt, um die DNA-Aufnahme zu ermöglichen. Anschließend wurde die Bakteriensuspension wieder bei 0 °C 10 Minuten inkubiert. Abschließend wurden die transformierten Bakterien auf einem LB-Agar, der 50 µg/ml Ampicillin enthielt, ausgestrichen.

Von den entstandenen Bakterienkolonien wurden 12 willkürlich ausgesucht und daraus im Mikromaßstab die Plasmide isoliert (Birnboim und Doly, Nucl. Acids Res. 7 (1979) 1513-1523). Die resultierende DNA wurde mit der Restriktionsendonuklease SacI geschnitten und die DNA auf einem Agarosegel (1%, 1x TBE-Puffer) aufgetrennt. Die Wanderung der DNA als lineares Molekül der Größe von etwa 4.400 bp bestätigte die Einführung einer SacI-Erkennungsstelle in das Plasmid. Eines dieser Plasmide wurde willkürlich ausgesucht. Mit der DNA aus der dazugehörigen Minipräparation wurde nochmals E.coli HB101 transformiert. Von den resultierenden transformierten Bakterien wurde eine Kolonie ausgewählt und in größerem Maßstab angezüchtet. Das daraus isolierte Plasmid wurde mit den Restriktionsendonukleasen EcoRI und BamHI geschnitten, die DNA auf einem 1%-igem Agarosegel aufgetrennt, und das kleinere Fragment aus dem Gel durch Elektroelution isoliert. Dieses etwa 460 bp lange EcoRI-BamHI DNA-Fragment wurde nach Sanger sequenziert (F. Sanger et al., Proc.Natl.Acad. Sci. (1977) 5463-5467). Das dermaßen analysierte Plasmid wurde pRH100 genannt.

7. Einbringung des synthetischen EqIFN-$\gamma$ Gens in das Expressionsplasmid pRH100

10 $\mu$g Plasmid pRH100 werden in 100 $\mu$l Reaktionspuffer mit SacI vollständig geschnitten und das Enzym durch 10-minütiges Erhitzen auf 70°C inaktiviert. Die überhängenden DNA-Enden werden durch Behandlung mit Klenow-Fragment (Amersham) in Gegenwart von je 10 $\mu$M aller vier Desoxynukleotidtriphosphate begradigt (30 min, 25°C). Die Reaktion wird durch Phenol/Chloroform-Extracktion gestoppt und die DNA druch Äthanolfällung konzentriert. Durch diese Behandlung entsteht im Anschluß an den Tryptophanpromotor ein stumpfes DNA-Ende, das mit dem Translationsstartcodon "ATG" endet. Die linearisierte Plasmid-DNA wird mit BamHI nachgeschnitten und der Vektoranteil nach elektrophoretischer Auftrennung aus einem Agarosegel isoliert.

50 ng des wie beschrieben vorbereiteten pRH100 Plasmid-Vektors werden mit 20 pMol der ligierten Oligonukleotide EG-1 bis EG-8 und EG-9 bis EG-14 gemischt und in 10 $\mu$l Ligationspuffer (66 mM Tris-HCl pH 7,2, 100 mM NaCl, 10 mM $MgCl_2$, 1mM EDTA, 5 mM Dithiothreit, 1 mM ATP) mit einer Einheit T4 DNA-Ligase (Boehringer Mannheim) 24 h bei 24°C inkubiert. Durch Behandlung mit Klaziumchlorid kompetent gemachter E.coli JM101 wird mit diesem Ligationsgemisch transformiert und über Nacht bei 37°C inkubiert. Von den erhaltenen Transformanten wird im Minipräparationsverfahren Plasmid-DNA isoliert und durch Restriktionsanalyse und Sequenzierung des HindIII-BamHI Inserts die Struktur ermittelt. Ein Plasmid der gewünschten Struktur für die Expression von reifem EqIFN-$\gamma$ wird pEqG-YYCl benannt.
In völlig analoger Weise werden die Oligonukleotdie EG-15,16 EG-3 bis EG-8 und EG-9 bis EG-14 in den pRH100 Vektor geklont, um das um drei Aminosäuren verkürzte EqIFN-$\gamma$ zu erhalten. Ein Plasmid der gewünschten Struktur wird pEqG-QAA1 benannt.

8. Expression der Interferonaktivität durch E.coli JM101, das Plasmid pEqG-YYC1 oder pEqG-QAA1 enthaltend

100 ml Bakterienkultur werden bei 37°C unter kräftigem Schütteln in folgendem tryptophanfreiem Medium inkubiert (Angaben pro Liter Medium): 10 g $(NH_4)_2PO_4$, 3,5 g $KH_2PO_4$ pH 7,3 mit NaOH, 0,5 g NaCl, 21 g Casaminosäuren (sauer hydrolisiert), 11 g Glucose, 1 mM $MgSO_4$, 0,1 mM $CaCl_2$, 1 mg Thiamin-HCl, 20 mg L-Cystein, 20 mg 3-$\beta$-Indolacrylsäure (IAA, Induktor für das Tryptophan-Operon), optionsweise 50-100 mg Ampicillin. Anschließend werden die Bakterien durch Zentrifugieren 5 Minuten bei 4000 UpM pelletiert, mit 1/10 des Kulturvolumens eiskaltem 50 mM Tris-HCl pH 8,0, 30 mM NaCl suspendiert und unter Eiskühlung zweimal 30 Sekunden mittels Ultraschallsonde (20 kHz, 100 Watt) aufgebrochen. Die Zelltrümmer werden 10 Minutes bei 10.000 UpM (4°C) entfernt und der Überstand nach Sterilfiltration auf Interferonaktivität in einem Assay, der den cytophatischen Effekt (CPE) von Vesicular Stomatitis Virus (VSV) oder Encephalomyocarditis Virus (EMCV) mißt, getestet.

Testsystem: NBL-6 Zellen (ATCC CCL 57, Pferdehaut-Epidermis-Zellen)/VSV A549 (ATCC CCL 185, humane Lungencarcinom-Zellinie)/EMCV

Der Titer auf A 549-Zellen wird mit human-Interferonstandard auf internationale Einheiten normiert.

9. Nachweis der exprimierten Pferde-Interferone durch Markierung der Proteine in Maxizellen

Plasmidcodierte Proteine können durch Verwendung der Maximzelltechnik selektiv in vivo markiert werden. E.coli CSR603 wird nach üblichem Verfahren mit den Expressionsplasmiden transformiert und auf Ampicillin-haltigen Agarplatten auf transformierte Bakterien selektioniert. Die Präparation der Maxizellen und das Markieren der Proteine erfolgt nach einer Vorschrift von A. Sancar (a.a.O.). Die Zellen werden in 15 ml Medium (siehe Beispiel 8) ohne Indolacrylsäure bei 37°C bis zu einer $OD_{600nm}$ = 0,5 gezüchtet und 10 ml dieser Kultur in einer Petrischale 5 Sekunden aus 50 cm Entfernung mit einer UV-Germicid-Lampe (15 Watt) unter Schwenken bestrahlt und eine Stunde bei 37°C weiterinkubiert. Die Kulturen werden mit 100 µg/ml D-Cycloserin versetzt, 14 Stunden bei 37°C inkubiert und die Bakterien asnchließend durch Zentrifugation geerntet. Die Zellen werden zweimal mit 5 ml Hershey-Salzlösung gewaschen, in 5 ml Hershey Medium mit 20 µg/ml Indocrylsäure suspendiert und 2 Stunden bei 37°C inkubiert. Zu jeder Kultur werden 5 µCi/ml $^{35}$S-Methionin (1000 Ci/mMol) zugesetzt und eine Stunde bei 37°C geschüttelt. Die Zellen werden geerntet in SDS- und 2-Mercaptoethanol-haltigem Elektrophorese-Probenpuffer lysiert und die Proteine auf einem 15%-igem Polyacrylamidgel aufgetrennt.

| Hershey-Salzlösung (pro Liter) | Hershey Medium (pro 100ml Hershey-Salzlösung) | |
|---|---|---|
| 5,4 g NaCl | 2 ml | 20% Glucose |
| 3,0 g KCl | 0,5 ml | 2% Threonin |
| 1,1 g $NH_4Cl$ | 1,0 ml | 1% Leucin |
| 15mg $CaCl_2.2H_2O$ | 1,0 ml | 2% Prolin |
| 0,2 g $MgCl_2.6H_2O$ | 1,0 ml | 2% Arginin |
| 0,2 mg $FeCl_3.6H_2O$ | 0,1 ml | 0,1% Thiamin |
| 87 mg $KH_2PO_4$ | | |
| 12,1 g Tris-HCl pH 7,4 | | |

Ein Autoradiogramm des getrockneten Geles wird nach 2 Tagen Exponierung auf DuPont Cronex x-ray Film unter Verwendung einer Kodak Lanex-Regular Verstärkerfolie bei -80°C dargestellt. Als Molekulargewichtsstandard wird ein $^{14}$C-methyliertes Proteingemisch (Amersham) verwendet. Als Kontrollen dienen das Plasmid pER103, das nur den Promotor ohne Interferongen enthält und das Plasmid pER21/1, das zwei Kopien des humanen IFN-α2arg Genes enthält.

10. Nachweise von mit EqIFN-γ hybridisierenden Sequenzen in genomischer Pferde-DNA

Zum Nachweis der Gesamtanzahl von Sequenzen im Pferdegenom, die hohe Homologie mit dem Interferongen EqIFN-γ aufweisen, wird wie folgt vorgegangen. 30 µg hochmolekulare Pferde-DNA (Beispiel 1) werden mit 100 Einheiten des entsprechenden Restriktionsenzym in 300 µl Reaktionsvolumen vollständig verdaut und jeweils 10 µg dieser geschnittenen DNA pro Spur auf einem 0,8%igem Agarosegel nach der Größe aufgetrennt.

Nach Southern-Transfer auf Nitrozellulosefilter, Denaturieren und Fixieren der DNA wird jedes Filter mit etwa 6x$10^6$ cpm radioaktiv markierter "probe" hybridisiert (17 Stunden bei 65°C, 5x SSC, 5x Denhardt-Lösung, 0,1% SDS, 20 µg/ml denaturierte Lachssperma-DNA). Als "probe" für EqIFN-γ wird ein Fragment aus dem Plasmid pEqG-YYC1 verwendet, das die codierende Sequenz für das gesamte reife Interferon enthält. Die Filter werden anschließend unter stringenten Bedingungen gewaschen: 4mal 45 Minuten bei 65°C mit 0,3x SSC (45 mM NaCl, 4,5 mM $Na_3$Citrat), 0,1% SDS. Die Autoradiographie erfolgt auf DuPont Cronex X-ray Film unter Verwendung von Kodak Lanex-Regular Verstärkerfolie 7 Tage bei -80°C.

11. Expression von equinem Interferon-gamma (QAA) in E.coli HB101/pEqG-QAA2 und HB101/pEqG-QAA3.

Um zu einer verbesserten Expression zu gelangen, wurden a) verbesserte Expressionsvektoren und b) eine verbesserte ribosomale Bindungsstelle verwendet. Die verbesserten Expressionsvektoren basieren auf dem trp-Promotor aus Serratia marcescens (Sma), der in der -35 Region an die Konsenus -35 Region durch einen Basenaustausch angeglichen ist (pRH281), bzw. auf einem Hybrid-trp Promotor, der die erste A/T-reiche Region des Escherichia coli (Eco) bzw. die zweite A/T-reiche Region plus Promotor von Sma besitzt (pRH282, S. Itoh, Gene 44 (1966), 29-36). Als ribosomale Bindungsstelle wurde jene des E.coli Enterotoxin II verwendet.

a) pRH281/5

Folgende Oligonukleotide wurden mittels eines Applied Biosystems DNA Synthesizer 381A hergestellt:

```
Trp-1: 5'-AATTGACGCTG-3'
Trp-3: 5'-ATCGCTAAAACATTGTGCAAAAAGAGGGTTGACATTGCCTTCGCGAACCA
          GTTAACTAGTACACA-3'
Trp-5: 5'-AGTTCACGGCTCGAGACGGTAAGGAGGTTTAATATGAGCTCGAATTCAT-3'
Trp-2: 5'-TTAGCGATCAGCGTC-3'
Trp-4: 5'-CGTGAACTTGTGTACTAGTTAACTGGTTCGCGAAGGCAATGTCAACCCT
          CTTTTTGCACAATGTT-3'
Trp-6: 5'-CGATGAATTCGAGCTCATATTAAACCTCCTTACCGTCTCGAGC-3'
```

Je 100 pMol Oligonukleotide Trp-2 bis Trp-5 wurden separat in 10 µl phosphoryliert. Trp-1 und -2, Trp-3 und -4 sowie Trp-5 und -6 wurden durch Aufkochen und langsames Abkühlen hybridisiert. Die Oligonukleotidpaarlösungen wurden vereint und durch Zusatz von T4-DNA Ligase ligiert. 3 µg pAT153 wurde mit EcoRI und ClaI doppelt geschnitten. Nach Reinigung des großen Fragmentes wurde dieses mit ca. 20 pMol Oligonukleotiden versetzt und ligiert. Die DNA wurde anschließend in E.coli HB101 transformiert und die Plasmide einiger resultierender Kolonien isoliert. Das den Promoter enthaltende Pst-HindIII Fragment wurde sequenziert. Nach Bestätigung der gewünschten Sequenz wurde ein Plasmid ausgewählt und mit pRH281/5 bezeichnet.

Die Sequenz des Promotorteiles lautet:

```
                                                   -35
5'-GAATTGACGCTGATCGCTAAAACATTGTGCAAAAAGAGGGTTGACATTGC
3'-CTTAACTGCGACTAGCGATTTTGTAACACGTTTTTCTCCCAACTGTAACG


                                          XhoI
                   -10          !Transkriptionsstart
CTTCGCGAACCAGTTAACTAGTACACAAGTTCACGGCTCGAGACGGTAAG
GAAGCGCTTGGTCAATTGATCATGTGTTCAAGTGCCGAGCTCTGCCATTC

RBS             SstI EcoRI ClaI
GAGGTTTAATATGAGCTCGAATTCATCGAT-3'
CTCCAAATTATACTCGAGCTTAAGTAGCTA-5'
```

Die Vorteile des neuen Expressionsvektors sind:

1) optimale -35 Region im trp-Sma Promotor

2) singuläre XhoI-Stelle vor der ribosomalen Bindungsstelle (RBS) erlaubt den Austausch der RBS gegen eine andere

3) Das Expressionsplasmid enthält einen Translationsstart ATG im Abstand von 5 Nukleotiden nach der RBS

4) Das G dieses ATG's ist die erste Base der SstI-Erkennungssequenz (GAGCTC). Durch Schnitt mit SstI und anschließender Erzeugung eines geraden Endes steht ein Expressionsvektor mit einem Translationsstart-ATG zur Verfügung, an das ein fremdes Gen beginnend mit der ersten Base des Leserahmens anligiert werden kann.

5) Die Verbindung RBS-ATG enthält kein G oder C

6) Durch die Wahl der Oligonukleotidesquenz am 5'Ende wurde die ursprüngliche EcoRI Schnittstelle zerstört. Dadurch kann am 3'Ende des Promotors eine Multiklonierstelle bestehend aus SstI, EcoRI, ClaI und HindIII (bereits im pAT153 Anteil) erzeugt werden.

b) pRH282/5

Auf gleiche Weise wurde der Expressionsvektor pRH282/5 zusammengebaut. Die Oligonukleotide Trp 1 und Trp2 wurden durch die Oligonukleotide Trp-7 und Trp-8 ersetzt:

```
Trp-7: 5'-AATTGCCCGTTCTGGATAATGTTTTTTGCGCCGACATCATCATGCT-3'
Trp-8: 5'-TTAGCGATCAGCATGATGATGTCGGCGCAAAAAACATTATCCAGAACGGGC-3'
```

Die Sequenz des Promotorteils in pRH282/5 lautet:

```
5'-GAATTGCCCGTTCTGGATAATGTTTTTTGCGCCGACATCATCATGCTGAT
3'-CTTAACGGGCAAGACCTATTACAAAAAACGCGGCTGTAGTAGTACGACTA

                                       -35
   CGCTAAAACATTGTGCAAAAAGAGGGTTGACATTGCCTTCGCGAACCAGT
   GCGATTTTGTAACACGTTTTTCTCCCAACTGTAACGGAAGCGCTTGGTCA

                           XhoI
   -10           !Transkriptionsstart     RBS
   TAACTAGTACACAAGTTCACGGCTCGAGACGGTAAGGAGGTTTAATATGA
   ATTGATCATGTGTTCAAGTGCCGAGCTCTGCCATTCCTCCAAATTATACT

   SstI EcoRI ClaI
   GCTCGAATTCATCGAT-3'
   CGAGCTTAAGTAGCTA-5'
```

c)pGN1

1 µg pUC18 wurde mit BamHI und SalI doppelt geschnitten. Aus 10 µg EqG-QAA1 wurde ebenfalls durch BamHI-SalI Doppelschnitt die zweite Hälfte des synthetischen Pferde-Gamma-Interferon Gens isoliert und dephosphoryliert. Ca. 20 ng Vektor wurden mit 100 ng Insert ligiert und die DNA in E.coli JM101 transformiert. Das Plasmid einer Kolonie wurde mittels Restriktionsenzymverdau geprüft und mit pGN1 bezeuchnet.

d)pGN3

Die erste Hälfte des synthetischen Gens wurde zusammen mit der ribosomalen Bindungsstelle aus Oligonukleotiden zusammengesetzt:

```
EqG-1: 5'-AGCTTCCCTCGAGAGGTTGAGGTGATTTATGCAGGCTGCTTTCTTTAAAG
          AAATCGAAAACCTGAAAGAATACTTCAACGCTCGTAACCCAGACGTTGGT-3'
EqG-2: 5'-GACGGTGGTCCGCTGTTCCTGGACATCCTGAAAAACTGGAAAGAAGACTC
          TGACAAAAAGATCATCCAGTCTCAG-3'
EqG-3: 5'-ATCGTTTCTTTCTACTTCAAACTGTTCGAAAACCTGAAAGACAACCAGGT
          TATCCAGAAATCGATGGACACTATCAAAGAAGATCTGTTCGTTAAATTCT
          TCAACTCGTCGACTCCG-3'
EqG-4: 5'-AATTCGGAGTCGACGAGTTGAAGAATTTAACGAACAGATCTTCTTTGATA
          GTGTCCATCGATTTCTGGATAACCTGGTTGTCTTTCAGGTTTTCGAACAG
          TTTGAAGTAGAAAGAAACGATCTGAGACTG-3'
EqG-5: 5'-GATGATCTTTTTGTCAGAGTCTTCTTTCCAGTTTTTCAGGATGTCCAGGA
          ACAGCGGACCACCGTCACCAACGTC-3'
EqG-6: 5'-TGGGTTACGAGCGTTGAAGTATTCTTTCAGGTTTTCGATTTCTTTAAAGA
          AAGCAGCCTGCATAAATCACCTCAACCTCTCGAGGGA-3'
```

Jeweils 50 pMol der Oligonukleotide wurden phosphoryliert, und zwar: EqG-2 zusammen mit EqG-5 in 7 µl, EqG-3 und EqG-8 allein in je 8 µl. Die Kinasereaktion wurde durch Erhitzen auf 100 °C gestoppt. Zu EqG-3 wurden 50 pMol EqG-4 (1µl) und zu EqG-8 50 pMol EqG-1 (1 µl) zugesetzt. Die Lösungen wurden nochmals auf 100 °C erhitzt und langsam abgekühlt. Die Oligonukleotidpaarlösungen wurden vereint und mit T4-DNA Ligase in insgesamt 30 µl ligiert, 2 µg pUC18 wurden mit EcoRI und HindIII doppelt geschnitten, der Vektorteil gelgereinigt und in 50 µl Wasser gelöst. 40 ng Vektor und ca. 2 pMol ligierte Oligonukleotide wurden in 10 µl ligiert und die DNA anschließend in E. coli JM101 transformiert. Das EcoRI-HindIII Insert einiger resultierender Plasmide wurde in M13mp9 umkloniert und die Sequenz überprüft. Ein Plasmid mit der erwarteten Sequenz wurde ausgewählt und mit pGN3 bezeichnet.

e) pGN20

Ca 3 µg pGN1 bzw. pGN3 wurden mit HindIII und SalI doppelt geschnitten. Das Insert des pGN3 und der Vektor/2. Hälfte des Eq-gamma-Interferon Gens aus pGN1 wurden gelgereinigt. 0,2 µg pGN3 wurden mit ca. 0,05 µg pGN3-Insert ligiert und die DNA in E.coli JM101 transformiert. Das Plasmid eines resultierenden Klons wurde nach Überprüfung des Restriktionsmusters ausgewählt und mit pGN20 bezeichnet.

f) pEqG-QAA2 bzw. pEqG-QAA3

Aus ca. 10 µg pGN20 wurde das XhoI-EcoRI Insert, das das synthetischen Pferde-gamma-Interferon Gen zusammen mit der ribosomalen Bindungsstelle des E.coli Enterotoxin II (C.H. Lee et al., Infect. Immun. 42 (1983), 264-268; S.L. Moseley et al., Infect. Immun. 39 (1983), 1167-1174)) enthält, isoliert. pRH281/5 bzw. pRH282/5 wurden mit XhoI und EcoRI doppelt geschnitten. 20 ng Vektor wurden mit 20 ng Insert ligiert, und die 20 ng Vektor wurden mit 20 ng Insert ligiert, und die DNA in E. coli HB101 transformiert. Einige Kolonien wurden ausgewählt, die Plasmide isoliert und durch Restriktionsanalyse überprüft. Jeweils ein Plasmid wurde ausgewählt und mit pEqG-QAA2 (Vektor: pRH281/5) bzw. pEqG-QAA3 (Vektor: pRH282/5) bezeichnet.

g) Lysattest auf Interferon-gamma Aktivität

Eine über Nacht Kultur von E.coli HB101/pEqG-QAA2 bzw. HB101/pEqG-QAA3 wurde 1:100 mit LB/Amp verdünnt (LB: 10g/l Trypton, 5g/l Hefeextrakt, 5g/l NaCl, 50 mg/l Ampicillin) und weiter bei 37 °C inkubiert. Bei Erreichen einer optischen Dichte (600nm) von 0,3 wurde 50 mg/l Indolacrylsäure zugesetzt, und die Kultur weitere zwei Stunden bei 37 °C inkubiert. Die Bakterien wurden abzentrifugiert und mittels Ultraschall aufgebrochen. Der steril filtriert Überstand wurde auf NBL-6 Zellen (ATCC CCL 57) auf gamma-Interferonaktivität getestet, wobei Vesicular Stomatitis Virus (VSV) als Virus eingesetzt wurde. Die Lysate beider transformierter Bakterienkluturen (E. coli HB101/pEqG-QAA2 bzw. HB101/pEqG-QAA3) zeigten etwa 0,1 bis 1 Million Einheiten/ml Interferonaktivität. Zur Kontrolle wurde ein identisch hergestelltes E.coli HB101/pRH281 Lysat getestet. Dieses Kontrollysat zeigte weniger als 100 Einheiten/ml.

**Patentansprüche**

**1.** DNA-Molekül, das eine Auswahl enthält aus den Nukleotidsequenzen

a)

```
R1 TACTGCCAGG CTGCTTTCTT TAAAGAAATC GAAAACCTGA AAGAATACTT  50
   CAACGCTCGT AACCCAGACG TTGGTGACGG TGGTCCGCTG TTCCTGGACA 100
   TCCTGAAAAA CTGGAAAGAA GACTCTGACA AAAAGATCAT CCAGTCTCAG 150
   ATCGTTTCTT TCTACTTCAA ACTGTTCGAA AACCTGAAAG ACAACCAGGT 200
   TATCCAGAAA TCGATGGACA CTATCAAAGA AGATCTGTTC GTTAAATTCT 250
   TCAACTCGTC GACTTCTAAA CTGGAAGACT TCCAGAAACT GATCCAGATC 300
   CCAGTTAACG ACCTGAAAGT TCAGCGTAAG GCTATCTCTG AACTGATCAA 350
   AGTTATGAAC GACCTGTCTC CAAAAGCTAA CCTGCGTAAA CGTAAACGTT 400
   CTCAGAACCC ATTCCGTGGT CGTCGTGCTC TTCAGTAA,
```

wobei $R^1$

```
ATGAATTATA CAAGTTTTAT CTTGGCTTTT CAGCTGTGTG CGATTTTGGG 50
TTCTTCTACC TAT,
ATGTAT,
```

oder
TAT
bedeutet,
b)

```
R2 GCTGCTTTCT TTAAAGAAAT CGAAAACCTG AAAGAATACT TCAACGCTCG  50
   TAACCCAGAC GTTGGTGACG GTGGTCCGCT GTTCCTGGAC ATCCTGAAAA 100
   ACTGGAAAGA AGACTCTGAC AAAAAGATCA TCCAGTCTCA GATCGTTTCT 150
   TTCTACTTCA AACTGTTCGA AAACCTGAAA GACAACCAGG TTATCCAGAA 200
   ATCGATGGAC ACTATCAAAG AAGATCTGTT CGTTAAATTC TTCAACTCGT 250
   CGACTTCTAA ACTGGAAGAC TTCCAGAAAC TGATCCAGAT CCCAGTTAAC 300
   GACCTGAAAG TTCAGCGTAA GGCTATCTCT GAACTGATCA AAGTTATGAA 350
   CGACCTGTCT CCAAAAGCTA ACCTGCGTAA ACGTAAACGT TCTCAGAACC 400
   CATTCCGTGG TCGTCGTGCT CTTCAGTAA,
```

wobei $R^2$

```
ATGAATTATA CAAGTTTTAT CTTGGCTTTT CAGCTGTGTG CGATTTTGGG 50
TCTTCTACCC AG,
ATGCAG,
```

oder
CAG
bedeutet,
c) einer Sequenz, die mit dem Gegenstrang einer der vorgenannten Sequenzen unter Bedingungen, die für mehr als 85% Homologie selektieren, hybridisiert, und die für ein Polypeptid mit der biologischen Aktivität von Pferde-Interferon-gamma kodiert.

**2.** DNA-Molekül nach Anspruch 1, dadurch gekennzeichnet, daß es funktionell mit einer Expressionskontrollsequenz verknüpft ist.

**3.** DNA-Molekül nach Anspruch 2, dadurch gekennzeichnet, dal die Expressionskontrollsequenz ein modifizierter *trp*-Promotor aus *Serratia marcescens* ist.

**4.** DNA-Molekül nach Anspruch 3, dadurch gekennzeichnet, daß die Expressionskontrollsequenz eine Auswahl enthält aus den Nukleotidsequenzen

a)
```
GAATTGACGC TGATCGCTAA AACATTGTGC AAAAAGAGGG TTGACATTGC  50
CTTCGCGAAC CAGTTAACTA GTACACAAGT TCACGGCTCG AGACGGTAAG 100
GAGGTTTAAT ATGAGCTCGA ATTCATCGAT,
```

b)
```
GAATTGCCCG TTCTGGATAA TGTTTTTTGC GCCGACATCA TCATGCTGAT  50
CGCTAAAACA TTGTGCAAAA AGAGGGTTGA CATTGCCTTC GCGAACCAGT 100
TAACTAGTAC ACAAGTTCAC GGCTCGAGAC GGTAAGGAGG TTTAATATGA 150
GCTCGAATTC ATCGAT.
```

**5.** Polypeptid, dadurch gekennzeichnet, daß es durch eines der DNA-Moleküle nach Anspruch 1 kodiert wird und die biologische Aktivität von Pferde-Interferon-gamma hat.

6. DNA-Molekül, dadurch gekennzeichnet, daß es eine Auswahl aus den Sequenzen

TACTACTGCC AGGCTGCTTT CTTTAAAGAA ATCGAAAACC TGAAAGAATA CTTCAAGCTC CG,

TTGAAGTATT CTTTCAGGTT TTCGATTTCT TTAAAGAAAG CAGCCTGGCA GTAGTA,

TAACCCAGAC GTTGGTGACG GTGGTCCGCT GTTCCTGGAC ATCCTGAAAA ACTGGAAAGA ACACTCTG,

TTCTTTCCAG TTTTTCAGGA TGTCCAGGAA CAGCGGACCA CCGTCACCAA CGTCGGGGTT ACGAGCG,

ACAAAAAGAT CATCCAGTCT CAGATCGTTT CTTTCTACTT CAAACTGTTC GAAAACCTGA AAGACAACC,

TTTCAGGTTT TCGAACAGTT TGAAGTAGAA AGAAACGATC TGAGACTGGA TGATCTTTTT GTCAGAGTC,

AGGTTATCCA GAAATCGATC GACACTATCA AAGAAGATCT GTTCGTTAAA TTCTTCAACT CG,

TCGACGAGTT GAAGAATTTA ACGAACAGAT CTTCTTTGAT AGTGTCCATC GATTTCTGGA TAACCTGGTT GTC,

TCGACTTCTA AACTGGAAGA CTTCCAGAAA CTGATCCAGA TCCCAGTTAA CGACCTGAAA,

GCTGAACTTT CAGGTCGTTA ACTGGGATCT GGATCAGTTT CTGGAAGTCT TCCAGTTTAG AAG,

GTTCAGCGTA AGGCTATCTC TGAACTGATC AAAGTTATGA ACGACCTGTC TCCAAAAGCT AA,

CGCAGGTTAG CTTTTGGAGA CAGGTCGTTC ATAACTTTGA TCAGTTCAGA GATAGCCTTA C,

CCTGCGTAAA CGTAAACGTT CTCAGAACCC ATTCCGTGGT CGTCGTGCTC TTCAGTAAG,

GATCCTTACT GAAGAGCACG ACGACCACGG AATGGGTTCT GAGAACGTTT ACGTTTA,

CAGGCTGCTT TCTTTAAAGA AATCGAAAAC CTGAAAGAAT ACTTCAACGC TCG,

TTGAAGTATT CTTTCAGGTT TTCGATTTCT TTAAAGAAAG CAGCCTG

enthält, daß mindestens zwei dieser DNA-Moleküle in beliebiger Kombination miteinander verknüpft sind, oder daß ein oder mehrere Tripletts ersetzt sind, die für dieselbe Aminosäure kodieren.

7. Wirtsorganismus, dadurch gekennzeichnet, daß er mit einem DNA-Molekül nach einem der Ansprüche 1 bis 4 transformiert ist.

8. Verfahren zur Herstellung eines Polypeptids nach Anspruch 5, dadurch gekennzeichnet, daß
   a) ein Wirtsorganismus mit einem der DNA-Moleküle nach den Ansprüche 1 bis 4 transformiert,
   b) der transformierte Wirtsorganismus kultiviert und
   c) das Polypeptid isoliert wird.

9. Pharmazeutische Zusammensetzung, dadurch gekennzeichnet, daß sie neben üblichen Hilfs- und/oder Trägerstoffen eine wirksame Menge eines Polypeptids nach Anspruch 5 enthält.

**Claims**

1. DNA molecule which contains a selection from the nucleotide sequences
   a)

```
R1 TACTGCCAGG CTGCTTTCTT TAAAGAAATC GAAAACCTGA AAGAATACTT  50
   CAACGCTCGT AACCCAGACG TTGGTGACGG TGGTCCGCTG TTCCTGGACA 100
   TCCTGAAAAA CTGGAAAGAA GACTCTGACA AAAAGATCAT CCAGTCTCAG 150
   ATCGTTTCTT TCTACTTCAA ACTGTTCGAA AACCTGAAAG ACAACCAGGT 200
   TATCCAGAAA TCGATGGACA CTATCAAAGA AGATCTGTTC GTTAAATTCT 250
   TCAACTCGTC GACTTCTAAA CTGGAAGACT TCCAGAAACT GATCCAGATC 300
   CCAGTTAACG ACCTGAAAGT TCAGCGTAAG GCTATCTCTG AACTGATCAA 350
   AGTTATGAAC GACCTGTCTC CAAAAGCTAA CCTGCGTAAA CGTAAACGTT 400
   CTCAGAACCC ATTCCGTGGT CGTCGTGCTC TTCAGTAA,
```

wherein $R^1$ denotes

```
ATGAATTATA CAAGTTTTAT CTTGGCTTTT CAGCTGTGTG CGATTTTGGG 50
TTCTTCTACC TAT,
ATGTAT,
```

or
TAT,
b)

```
R2 GCTGCTTTCT TTAAAGAAAT CGAAAACCTG AAAGAATACT TCAACGCTCG  50
   TAACCCAGAC GTTGGTGACG GTGGTCCGCT GTTCCTGGAC ATCCTGAAAA 100
   ACTGGAAAGA AGACTCTGAC AAAAAGATCA TCCAGTCTCA GATCGTTTCT 150
   TTCTACTTCA AACTGTTCGA AAACCTGAAA GACAACCAGG TTATCCAGAA 200
   ATCGATGGAC ACTATCAAAG AAGATCTGTT CGTTAAATTC TTCAACTCGT 250
   CGACTTCTAA ACTGGAAGAC TTCCAGAAAC TGATCCAGAT CCCAGTTAAC 300
   GACCTGAAAG TTCAGCGTAA GGCTATCTCT GAACTGATCA AAGTTATGAA 350
   CGACCTGTCT CCAAAAGCTA ACCTGCGTAA ACGTAAACGT TCTCAGAACC 400
   CATTCCGTGG TCGTCGTGCT CTTCAGTAA,
```

wherein $R^2$ denotes

```
ATGAATTATA CAAGTTTTAT CTTGGCTTTT CAGCTGTGTG CGATTTTGGG 50
TCTTCTACCC AG,
ATGCAG,
```

or
CAG,
c) a sequence which hybridise with the counter-strand of one of the above-mentioned sequences under conditions which select for more than 85% homology, and which codes for a polypeptide with the biological activity of horse interferon-gamma.

2. DNA molecule according to claim 1, characterised in that it is functionally linked to an expression control sequence.

3. DNA molecule according to claim 2, characterised in that the expression control sequence is a modified trp-promotor from Serratia marcescens.

4. DNA molecule according to claim 3, characterised in that the expression control sequence contains a selection from the nucleotide sequences

a)

```
GAATTGACGC TGATCGCTAA AACATTGTGC AAAAAGAGGG TTGACATTGC  50
CTTCGCGAAC CAGTTAACTA GTACACAAGT TCACGGCTCG AGACGGTAAG 100
GAGGTTTAAT ATGAGCTCGA ATTCATCGAT,
```

b)

```
GAATTGCCCG TTCTGGATAA TGTTTTTTGC GCCGACATCA TCATGCTGAT  50
CGCTAAAACA TTGTGCAAAA AGAGGGTTGA CATTGCCTTC GCGAACCAGT 100
TAACTAGTAC ACAAGTTCAC GGCTCGAGAC GGTAAGGAGG TTTAATATGA 150
GCTCGAATTC ATCGAT.
```

5. Polypeptide, characterised in that it is coded by one of the DNA molecules according to claim 1 and has the biological activity of horse interferon-gamma.

**6.** DNA molecule, characterised in that it contains a selection from the sequences

TACTACTGCC AGGCTGCTTT CTTTAAAGAA ATCGAAAACC TGAAAGAATA CTTCAAGCTC CG,

TTGAAGTATT CTTTCAGGTT TTCGATTTCT TTAAAGAAAG CAGCCTGGCA GTAGTA,

TAACCCAGAC GTTGGTGACG GTGGTCCGCT GTTCCTGGAC ATCCTGAAAA ACTGGAAAGA ACACTCTG,

TTCTTTCCAG TTTTTCAGGA TGTCCAGGAA CAGCGGACCA CCGTCACCAA CGTCGGGGTT ACGAGCG,

ACAAAAAGAT CATCCAGTCT CAGATCGTTT CTTTCTACTT CAAACTGTTC GAAAACCTGA AAGACAACC,

TTTCAGGTTT TCGAACAGTT TGAAGTAGAA AGAAACGATC TGAGACTGGA TGATCTTTTT GTCAGAGTC,

AGGTTATCCA GAAATCGATC GACACTATCA AAGAAGATCT GTTCGTTAAA TTCTTCAACT CG,

TCGACGAGTT GAAGAATTTA ACGAACAGAT CTTCTTTGAT AGTGTCCATC GATTTCTGGA TAACCTGGTT GTC,

TCGACTTCTA AACTGGAAGA CTTCCAGAAA CTGATCCAGA TCCCAGTTAA CGACCTGAAA,

GCTGAACTTT CAGGTCGTTA ACTGGGATCT GGATCAGTTT CTGGAAGTCT TCCAGTTTAG AAG,

GTTCAGCGTA AGGCTATCTC TGAACTGATC AAAGTTATGA ACGACCTGTC TCCAAAAGCT AA,

CGCAGGTTAG CTTTTGGAGA CAGGTCGTTC ATAACTTTGA TCAGTTCAGA GATAGCCTTA C,

CCTGCGTAAA CGTAAACGTT CTCAGAACCC ATTCCGTGGT CGTCGTGCTC TTCAGTAAG,

GATCCTTACT GAAGAGCACG ACGACCACGG AATGGGTTCT GAGAACGTTT ACGTTTA,

CAGGCTGCTT TCTTTAAAGA AATCGAAAAC CTGAAAGAAT ACTTCAACGC TCG,

TTGAAGTATT CTTTCAGGTT TTCGATTTCT TTAAAGAAAG CAGCCTG

in that at least two of these DNA molecules are linked to one another in any desired combination, or in that one or more triplets which code for this amino acid are replaced.

**7.** Host organism, characterised in that it is transformed with a DNA molecule according to one of claims 1 to 4.

**8.** Process for preparing a polypeptide according to claim 5, characterised in that
a) a host organism is transformed with one of the DNA molecules according to claims 1 to 4,
b) the transformed host organism is cultivated and
c) the polypeptide is isolated.

**9.** Pharmaceutical composition, characterised in that it contains, in addition to conventional excipients and/or carriers, an effective amount of a polypeptide according to claim 5.

**Revendications**

**1.** Molécule d'ADN qui contient un choix parmi les séquences nucléotidiques
a)

```
R1 TACTGCCAGG CTGCTTTCTT TAAAGAAATC GAAAACCTGA AAGAATACTT  50
   CAACGCTCGT AACCCAGACG TTGGTGACGG TGGTCCGCTG TTCCTGGACA 100
   TCCTGAAAAA CTGGAAAGAA GACTCTGACA AAAAGATCAT CCAGTCTCAG 150
   ATCGTTTCTT TCTACTTCAA ACTGTTCGAA AACCTGAAAG ACAACCAGGT 200
   TATCCAGAAA TCGATGGACA CTATCAAAGA AGATCTGTTC GTTAAATTCT 250
   TCAACTCGTC GACTTCTAAA CTGGAAGACT TCCAGAAACT GATCCAGATC 300
   CCAGTTAACG ACCTGAAAGT TCAGCGTAAG GCTATCTCTG AACTGATCAA 350
   AGTTATGAAC GACCTGTCTC CAAAAGCTAA CCTGCGTAAA CGTAAACGTT 400
   CTCAGAACCC ATTCCGTGGT CGTCGTGCTC TTCAGTAA,
```

où $R^1$ représente

```
ATGAATTATA CAAGTTTTAT CTTGGCTTTT CAGCTGTGTG CGATTTTGGG 50
TTCTTCTACC TAT,
ATGTAT,
```

ou
TAT
b)

```
R2 GCTGCTTTCT TTAAAGAAAT CGAAAACCTG AAAGAATACT TCAACGCTCG  50
   TAACCCAGAC GTTGGTGACG GTGGTCCGCT GTTCCTGGAC ATCCTGAAAA 100
   ACTGGAAAGA AGACTCTGAC AAAAAGATCA TCCAGTCTCA GATCGTTTCT 150
   TTCTACTTCA AACTGTTCGA AAACCTGAAA GACAACCAGG TTATCCAGAA 200
   ATCGATGGAC ACTATCAAAG AAGATCTGTT CGTTAAATTC TTCAACTCGT 250
   CGACTTCTAA ACTGGAAGAC TTCCAGAAAC TGATCCAGAT CCCAGTTAAC 300
   GACCTGAAAG TTCAGCGTAA GGCTATCTCT GAACTGATCA AAGTTATGAA 350
   CGACCTGTCT CCAAAAGCTA ACCTGCGTAA ACGTAAACGT TCTCAGAACC 400
   CATTCCGTGG TCGTCGTGCT CTTCAGTAA,
```

où $R^2$ représente

```
ATGAATTATA CAAGTTTTAT CTTGGCTTTT CAGCTGTGTG CGATTTTGGG 50
TCTTCTACCC AG,
ATGCAG,
```

ou
CAC
c) une séquence qui s'hybride avec le brin opposé de l'une des séquence précitées dans des conditions qui opèrent une sélection pour plus de 85% d'homologie, et qui code pour un polypeptide présentant l'activité biologique de l'interféron gamma de cheval.

2. Molécule d'ADN selon la revendication 1, caractérisée en ce qu'elle est reliée de manière fonctionnelle à une séquence de régulation de l'expression.

3. Molécule d'ADN selon la revendication 2, caractérisée en ce que la séquence de régulation de l'expression est un promoteur trp modifié provenant de Serratia marcescens.

4. Molécule d'ADN selon la revendication 3, caractérisée en ce que la séquence de régulation de l'expression contient un choix parmi les séquences nucléotidiques

a)
```
GAATTGACGC TGATCGCTAA AACATTGTGC AAAAAGAGGG TTGACATTGC  50
CTTCGCGAAC CAGTTAACTA GTACACAAGT TCACGGCTCG AGACGGTAAG 100
GAGGTTTAAT ATGAGCTCGA ATTCATCGAT,
```

b)
```
GAATTGCCCG TTCTGGATAA TGTTTTTTGC GCCGACATCA TCATGCTGAT  50
CGCTAAAACA TTGTGCAAAA AGAGGGTTGA CATTGCCTTC GCGAACCAGT 100
TAACTAGTAC ACAAGTTCAC GGCTCGAGAC GGTAAGGAGG TTTAATATGA 150
GCTCGAATTC ATCGAT.
```

5. Polypeptide caractérisé en ce qu'il est codé par l'une des molécules d'ADN selon la revendication 1 et présente l'activité biologique de l'interféron gamma de cheval.

6. Molécule d'ADN caractérisée en ce qu'elle contient un choix parmi les séquences

```
TACTACTGCC AGGCTGCTTT CTTTAAAGAA ATCGAAAACC TGAAAGAATA
CTTCAAGCTC CG,
TTGAAGTATT CTTTCAGGTT TTCGATTTCT TTAAAGAAAG CAGCCTGGCA
GTAGTA,
TAACCCAGAC GTTGGTGACG GTGGTCCGCT GTTCCTGGAC ATCCTGAAAA
ACTGGAAAGA ACACTCTG,
TTCTTTCCAG TTTTTCAGGA TGTCCAGGAA CAGCGGACCA CCGTCACCAA
CGTCGGGGTT ACGAGCG,
```

ACAAAAAGAT CATCCAGTCT CAGATCGTTT CTTTCTACTT CAAACTGTTC
GAAAACCTGA AAGACAACC,

TTTCAGGTTT TCGAACAGTT TGAAGTAGAA AGAAACGATC TGAGACTGGA
TGATCTTTTT GTCAGAGTC,

AGGTTATCCA GAAATCGATC GACACTATCA AAGAAGATCT GTTCGTTAAA
TTCTTCAACT CG,

TCGACGAGTT GAAGAATTTA ACGAACAGAT CTTCTTTGAT AGTGTCCATC
GATTTCTGGA TAACCTGGTT GTC,

TCGACTTCTA AACTGGAAGA CTTCCAGAAA CTGATCCAGA TCCCAGTTAA
CGACCTGAAA,

GCTGAACTTT CAGGTCGTTA ACTGGGATCT GGATCAGTTT CTGGAAGTCT
TCCAGTTTAG AAG,

GTTCAGCGTA AGGCTATCTC TGAACTGATC AAAGTTATGA ACGACCTGTC
TCCAAAAGCT AA,

CGCAGGTTAG CTTTTGGAGA CAGGTCGTTC ATAACTTTGA TCAGTTCAGA
GATAGCCTTA C,

CCTGCGTAAA CGTAAACGTT CTCAGAACCC ATTCCGTGGT CGTCGTTGCTC
TTCAGTAAG,

GATCCTTACT GAAGAGCACG ACGACCACGG AATGGGTTCT GAGAACGTTT
ACGTTTA,

CAGGCTGCTT TCTTTAAAGA AATCGAAAAC CTGAAAGAAT ACTTCAACGC
TCG,

TTGAAGTATT CTTTCAGGTT TTCGATTTCT TTAAAGAAAG CAGCCTG,

en ce qu'au moins deux de ces molécules d'ADN sont reliées entre elles en une combinaison quelconque, ou en ce qu'un ou plusieurs triplets qui codent pour le même acide aminé sont remplacés.

**7.** Organisme hôte caractérisé en ce qu'il est transformé avec une molécule d'ADN selon l'une des revendications 1 à 4.

**8.** Procédé de préparation d'un polypeptide selon la revendication 5, caractérisé en ce que
a) un organisme hôte est transformé avec l'une des molécules d'ADN selon les revendications 1 à 4,
b) l'organisme hôte transformé est cultivé et
c) le polypeptide est isolé.

**9.** Composition pharmaceutique caractérisée en ce qu'elle contient, outre des adjuvants et/ou véhicules courants, une quantité efficace d'un polypeptide selon la revendication 5.

```
EqIFN-gamma   (Lambda Eq- 2  BamHI-fragment)

                                                       GGATC     5
CCACAAGAATGGCACGGGTGGGCATAATGGGTCTGTCTCATCGTCAAAAGACCCAAGGAG    65
TTGAAAGGAAACTCTAACTACAACACCAAAATGCCACAAAACCATAGTTATTAATACAAA   125
CTAACTAGCATCTGTGCCTATCTGTCACCATCTCATCTGAAAAAACTTGTGAAAATACGT   185
AATCCTGATGAGACTTCAATTAGGTATAAAAACCAGCCCCAGAAGGCGAGGCAGTACACT   245
CTTCTGATCGCCGGTAGGGCAGCTATTAGAAAAGAAAGATCAGCTGAGGCCTTGGGACCT   305
GATCAGCTTAGTACAGAAGTGACTGCTTTCAACTACTTAGGCCTAACTCTCTCCGAAACA   365

-20                 -15                 -10
Met Asn Tyr Thr Ser Phe Ile Leu Ala Phe Gln Leu Cys Ala Ile
ATG AAT TAT ACA AGT TTT ATC TTG GCT TTT CAG CTG TGT GCG ATT        410

 -5                  -1   1              5                  10
Leu Gly Ser Ser Thr Tyr Tyr Cys Gln Ala Ala Phe Phe Lys Glu
TTG GGT TCT TCT ACC TAT TAC TGC CAG GCC GCG TTT TTT AAA GAA        455
                 15
Ile Glu Asn Leu Lys Glu Tyr Phe
ATA GAA AAC CTA AAG GAA TAT TTT GTAAGTATGACCTTTTAATAATACTTAC     507

        I N T R O N  1

TTGTGGTTGAAATGACTGAACGTTGTCTTGGAGTTGGATCTCTGATAGGCTGTCCTCTCT   567
ACTCCACAGTCATCTTGAGAAGACTGGGTGTTATTTTCTCTGTTTGTTGACTGGATGAGT   627
TTTTCTTTTTTTTACTAAATGATCTAGATATTGCTTTAACCCTCTGCTCAATTTGCTATA   687
GAGACTTAGAGAGGGTTCATGAATCTTCCAAAAGATGGGCTTAACAGGTTTATAAAGCAT   747
AGTGAAGTTGACAATTTTGTGGTGAGAAGCCACTGAATTGTGATAAGTCAAGTAGTGTGG   807
ACATTGAAAAAATGACTAGCTATTAGTTTCTAACTTCTCAGGTTACTATGATGGTGACAA   867
TAAAAGGTCAAGATTAGCATTAAAATGGTAATCTGAAATAATTGATCAGTTAAAGAAGGC   927
GCTGTCCTGAAAGGTTTGGCTGAAAAAAAATCACTTTCAGGTGTTTTCCTCCAAAAAATG   987
ATTTTAAAATCTTACTGCCCCGTTTGTGTTAGCTGTGAAGTACTCTGGAACTCAGTCAAT  1047
TGCTGAGATTTTGTACGAGTTATAAGCTGGCTTATATTTAAAAAATTTTTTTGTTTTTGT  1107
TTTATGAGTTTCTTTTAAAATGTTATTTATGGTTAATTAAAATAGTTTTTGCATTTTAAA  1167
TATTTTATTATTTGTCCAAAATTTAGCTATTTTAATTATAGTTGGAGCTCTCTTTTAGAG  1227
CTGACATAAGACCATAGGGGAGGCACAGATAGATGTGATGGAGCCCTGTACCAGACGGGG  1287
GCAGTATCTTATAGTGGGTTGCCTTTGCTGATCTTTTTACTAGACTTGAAATTATTTGCT  1347
TTTCCTTCCTATGGTTATTTGGGACTATTGAAGTATCACCAGCCCTGTTGAGTTCATCTG  1407
TAATATTGTAATTCAAGGGTTACACTAGAAAATAAGAAAGCTAAAACAGCACGATAATCT  1467
TTGGCTACATCCAACACAATAGCTTTTGGGAATACTTATTGTTAGAACTAAACAGAGGGT  1527
TGAAAAGAAAATCAGTGAATACTGTCAGCATCTGAGTTCAATAAAACGTGAAGTACATTT  1587

                                                          20
                                                     Asn Ala
TTAGGGCAATTCATGGACTAATTGTAAACCAAGTTTTCCTTCCTTTTTCAG AAC GCA   1644

                25                  30                  35
Arg Asn Pro Asp Val Gly Asp Gly Gly Pro Leu Phe Leu Asp Ile
AGA AAC CCA GAT GTA GGG GAT GGT GGG CCT CTT TTC CTG GAT ATC        1689
```

Fig. 1a

40 I N T R O N 1
Leu Lys Asn Trp Lys Glu
TTG AAG AAC TGG AAA GAG GTAAGCTAAGTATTTCCATTTGGTTGATTTTCCTGT 1743
TGCTTATTTTCTGGTGGATGAATTCACACCAACCTCTCTTTGTGCTCTTTTCTCCCTAG 1802

45 50 55
Asp Ser Asp Lys Lys Ile Ile Gln Ser Gln Ile Val Ser Phe Tyr
GAT AGT GAC AAA AAA ATA ATT CAG AGC CAA ATC GTC TCC TTC TAC 1847

60 65 70
Phe Lys Leu Phe Glu Asn Leu Lys Asp Asn Gln Val Ile Gln Lys
TTC AAA CTC TTT GAA AAC TTG AAA GAT AAC CAG GTC ATT CAA AAG 1892

75 80 85
Ser Met Asp Thr Ile Lys Glu Asp Leu Phe Val Lys Phe Phe Asn
AGC ATG GAC ACC ATC AAG GAG GAC CTG TTC GTT AAG TTC TTT AAC 1937

90 95 100
Ser Ser Thr Ser Lys Leu Glu Asp Phe Gln Lys Leu Ile Gln Ile
AGC AGC ACC AGC AAG CTG GAA GAC TTC CAA AAG CTG ATT CAG ATT 1982

I N T R O N
Pro
CCG GTGAGGAGATCTTAATTCTTTCTTTGGTTTCATTACAGAGGTTCTTGCAAAGTGCT 2041
TACGTCCCAGAAAGTAGAAATGAACTATGAAATGAACCCGTGGCCAAAACTCCTCCTTCC 2101
TAATTCCATTTGTGCTTTGAGAGACTTTGCTAAGTCAGTATGGGAATCATTTAAATTTGT 2161
GATTTGGGGAAATGCTGGCACTATGACTACTGCACAAAGGCAGGTGAAGGGACAAATCCA 2221
GTGAGGAGGGGGCAGTGAAGAAGTGGAGGGGAGTCTGGAGAAGCAGGTCTCTCCTTGCCC 2281
CTTGTTCGTGAGATGAAATCCTCCTGCTTTGGATGGGAGGCTGCGTGTCTTGGTGGAAAG 2341
AGCAGTGGGAGGAGGGAGAAGATTTGTGCTCCTCCCAGCTCAGCCACCAAGAAACTGTGA 2401
CCTCAGATGAATCACAGGCCTGGCTGGGGCTCAGTTTCCTCATCTTAAAAGAGGCCTATT 2461
GGGTTCACTAAAATTTCTATGATCTTCTTTGCTCTATAATCCTACAATTCTGTGGACAGA 2521
AAATGAAATGAGGTAGGAGAAAGAAATAGCCTTTGAAGAGGTTCTTGGGCATTCCACTGC 2581
CAGGCTCTGGTCAACCTTCATACTCTGCAGCCCAAGAAGAGGCAAGACCATTTGTCTGTT 2641
TTTGGAAATGCAAATAGGCGGCATTTATACCTCACGAAAGAACTGTTCTGTCAACTTTTG 2701
GATACTGGGCTATCTTGGCTGGAGAAATCCTTAGGCTCCCAAACTTTCTCTCATGAAATT 2761
GTCTTGAGTCTTTAAATTTATGGCTTCTCGAAGCTGAGAGATAACTTTAAGCATAAAGAC 2821
AAATTACATTTTCCAACATTTTGTCTAAGAGACAAAGACCTCCACATGCCTTTGGGTTTG 2881
GCCTGGATCTAAATGGGCTTGAATGAGAAGGGGAGGGTGTTGTTATGACTATGTTTAGAA 2941
GAGAAAACAGAGGTTTGGAGAGGTTAAGTGGCTGGTTCAAAGTCAGAGTTATTGCACACA 3001
CAGGATTCGAACCCATATGTTTTGTCCCTCCACTTTAGGGTTCTTTTCGCTACATAATTT 3061
TGAGAATTCTGTACCAGTCAATTTAAGGATGTGTGATGTTCCCCATCCTATTACAGCACA 3121
ACCAGCAATTTAATTATAATTTTAGTCTTAACTGCTGAAGAAAGCAGCATTACATATTAA 3181
GCTAACATATTCCTGGTGAAAGCAACTTTTTCAAAGGAATATTTCTATTTTCATGGACCA 3241
TGACAGTAGCACAGCCTGATGGCTTGTATGCCTGAAACTAATTTTGCTGTTTTCTTTCCC 3301

105 110 115
Val Asn Asp Leu Lys Val Gln Arg Lys Ala Ile Ser Glu Leu
AATAG GTA AAT GAT CTG AAG GTC CAG CGC AAA GCA ATA AGT GAA CTC 3348

120 125 130
Ile Lys Val Met Asn Asp Leu Ser Pro Lys Ala Asn Leu Arg Lys
ATC AAA GTG ATG AAT GAT CTG TCG CCC AAA GCT AAC CTG AGG AAG 3393

Fig. 1b

```
              135                 140                 145
Arg Lys Arg Ser Gln Asn Pro Phe Arg Gly Arg Arg Ala Leu Gln
CGG AAG AGG AGT CAG AAT CCA TTT CGA GGC CGG AGA GCG TTG CAA   3438

***
TAG TGGTCATCCTGCCTGCAATATTTGAATTTTTAAATCTAAATCTATTTATTAATATT  3497
TAATATTTTACATTATTTATATGGGAATATATTTTTAAACTCATCAAAGTATTTATAAT  3557
AGCAACTTTTATGTAATGAAAATGGGTATCTATTAATATATATATTATTTATAATTCCTG  3617
TATGGTGTGACTATTTCACTTGACCCTTTTTTTTCTGACCAACTAGGCAAGATTATGTGA  3677
TTACAAGGCTTTAACTCAGGGGCCAACTAGGGAGTGGGTAGCCGACCTACCAAGACCCTG  3737
TGAGCTGTGTGTTTATTTCCCTCAATGATACAATGAACACTTATAAAGGAAAGGAGGGCC  3797
TCCAGTCACTGCCTGTTGGAGAACATGTCTGCATTGTGAGCCACTGCTTAATGGCATGTC  3857
AAACCACGCTTGAATGTGTCAGATGATAGGGCTTGTCCCCTGATAAAGCTTAGTATCTCC  3917
TCTCATGCCTAGTGCTTCAGAATATTGTTGACAACTGTGACTGCACCCAAATGGAAAGTA  3977
ATTTATTTGTTTAGTTTACCAATATTTAATAAATATGAATAAAGTATAATTTCATAACTA  4037
TTTATGCTGCGTCCGGCTTTTTCTAAGTGAGGACTGGGGTAAATGAACTACAAACTAATG  4097
AATCAGTAAGAGGGAACTCGTTTTTAGCGGTGGAAATCTTAGCTGGATTAAGCCCCATGA  4157
AACGTGGTATTTCTCTCCACTGGAGATTTGTTGGCTACTACTCCTCCATGTAGCAGCTCT  4217
TTATCTTTCCAAAATATAAATTTAATTATGTCACCATTTACTTCAGAGCTTCTGCGATGG  4277
AAAGTAGTTCAAATAGTTTAGCTTAGCACACAAAGCTTTGTTTCTCCCTCCTCCCTCAAC  4337
TCTGCACTGTGCTCTTCATCTTGGTGTCCCCACGTCCTCTGTCCACTTCGGGCAAACCAC  4397
CGGGAATGTCATGGTGAGGGTGAGCTCTAGGGAGAGAGGGCTGGATTAGAATTTCGGCCC  4457
CACCATTACCAGTAGTATGACCTTTAATGAATTACTTGTATTCTCTAAGCTCCAGTTTCC  4517
TCATCTGACACAAGAGAATAATTGTGCCTAAAATTGTGGTGAGAGTTTGTTCTTTCACTC  4577
AAGAAGTGTTTACTGGAGCATCCACTAGTTGCCTAGTGCTGTTCTAGGCACTTGAGATAC  4637
ATTTGTGAACAAAATAGTCAAGGATCC                                   4664
```

Fig. 1c

GAMMA-INTERFERON

```
           S1        S10       S20    1         10         20         30         40         50         60         70
HUMAN      MKYTSYILAFQLCIVLGSLG   CYCQDPYVKE AENLKKYFNA GHSDVADNGT LFLGILKNWK EESDRKIMQS QIVSFYFKLF KNFKDDQSIQ
EQUINE     MNYTSFILAFQLCAILGSST   YYCQAAFFKE IENLKEYFNA RNPDVGDGGP LFLDILKNWK EDSDKKIIQS QIVSFYFKLF ENLKDNQVIQ
BOVINE     MKYTSYFLALLLCGLLGFSG   SYGQGQFFRE IENLKEYFNA SSPDVAKGGP LFSDILKNWK DESDKKIIQS QIVSFYFKLF ENLKDNQVIQ
MURINE     MNATHCILALQLFLMAV-SG   CYCHGTVIES LESLNNYFNS SGIDV-EEKS LFLDIWRNWQ KDGDMKILQS QIISFYLRLF EVLKDNQAIS
RAT        MSATRRVLVLQLCLMAL-SG   CYCQGTLIES LESLKNYFNS SSMDAMEGKS LLLDIWRNWQ KDGNTKILES QIISFYLRLF EVLKDNQAIS

CONSENSUS  M--T---La--Lc-----sg   -Ycq------ -E-Lk-YFN- ---Dv----- Lfldl--NW- ---d-KI-qS QI-SFY--LF e-lKDnQ-I-

                     80         90        100        110        120        130        140        150
HUMAN      KSVETIKEDM NVKFFNSNKK KRDDFEKLTN YSVTDLNVQR KAIHELIQVM AELSPAAKTG KRKRSQMLFR GRRAFQ
EQUINE     KSMDTIKEDL FVKFFNSSTS KLEDFQKLIQ IPVNDLKVQR KAISELIKVM NDLSPKANLR KRKRSQNPFR GRRALQ
BOVINE     RSMDIIKQDM FQKFLNGSSE KLEDFKKLIQ IPVDDLQIQR KAINELIKVM NDLSPKSNLR KRKRSQNLFR GRRASM
MURINE     NNISVIESHL ITTFFSNSKA KKDAFMSIAK FEVNNPQVQR QAFNELIRVV HQLLPESSLR KRKRSRC
RAT        NNISVIESHL ITNFFSNSKA KKDAFMSIAK FEVNNPQIQH KAVNELIRVI HQLSPESSLR KRKRSRC

CONSENSUS  -----I---- ---Ff--s-- K---F----- --V-----Qr kA--ELI-V- --LsP---lr KRKRS---FR GRRA--
```

Fig. 2

```
      1           5                   10                  15
     Tyr Tyr Cys Gln Ala Ala Phe Phe Lys Glu Ile Glu Asn Leu Lys Glu Tyr
     |--------------------------------(1)--------------------------------
                 |--------------------(15)-------------------------------
5'-  TAC TAC TGC CAG GCT GCT TTC TTT AAA GAA ATC GAA AAC CTG AAA GAA TAC   51
3'-  ATG ATG ACG GTC CGA CGA AAG AAA TTT CTT TAG CTT TTG GAC TTT CTT ATG
                 |--------------------(16)-------------------------------
     |--------------------------------(2)--------------------------------
                               AheIII

         20                  25                  30                  35
Phe Asn Ala Arg Asn Pro Asp Val Gly Asp Gly Gly Pro Leu Phe Leu Asp Ile
---(1)------| |--------------------(3)-----------------------------------
---(15)-----|
TTC AAC GCT CGT AAC CCA GAC GTT GGT GAC GGT GGT CCG CTG TTC CTG GAC ATC   105
AAG TTG CGA GCA TTG GGT CTG CAA CCA CTG CCA CCA GGC GAC AAG GAC CTG TAG
-(16)-| |-----------------------(4)--------------------------------------
-(2)--|                                     AvaII

                40                  45                  50
Leu Lys Asn Trp Lys Glu Asp Ser Asp Lys Lys Ile Ile Gln Ser Gln Ile Val
--------------(3)---------| |------------------------(5)-----------------
CTG AAA AAC TGG AAA GAA GAC TCT GAC AAA AAG ATC ATC CAG TCT CAG ATC GTT   159
GAC TTT TTG ACC TTT CTT CTG AGA CTG TTT TTC TAG TAG GTC AGA GTC TAG CAA
--------------(4)-------------| |--------------------(6)-----------------

    55                  60                  65                  70
Ser Phe Tyr Phe Lys Leu Phe Glu Asn Leu Lys Asp Asn Gln Val Ile Gln Lys
------------------(5)------------------------------| |--------(7)-------
TCT TTC TAC TTC AAA CTG TTC GAA AAC CTG AAA GAC AAC CAG GTT ATC CAG AAA   213
AGA AAG ATG AAG TTT GAC AAG CTT TTG GAC TTT CTG TTG GTC CAA TAG GTC TTT
------------------(6)-------------------------| |---------(8)------------

                75                  80                  85
Ser Met Asp Thr Ile Lys Glu Asp Leu Phe Val Lys Phe Phe Asn Ser Ser Thr
----------------------(7)--------------------------------------| |-(9)--
TCG ATG GAC ACT ATC AAA GAA GAT CTG TTC GTT AAA TTC TTC AAC TCG TCG ACT   267
AGC TAC CTG TGA TAG TTT CTT CTA GAC AAG CAA TTT AAG AAG TTG AGC AGC TGA
----------------------(8)--------------------------------------| |-(10)
ClaI                     BglII                                 SalI

90                  95                  100                 105
Ser Lys Leu Glu Asp Phe Gln Lys Leu Ile Gln Ile Pro Val Asn Asp Leu Lys
--------------------------------(9)-------------------------------------|
TCT AAA CTG GAA GAC TTC CAG AAA CTG ATC CAG ATC CCA GTT AAC GAC CTG AAA   321
AGA TTT GAC CTT CTG AAG GTC TTT GAC TAG GTC TAG GGT CAA TTG CTG GAC TTT
--------------------------------(10)------------------------------------

        110                 115                 120                 125
Val Gln Arg Lys Ala Ile Ser Glu Leu Ile Lys Val Met Asn Asp Leu Ser Pro
|-----------------------------(11)--------------------------------------
GTT CAG CGT AAG GCT ATC TCT GAA CTG ATC AAA GTT ATG AAC GAC CTG TCT CCA   375
CAA GTC GCA TTC CGA TAG AGA CTT GAC TAG TTT CAA TAC TTG CTG GAC AGA GGT
-(10)-| |---------------------(12)--------------------------------------

                130                 135                 140
Lys Ala Asn Leu Arg Lys Arg Lys Arg Ser Gln Asn Pro Phe Arg Gly Arg Arg
-(11)-| |---------------------------(13)--------------------------------
AAA GCT AAC CTG CGT AAA CGT AAA CGT TCT CAG AAC CCA TTC CGT GGT CGT CGT   429
TTT CGA TTG GAC GCA TTT GCA TTT GCA AGA GTC TTG GGT AAG GCA CCA GCA GCA
-(12)-----------| |-----------------(14)--------------------------------

    145
Ala Leu Gln ***
----(13)-------|
GCT CTT CAG TAA G       -3'                                               442
CGA GAA GTC ATT CCTAG   -5'                                               446
----(14)-----------|
                BamHI
```

Fig. 3

```
     1               5                   10                  15
     Tyr Tyr Cys Gln Ala Ala Phe Phe Lys Glu Ile Glu Asn Leu Lys Glu Tyr Phe
eq   TAT TAC TGC CAG GCC GCG TTT TTT AAA GAA ATA GAA AAC CTA AAG GAA TAT TTT
       *               *   *   *               *           *   *       *   *
syn  TAC TAC TGC CAG GCT GCT TTC TTT AAA GAA ATC GAA AAC CTG AAA GAA TAC TTC

         20                  25                  30                  35
     Asn Ala Arg Asn Pro Asp Val Gly Asp Gly Gly Pro Leu Phe Leu Asp Ile Leu
eq   AAC GCA AGA AAC CCA GAT GTA GGG GAT GGT GGG CCT CTT TTC CTG GAT ATC TTG
           * * *           *   *   *   *       *   *   *           *     *
syn  AAC GCT CGT AAC CCA GAC GTT GGT GAC GGT GGT CCG CTG TTC CTG GAC ATC CTG

                 40                  45                  50
     Lys Asn Trp Lys Glu Asp Ser Asp Lys Lys Ile Ile Gln Ser Gln Ile Val Ser
eq   AAG AAC TGG AAA GAG GAT AGT GAC AAA AAA ATA ATT CAG AGC CAA ATC GTC TCC
       *               *   * **            *   *   *     ***   *       *   *
syn  AAA AAC TGG AAA GAA GAC TCT GAC AAA AAG ATC ATC CAG TCT CAG ATC GTT TCT

     55                  60                  65                  70
     Phe Tyr Phe Lys Leu Phe Glu Asn Leu Lys Asp Asn Gln Val Ile Gln Lys Ser
eq   TTC TAC TTC AAA CTC TTT GAA AAC TTG AAA GAT AAC CAG GTC ATT CAA AAG AGC
                       *   *         *         *           *   *   *   * ***
syn  TTC TAC TTC AAA CTG TTC GAA AAC CTG AAA GAC AAC CAG GTT ATC CAG AAA TCG

             75                  80                  85                  90
     Met Asp Thr Ile Lys Glu Asp Leu Phe Val Lys Phe Phe Asn Ser Ser Thr Ser
eq   ATG GAC ACC ATC AAG GAG GAC CTG TTC GTT AAG TTC TTT AAC AGC AGC ACC AGC
               *       *   *   *               *       *     *** ***   * ***
syn  ATG GAC ACT ATC AAA GAA GAT CTG TTC GTT AAA TTC TTC AAC TCG TCG ACT TCT

                     95                  100                 105
     Lys Leu Glu Asp Phe Gln Lys Leu Ile Gln Ile Pro Val Asn Asp Leu Lys Val
eq   AAG CTG GAA GAC TTC CAA AAG CTG ATT CAG ATT CCG GTA AAT GAT CTG AAG GTC
       *                   *   *       *       *   *   *   *   *       *   *
syn  AAA CTG GAA GAC TTC CAG AAA CTG ATC CAG ATC CCA GTT AAC GAC CTG AAA GTT

         110                 115                 120                 125
     Gln Arg Lys Ala Ile Ser Glu Leu Ile Lys Val Met Asn Asp Leu Ser Pro Lys
eq   CAG CGC AAA GCA ATA AGT GAA CTC ATC AAA GTG ATG AAT GAT CTG TCG CCC AAA
           *   *   *   * **        *           *       *   *       *   *
syn  CAG CGT AAG GCT ATC TCT GAA CTG ATC AAA GTT ATG AAC GAC CTG TCT CCA AAA

                 130                 135                 140
     Ala Asn Leu Arg Lys Arg Lys Arg Ser Gln Asn Pro Phe Arg Gly Arg Arg Ala
eq   GCT AAC CTG AGG AAG CGG AAG AGG AGT CAG AAT CCA TTT CGA GGC CGG AGA GCG
                 * *   *   *   * * * **        *       *   *   *   * * *   *
syn  GCT AAC CTG CGT AAA CGT AAA CGT TCT CAG AAC CCA TTC CGT GGT CGT CGT GCT

     145
     Leu Gln ***
eq   TTG CAA TAG
     * *   *   *
syn  CTT CAG TAA
```

Fig. 4

Codon Verwendung von reifem EqIFN-gamma: natürliches Gen
(synthetisches Gen)

| | T | C | A | G | |
|---|---|---|---|---|---|
| T | 6 ( 1) Phe | 0 (7) Ser | 2 ( 0) Tyr | 0 (0) Cys | T |
| | 6 (11) Phe | 1 (0) Ser | 2 ( 4) Tyr | 1 (1) Cys | C |
| | 0 ( 0) Leu | 0 (0) Ser | 0 ( 1) STOP | 0 (0) STOP | A |
| | 3 ( 0) Leu | 1 (3) Ser | 1 ( 0) STOP | 1 (1) Trp | G |
| C | 1 ( 1) Leu | 1 (0) Pro | 0 ( 0) His | 0 (8) Arg | T |
| | 2 ( 0) Leu | 1 (0) Pro | 0 ( 0) His | 1 (0) Arg | C |
| | 1 ( 0) Leu | 2 (4) Pro | 4 ( 0) Gln | 1 (0) Arg | A |
| | 7 (13) Leu | 1 (1) Pro | 6 (10) Gln | 2 (0) Arg | G |
| A | 4 ( 0) Ile | 0 (2) Thr | 3 ( 0) Asn | 3 (0) Ser | T |
| | 4 (11) Ile | 2 (0) Thr | 8 (11) Asn | 5 (0) Ser | C |
| | 3 ( 0) Ile | 0 (0) Thr | 9 (17) Lys | 2 (0) Arg | A |
| | 2 ( 2) Met | 0 (0) Thr | 10 ( 2) Lys | 2 (0) Arg | G |
| G | 1 ( 7) Val | 1 (6) Ala | 7 ( 1) Asp | 1 (4) Gly | T |
| | 3 ( 0) Val | 1 (0) Ala | 4 (10) Asp | 1 (0) Gly | C |
| | 2 ( 0) Val | 2 (0) Ala | 6 ( 8) Glu | 0 (0) Gly | A |
| | 1 ( 0) Val | 2 (0) Ala | 2 ( 0) Glu | 2 (0) Gly | G |

Fig. 5

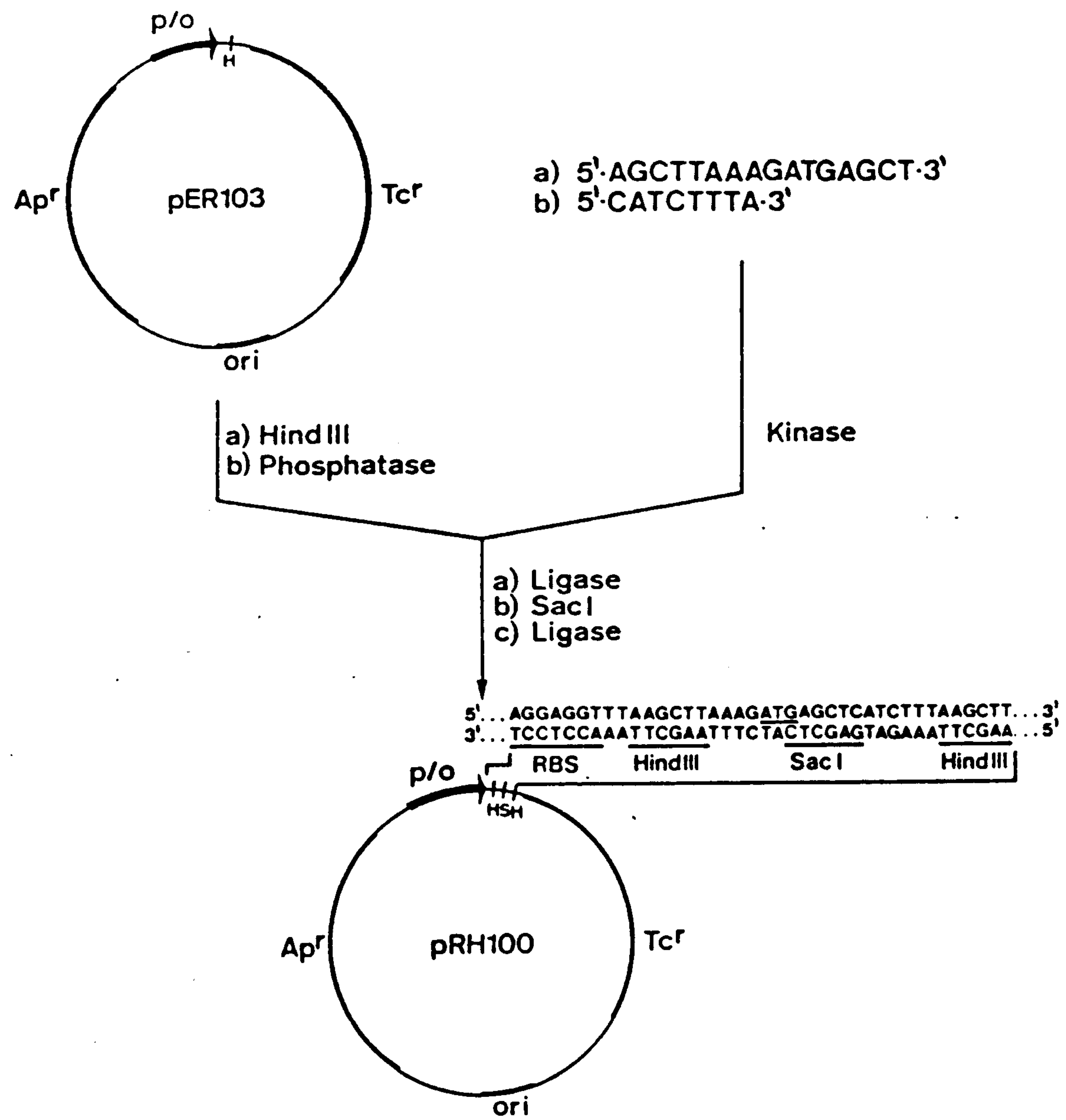


H: HindIII, S: SacI

$Ap^r$: Ampicillin resistance gene

$Tc^r$: Tetracyclin resistance gene

ori: origin of replication

RBS: ribosome binding site

p/o: tryptophan promoter/operator

Fig. 6

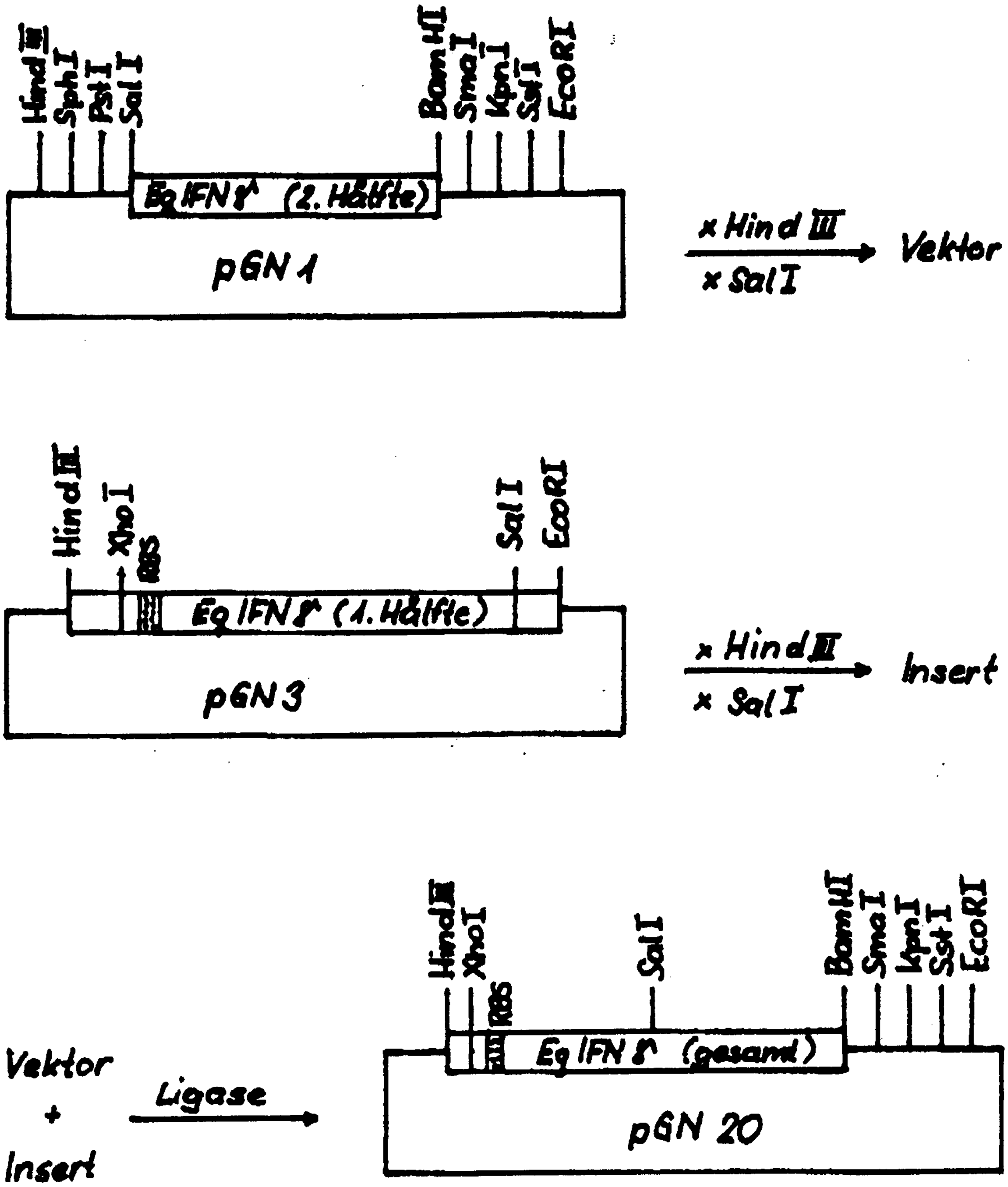
HindIII
SphI
PstI
SalI
BamHI
SmaI
KpnI
SalI
EcoRI
Eq IFN γ (2. Hälfte)
pGN 1
× HindIII
× SalI
Vektor
HindIII
XhoI
RBS
SalI
EcoRI
Eq IFN γ (1. Hälfte)
pGN 3
× HindIII
× SalI
Insert
Vektor
+
Insert
Ligase
HindIII
XhoI
RBS
SalI
BamHI
SmaI
KpnI
SstI
EcoRI
Eq IFN γ (gesamt)
pGN 20

Fig. 7